(19) Europäisches Patentamt
European Patent Office
Office européen des brevets

(11) **EP 2 251 005 B1**

(12) **EUROPEAN PATENT SPECIFICATION**

(45) Date of publication and mention
of the grant of the patent:
**10.10.2012 Bulletin 2012/41**

(51) Int Cl.:
*A61K 9/20* (2006.01)     *A61K 31/5375* (2006.01)
*A61K 47/10* (2006.01)    *A61K 47/32* (2006.01)
*A61K 47/36* (2006.01)    *A61K 47/38* (2006.01)

(21) Application number: **09710592.8**

(22) Date of filing: **13.02.2009**

(86) International application number:
**PCT/JP2009/052439**

(87) International publication number:
**WO 2009/102038 (20.08.2009 Gazette 2009/34)**

(54) **ORALLY DISINTEGRATING TABLETS**

ORAL ZERFALLENDE TABLETTEN

COMPRIMÉS SE DÉLITANT ORALEMENT

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **13.02.2008   JP 2008032490**
**23.04.2008   JP 2008113249**

(43) Date of publication of application:
**17.11.2010   Bulletin 2010/46**

(73) Proprietor: **Dainippon Sumitomo Pharma Co., Ltd.**
**Osaka 541-8524 (JP)**

(72) Inventors:
• **FUJIWARA, Keiichi**
**Ibaraki-shi**
**Osaka 567-0878 (JP)**
• **OCHIAI, Yasushi**
**Ibaraki-shi**
**Osaka 567-0878 (JP)**
• **KIMURA, Yohei**
**Ibaraki-shi**
**Osaka 567-0878 (JP)**

(74) Representative: **Webster, Jeremy Mark et al**
**Mewburn Ellis LLP**
**33 Gutter Lane**
**London**
**EC2V 8AS (GB)**

(56) References cited:
EP-A1- 1 964 549       WO-A1-2007/018057
WO-A1-2007/043538     WO-A1-2007/072840
WO-A1-2009/011451     US-B1- 6 413 541

• **WESTERHUIS J A ET AL: "Optimisation of the composition and production of mannitol/ microcrystalline cellulose tablets", INTERNATIONAL JOURNAL OF PHARMACEUTICS (AMSTERDAM), vol. 143, no. 2, 1996, pages 151-162, XP002660995, ISSN: 0378-5173**
• **FUJINAWA T. ET AL.: 'Kokunai Soku Hokaijo no Hokaisei no Hyoka Hoho ni Kansuru Kenkyu' DAI 46 KAI HOKURIKU SHIN'ETSU YAKUZAISHI TAIKAI/DAI 39 KAI HOKURIKU SHIN'ETSU YAKUZAISHI GAKUJUTSU TAIKAI/DAI 8 KAI TOYAMA-KEN KUSURI GAKKAI NENKAI KOEN YOSHISHU 2006, page 81**

EP 2 251 005 B1

**Description**

Technical Field

The present invention relates to an orally disintegrating tablet having sufficient hardness and superior in the disintegration property in the oral cavity. More particularly, the present invention relates to an orally disintegrating tablet that disintegrates rapidly in the oral cavity even when taken with a small amount of water or without water, and has hardness equivalent to that of generally tablets, particularly, an orally disintegrating tablet having sufficient hardness even under moist conditions such as after opening a packaged container and capable of maintaining good disintegrability.

Background Art

With the advent of an aging society, the development of an orally disintegrating tablet easy to take even for senile patients, for whom swallowing a tablet is not easy or difficult, is desired, and various orally disintegrating tablets are now commercially available.
Senile patients often take several kinds of medicaments at once, and use of various medicaments may require different medication hours, which often renders ingestion by accurate dosage and administration difficult to follow. Moreover, quite a number of senile patients have lame fingers, and it is highly burdensome for them to pick up a medicament from separate containers. To solve these problems, an increasing number of hospitals and pharmacies employ one dose package, in which a plurality of medicaments are placed in a single package, in an attempt to improve compliance by preventing senile patients from forgetting to take medicaments, reducing burden of picking up a medicament from a container and the like.
To employ one dose package, however, it is important that the property of the medicament be maintained even after it is picked up from the packaging container. For an orally disintegrating tablet, among others, maintenance of disintegration property and hardness even under high humidity conditions is essential. Particularly, to prevent medical malpractice during one dose packaging, an increasing number of hospitals and pharmacies use automatic packaging machines. Thus, hardness of the medicament is required, which is capable of enduring the physical impact from an automatic packaging machine to which it is applied after being picked up from a packaging container.
However, when rapid disintegration property is conferred to conventional orally disintegrating tablets, problems occur in that the hardness thereof cannot be increased with ease as compared to conventional tablets, the hygroscopicity thereof increases, the hardness thereof drastically decreases under moist conditions, thus failing to maintain necessary hardness, and the tablets can break easily before intake. For this reason, there is a demand for the creation of an orally disintegrating tablet that maintains disintegrability and hardness even when stored under moist conditions in medical practice settings.
Therefore, many reports have heretofore been made with regard to orally disintegrating tablets. For example, patent document 1 discloses, as a preparation showing adequate hardness and rapid disintegration property, as well as productivity free of problems, a rapid disintegrating solid formulation containing a) an active ingredient, b) sugar or sugar alcohol having an average particle size of 30 $\mu$m to 300 $\mu$m, c) disintegrant and d) cellulose, and discloses carmellose calcium, sodium carboxymethyl starch, croscarmellose sodium and crospovidone as disintegrants, and crystalline cellulose, powder cellulose, low-substituted hydroxypropylcellulose and carmellose as celluloses.
In addition, patent document 2 discloses, as an orally disintegrating tablet showing sufficient hardness, which is easy to take and superior in rapid disintegration property in the oral cavity, a rapid disintegrating solid tablet containing D-mannitol having an average particle size of 31 $\mu$m to 80 $\mu$m, active ingredient, disintegrant and 0.01 wt% to 0.5 wt% of stearic acid or stearic acid metal salt, and recites low-substituted hydroxypropylcellulose, crystalline cellulose, carmellose calcium and croscarmellose sodium as disintegrants. As a preparation method of the aforementioned tablet, preparation by an "external lubrication tableting method" is disclosed, wherein a lubricant such as stearic acid and the like is externally localized.
Patent document 3 discloses an orally-disintegrating composition containing mannitol, disintegrant, cellulose, lubricant, and at least one kind of starches and lactose, which disintegrates rapidly in the oral cavity and has practically sufficient strength, and recites powder cellulose and crystalline cellulose as celluloses, and crospovidone and croscarmellose sodium as preferable disintegrants. Moreover, it specifies the mixing ratio of mannitol and starch or lactose which provides an effect of reducing disorder during compression molding.
Patent document 4 discloses an orally rapidly disintegrating tablet containing (1) a drug, (2) a water-soluble polymer, (3) D-mannitol having a particular shape, (4) one kind selected from carboxymethylcellulose and rice starch, which has sufficient hardness and is superior in disintegration property and comfortable ingestion.
Patent document 5 discloses a rapid disintegrating solid formulation containing group 1 containing a) an active ingredient, b-1) sugar and/or sugar alcohol and c-1) cellulose and group 2 containing b-2) sugar and/or sugar alcohol and c-1) cellulose, wherein group 1 and/or group 2 contain d) solubilizing agent(s), which is superior in disintegration property, dissolution control property and productivity, and discloses crystalline cellulose, powder cellulose, low-substituted hydroxypropylcellulose, carmellose and the like as celluloses.

Patent documents 1, 2 and 4 are characterized by the use of particular sugar or sugar alcohol such as mannitol and the like, and patent documents 1 and 3 provide effects by using a swellable disintegrant with high disintegrability such as carmellose calcium, sodium carboxymethyl starch, croscarmellose sodium, crospovidone and the like as an essential ingredient. However, conventional orally-disintegrating tablets include those having insufficient hardness, those not usable for one dose package, and those showing insufficient disintegration property in the oral cavity.

Each of the above-mentioned patent documents discloses that crystalline cellulose is or can be used. While crystalline cellulose is known to be a highly compactable material, the form thereof varies widely and many grades exist depending on for example, average particle size and shape. Recently, crystalline cellulose having a very low bulk density of less than 0.2 g/cm$^3$ has been prepared, and application to powders for wet-granule tableting of low compactable drugs (improvement of compactibility and friability by addition of less than 10%), application to direct-compression tableting of a tablet containing a main drug at a high level (suppression of tableting trouble when drug content is high), and application to direct-compression tableting of a liquid main drug (suppression of exude of liquid main drug during compression) are described (non-patent document 1). However, specific description relating to the application to orally disintegrating tablets is not provided.

patent document 1: WO00/78292

patent document 2: W02003/103713

patent document 3: JP-A-2000-273039

patent document 4: JP-A-2007-197438

patent document 5: JP-A-2003-34655

non-patent document 1: ASAHI KASEI CHEMICALS CORPORATION, the 3rd Lecture Meeting by Association for Innovative Formulation Technology, published November 28, 2006, P121 - 136

Disclosure of the Invention

Problems to be Solved by the Invention

Therefore, the present invention aims to provide an orally disintegrating tablet capable of being enclosed in one dose package, that is, an orally disintegrating tablet that possesses both adequate hardness and rapid disintegrability in the oral cavity, maintains orally disintegrability even under moist conditions, and maintains hardness of not less than a predetermined level necessary for using an automatic packaging machine.

Means of Solving the Problems

In an attempt to solve the above-mentioned problems, the present inventors had an idea of preparing an orally disintegrating tablet by using crystalline cellulose having a low bulk density. Using crystalline cellulose having a low bulk density, an orally disintegrating tablet having high hardness and rapid disintegration property was obtained. Unexpectedly, however, the disintegration time under moist conditions in the oral cavity was found to become longer. Thus, an orally disintegrating tablet comprising crystalline cellulose, and crospovidone, croscarmellose sodium and carmellose calcium, which are disintegrants with high disintegrability, in combination was prepared. Using such disintegrants, an orally disintegrating tablet having sufficient hardness and rapid disintegration property cannot be obtained, or even if obtained, a problem of concave/convex in the appearance which causes drastic decrease in the hardness was found to occur after humidification.

Furthermore, the present inventors have conducted intensive studies and found that by combining two or more kinds of ingredients selected from carmellose, low-substituted hydroxypropylcellulose and cornstarch with an active ingredient, mannitol and crystalline cellulose having a bulk density of not more than 0.18 g/cm$^3$, the obtainable orally disintegrating tablet has superior disintegrability and adequate hardness as compared to conventionally known orally disintegrating tablets, maintains rapid oral disintegrability even under moist conditions, and maintains hardness of not less than a predetermined level necessary for packing by an automatic packaging machine, which resulted in the completion of the present invention.

Accordingly, the present invention relates to the following [1] to [32].

[1] An orally disintegrating tablet comprising (1) an active ingredient, (2) mannitol, (3) crystalline cellulose and (4) at least two kinds of particular ingredients selected from the group consisting of low-substituted hydroxypropylcellulose, cornstarch and carmellose, wherein the blending ratio of each ingredient relative to 100 wt% of the disintegrating tablet is (1) 0.01 to 50 wt%, (2) 20 to 86 wt%, (3) 10 to 30 wt%, and (4) 1 to 20 wt% for each particular ingredient and 3 to 60 wt% as the total of the particular ingredients to be blended, and an assembly of (3) crystalline cellulose to be blended has a bulk density of not more than 0.18 g/cm$^3$.

[2] The orally disintegrating tablet of the above-mentioned [1], wherein the (4) particular ingredient comprises carmellose and cornstarch.

[3] The orally disintegrating tablet of the above-mentioned [1] or [2], wherein the blending ratio of (2) mannitol is 20 to 75 wt%, relative to 100 wt% of the disintegrating tablet.

[4] The orally disintegrating tablet of the above-mentioned [3], wherein, in (4) particular ingredient, the blending ratio of carmellose is 1 to 10 wt% and that of cornstarch is 5 to 20 wt%, relative to 100 wt% of the disintegrating tablet.

[5] The orally disintegrating tablet of the above-mentioned [1], wherein the particular ingredient of (4) comprises low-substituted hydroxypropylcellulose, carmellose and cornstarch.

[6] The orally disintegrating tablet of the above-mentioned [5], wherein the particular ingredient of (4) comprises 1 to 10 wt% of low-substituted hydroxypropylcellulose, 1 to 10 wt% of carmellose and 5 to 20 wt% of cornstarch, relative to 100 wt% of the disintegrating tablet.

[7] The orally disintegrating tablet of any one of the above-mentioned [1] to [6], wherein (1) the active ingredient is 4-amino-5-chloro-2-ethoxy-N-[[4-(4-fluorobenzyl)-2-morpholinyl]methyl]benzamide (hereinafter to be referred to as "compound A") or a pharmaceutically acceptable salt thereof.

[8] The orally disintegrating tablet of any one of the above-mentioned [1] to [7], further comprising (5) at least one kind of water-soluble polymer selected from the group consisting of methylcellulose, hydroxypropylcellulose, polyvinyl alcohol, hypromellose and polyvinylpyrrolidone in a total blending ratio of 0.5 to 10 wt% relative to 100 wt% of the disintegrating tablet.

[9] The orally disintegrating tablet of the above-mentioned [8], wherein water-soluble polymer (5) is at least one kind of water-soluble polymer selected from the group consisting of methylcellulose and hydroxypropylcellulose, and the total blending ratio thereof relative to 100 wt% of the disintegrating tablet is 0.5 to 5 wt%.

[10] An orally disintegrating tablet obtainable by mixing and forming; active ingredient-containing particles obtainable by mixing and particulating (1a) an active ingredient, (2a) mannitol and (5a) at least one kind of water-soluble polymer selected from the group consisting of methylcellulose and hydroxypropylcellulose; (2b) mannitol; (3b) crystalline cellulose; (4b) at least two kinds of particular ingredients selected from the group consisting of low-substituted hydroxypropylcellulose, cornstarch and carmellose and, where necessary; (5b) at least one kind of water-soluble polymer selected from the group consisting of methylcellulose, hydroxypropylcellulose, polyvinyl alcohol, hypromellose and polyvinylpyrrolidone, wherein the blending ratio relative to 100 wt% of the disintegrating tablet is (1a) 0.01 to 50 wt%, the total of (2a) and (2b) 20 to 86 wt%, (3b) 10 to 30 wt%, each particular ingredient of (4b) 1 to 20 wt% and the total of the particular ingredients to be blended 3 - 60 wt%, and when (5a) and (5b) are blended, the total thereof 0.5 to 10 wt%, and an assembly of (3b) crystalline cellulose to be blended has a bulk density of not more than 0.18 g/cm$^3$.

[11] An orally disintegrating tablet obtainable by forming a mixture of; granules obtainable by granulating a mixture of (1) an active ingredient, (2) mannitol, (3b) crystalline cellulose and (4b) at least one kind of particular ingredient selected from the group consisting of low-substituted hydroxypropylcellulose, cornstarch and carmellose and, where necessary, (5b) at least one kind of water-soluble polymer selected from the group consisting of methylcellulose, hydroxypropylcellulose, polyvinyl alcohol, hypromellose and polyvinylpyrrolidone;
(3c) crystalline cellulose; (4c) at least one kind of particular ingredient selected from the group consisting of low-substituted hydroxypropylcellulose, cornstarch and carmellose, wherein the blending ratio of each ingredient relative to 100 wt% of the disintegrating tablet is (1) 0.01 to 50 wt%, (2b) 20 to 86 wt%, the total of (3b) and (3c) 10 to 30 wt%, and at least two kinds of particular ingredients are contained in (4b) and (4c), the blending ratio of each particular ingredient is 1 to 20 wt% and the total of the particular ingredients is 3 to 60 wt% and the blending ratio of (5b) when blended is 0.5 to 10 wt%, and an assembly of (3b) crystalline cellulose and (3c) crystalline cellulose to be blended has a bulk density of not more than 0.18 g/cm$^3$.

[12] An orally disintegrating tablet comprising; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;
(2b) mannitol; (3b) crystalline cellulose; (4b) at least two kinds of particular ingredients selected from the group consisting of low-substituted hydroxypropylcellulose, cornstarch and carmellose and, where necessary; (5b) at least one kind of water-soluble polymer selected from the group consisting of methylcellulose, hydroxypropylcellulose, polyvinyl alcohol, hypromellose and polyvinylpyrrolidone, wherein the blending ratio relative to 100 wt% of the disintegrating tablet is (1a) 0.1 to 10 wt%, the total of (2a) and (2b) 20 to 86 wt%, (3b) 10 to 30 wt%, (4b) 1 to 20 wt% for each particular ingredient and 3 to 60 wt% as the total of the particular ingredients to be blended and 0.5 to 10 wt% as the total of (5a) and (5b), and the assembly of (3b) crystalline cellulose to be blended has a bulk density of not more than 0.18 g/cm$^3$.

[13] The orally disintegrating tablet of the above-mentioned [12], comprising; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;
(2b) mannitol; (3b) crystalline cellulose; (4b) at least two kinds of particular ingredients selected from the group consisting of low-substituted hydroxypropylcellulose, cornstarch and carmellose; and (5b) at least one kind of water-soluble polymer selected from the group consisting of methylcellulose and hydroxypropylcellulose, wherein the blending ratio relative to 100 wt% of the disintegrating tablet is (1a) 0.1 to 10 wt%, the total of (2a) and (2b) 20 to 86 wt%, (3b) 10 to 30 wt%, (4b) 1 to 20 wt% for each particular ingredient and 3 to 60 wt% as the total of the particular ingredients to be blended and 0.5 to 10 wt% as the total of (5a) and (5b), and the assembly of (3b) crystalline cellulose to be blended has a bulk density of not more than 0.18 g/cm$^3$.

[14] An orally disintegrating tablet obtainable by forming a mixture of: granules obtainable by granulating a mixture of; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose; (2b) mannitol; (3b) crystalline cellulose; (4b) at least one kind of particular ingredient selected from the group consisting of low-substituted hydroxypropylcellulose, cornstarch and carmellose; and (5b) at least one kind of water-soluble polymer selected from the group consisting of methylcellulose, hydroxypropylcellulose, polyvinyl alcohol, hypromellose and polyvinylpyrrolidone:

(3c) crystalline cellulose: (4c) at least one kind of particular ingredient selected from the group consisting of low-substituted hydroxypropylcellulose, cornstarch and carmellose, wherein the blending ratio relative to 100 wt% of the disintegrating tablet is (1a) 0.1 to 10 wt%, the total of (2a) and (2b) 20 to 86 wt%, the total of (3b) and (3c) 10 to 30 wt%, at least two kinds of particular ingredients are contained in (4b) and (4c) and the blending ratio of each particular ingredient in total is 1 to 20 wt%, the total of the particular ingredients to be blended is 3 to 60 wt% and the total of (5a) and (5b) is 0.5 to 10 wt%,
and the assembly of (3b) crystalline cellulose and (3c) crystalline cellulose to be blended has a bulk density of not more than 0.18 $g/cm^3$.

[15] The orally disintegrating tablet of the above-mentioned [14], which is obtainable by forming a mixture of: granules obtainable by granulating a mixture of; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;
(2b) mannitol; (3b) crystalline cellulose; (4.1b) low-substituted hydroxypropylcellulose; (4.3b) carmellose; and (5b) at least one kind of water-soluble polymer selected from the group consisting of methylcellulose and hydroxypropylcellulose:
(3c) crystalline cellulose: (4.1c) low-substituted hydroxypropylcellulose: and (4.2c) cornstarch, wherein the blending ratio relative to 100 wt% of the disintegrating tablet is (1a) 0.1 to 10 wt%, the total of (2a) and (2b) 30 to 75 wt%, the total of (3b) and (3c) 10 to 30 wt%, the total of (4.1b) and (4.1c) 1 to 20 wt%, (4.2c) 5 to 20 wt%, (4.3b) 1 to 20 wt% and the total of (5a) and (5b) is 0.5 to 10 wt%, and the assembly of (3b) crystalline cellulose and (3c) crystalline cellulose to be blended to be blended in combination has a bulk density of not more than 0.18 $g/cm^3$.

[16] The orally disintegrating tablet of any one of the above-mentioned [12] to [15], wherein the blending ratio relative to 100 wt% of the disintegrating tablet is 1 to 30 wt% for (2a) mannitol, and 0.5 to 9.9 wt% for (5a) water-soluble polymer.

[17] The orally disintegrating tablet of any one of the above-mentioned [12] to [15], wherein the (5a) water-soluble polymers comprise methylcellulose and hydroxypropylcellulose, and the (5b) water-soluble polymer is hydroxypropylcellulose.

[18] The orally disintegrating tablet of any one of the above-mentioned [1] to [17], wherein the assembly of crystalline cellulose to be blended has a bulk density of not more than 0.18 $g/cm^3$ and the rate thereof relative to 100 wt% of the disintegrating tablet is 18 to 30 wt%.

[19] The orally disintegrating tablet of any one of the above-mentioned [1] to [17], wherein the assembly of crystalline cellulose to be blended has a bulk density of not more than 0.15 $g/cm^3$ and the rate thereof relative to 100 wt% of the disintegrating tablet is 10 to 30 wt%.

[20] An orally disintegrating tablet comprising (1) an active ingredient, (2) mannitol, (3) crystalline cellulose, (4) at least two kinds of particular ingredients selected from the group consisting of (4.1) low-substituted hydroxypropylcellulose, (4.2) cornstarch and (4.3) carmellose, and (5) at least one kind of water-soluble polymer selected from the group consisting of methylcellulose and hydroxypropylcellulose, wherein the blending ratio of each ingredient relative to 100 wt% of the disintegrating tablet is (1) 0.1 to 45 wt%, (2) 20 to 75 wt%, (3) 15 to 25 wt%, (4) the blending ratio of each particular ingredient when blended is (4.1) 1 to 16 wt%, (4.2) 5 to 20 wt%, (4.3) 1 to 10 wt%, and the total of the particular ingredients to be blended is 5 to 40 wt%, and (5) 0.5 to 8 wt%, and assembly of (3) crystalline cellulose to be blended has a bulk density of not more than 0.18 $g/cm^3$.

[21] An orally disintegrating tablet comprising (1) an active ingredient, (2) mannitol, (3) crystalline cellulose, (4) at least two kinds of particular ingredients selected from the group consisting of (4.1) low-substituted hydroxypropylcellulose, (4.2) cornstarch and (4.3) carmellose, and (5) at least one kind of water-soluble polymer selected from the group consisting of methylcellulose and hydroxypropylcellulose, wherein the blending ratio of each ingredient relative to 100 wt% of the disintegrating tablet is (1) 0.1 to 25 wt%, (2) 30 to 75 wt%, (3) 15 to 25 wt%, (4) the blending ratio of each particular ingredient when blended is (4.1) 1 to 6 wt%, (4.2) 5 to 20 wt%, (4.3) 1 to 5 wt%, and the total of the particular ingredients to be blended is 5 to 35 wt%, and (5) 0.5 to 8 wt%, and assembly of (3) crystalline cellulose has a bulk density of not more than 0.18 g/cm$^3$.

[22] An orally disintegrating tablet comprising; active ingredient-containing particles obtainable by mixing and particulating (1a) compound A or a pharmaceutically acceptable salt thereof, (2a) mannitol and (5a) methylcellulose or methylcellulose and hydroxypropylcellulose;
(2b) mannitol; (3b) crystalline cellulose; (4b) at least two kinds of particular ingredients selected from the group consisting of (4.1b) low-substituted hydroxypropylcellulose, (4.2b) cornstarch and (4.3b) carmellose; and (5b) at least one kind of water-soluble polymer selected from the group consisting of methylcellulose and hydroxypropylcellulose, wherein the blending ratio relative to 100 wt% of the disintegrating tablet is (1a) 1 to 8 wt%, the total of (2a) and (2b) 20 to 75 wt%, (3b) 15 to 25 wt%, (4b) the blending ratio of each particular ingredient when blended is (4.1b) 1 to 16 wt%, (4.2b) 5 to 20 wt%, (4.3b) 1 to 10 wt%, and the total of particular ingredients to be blended is 5 to 40 wt% and the total of (5a) and (5b) is 0.5 to 8 wt%, and an assembly of (3b) crystalline cellulose to be blended has a bulk density of not more than 0.18 g/cm$^3$.

[23] An orally disintegrating tablet comprising; active ingredient-containing particles obtainable by mixing and particulating (1a) compound A or a pharmaceutically acceptable salt thereof, (2a) mannitol and (5a) methylcellulose or methylcellulose and hydroxypropylcellulose;
(2b) mannitol; (3b) crystalline cellulose; (4b) at least two kinds of particular ingredients selected from the group consisting of (4.1b) low-substituted hydroxypropylcellulose, (4.2b) cornstarch and (4.3b) carmellose; and (5b) at least one kind of water-soluble polymer selected from the group consisting of methylcellulose and hydroxypropylcellulose, wherein the blending ratio relative to 100 wt% of the disintegrating tablet is (1a) 1 to 5 wt%, the total of (2a) and (2b) 30 to 75 wt%, (3b) 15 to 25 wt%, (4b) the blending ratio of each particular ingredient to be blended is (4.1b) 1 to 6 wt%, (4.2b) 5 to 20 wt%, (4.3b) 1 to 5 wt%, and the total of particular ingredients to be blended is 5 to 35 wt% and the total of (5a) and (5b) is 0.5 to 8 wt%, and an assembly of (3b) crystalline cellulose to be blended has a bulk density of not more than 0.18 g/cm$^3$.

[24] An orally disintegrating tablet obtainable by forming a mixture of: granules obtainable by granulating a mixture of; active ingredient-containing particles obtainable by mixing and particulating (1a) compound A or a pharmaceutically acceptable salt thereof, (2a) mannitol and (5a) methylcellulose or methylcellulose and hydroxypropylcellulose; (2b) mannitol; (3b) crystalline cellulose; (4.1b) low-substituted hydroxypropylcellulose; (4.3b) carmellose; and (5b) at least one kind of water-soluble polymer selected from the group consisting of methylcellulose and hydroxypropylcellulose:
(3c) crystalline cellulose: (4.1c) low-substituted hydroxypropylcellulose: and (4.2c) cornstarch, wherein the blending ratio relative to 100 wt% of the disintegrating tablet is (1a) 1 to 8 wt%, the total of (2a) and (2b) 20 to 75 wt%, the total of (3b) and (3c) 15 to 25 wt%, the total of (4.1b) and (4.1c) 1 to 16 wt%, (4.2c) 5 to 20 wt%, (4.3b) 1 to 10 wt% and the total of (5a) and (5b) is 0.5 to 8 wt%, and an assembly of (3b) crystalline cellulose and (3c) crystalline cellulose to be blended in combination has a bulk density of not more than 0.18 g/cm$^3$.

[25] An orally disintegrating tablet obtainable by forming a mixture of: granules obtainable by granulating a mixture of; active ingredient-containing particles obtainable by mixing and particulating (1a) compound A or a pharmaceutically acceptable salt thereof, (2a) mannitol and (5a) methylcellulose or methylcellulose and hydroxypropylcellulose;

(2b) mannitol; (3b) crystalline cellulose; (4.1b) low-substituted hydroxypropylcellulose; (4.3b) carmellose; and (5b) at least one kind of water-soluble polymer selected from the group consisting of methylcellulose and hydroxypropylcellulose:

(3c) crystalline cellulose: (4.1c) low-substituted hydroxypropylcellulose: and (4.2c) cornstarch, wherein the blending ratio relative to 100 wt% of the disintegrating tablet is (1a) 1 to 5 wt%, the total of (2a) and (2b) 30 to 75 wt%, the total of (3b) and (3c) 15 to 25 wt%, the total of (4.1b) and (4.1c) 1 to 6 wt%, (4.2c) 5 to 20 wt%, (4.3b) 1 to 5 wt% and the total of (5a) and (5b) is 0.5 to 8 wt%, and an assembly of (3b) crystalline cellulose and (3c) crystalline cellulose in combination has a bulk density of not more than 0.18 g/cm$^3$.

[26] An orally disintegrating tablet comprising (1) an active ingredient, (2) mannitol, (3) crystalline cellulose and (4) at least two kinds of particular ingredients selected from the group consisting of low-substituted hydroxypropylcellulose, cornstarch and carmellose, which shows an absolute hardness of not less than 1.5N/mm$^2$ after preservation under the conditions of 25°C, relative humidity 75% for 3 days.

[27] A process of preparing an orally disintegrating tablet, comprising mixing and forming; active ingredient-containing particles obtainable by mixing and particulating (1a) an active ingredient, (2a) mannitol and (5a) at least one kind of water-soluble polymer selected from the group consisting of methylcellulose and hydroxypropylcellulose;

(2b) mannitol; (3b) crystalline cellulose; (4b) at least two kinds of particular ingredients selected from the group consisting of low-substituted hydroxypropylcellulose, cornstarch and carmellose and, where necessary, (5b) at least one kind of water-soluble polymer selected from the group consisting of methylcellulose, hydroxypropylcellulose, polyvinyl alcohol, hypromellose and polyvinylpyrrolidone, wherein the blending ratio relative to 100 wt% of the disintegrating tablet is (1a) 0.01 to 50 wt%, the total of (2a) and (2b) 20 to 86 wt%, (3b) 10 to 30 wt%, each particular ingredient of (4b) 1 to 20 wt% and the total of the particular ingredients to be blended 3 - 60 wt%, and when (5a) and (5b) are blended, the total thereof 0.5 to 10 wt%, and an assembly of (3b) crystalline cellulose to be blended has a bulk density of not more than 0.18 g/cm$^3$.

[28] A process of preparing an orally disintegrating tablet, comprising forming a mixture of; granules obtainable by granulating a mixture of (1) an active ingredient, (2b) mannitol, (3b) crystalline cellulose and (4b) at least one kind of particular ingredient selected from the group consisting of low-substituted hydroxypropylcellulose, cornstarch and carmellose and, where necessary, (5b) at least one kind of water-soluble polymer selected from the group consisting of methylcellulose, hydroxypropylcellulose, polyvinyl alcohol, hypromellose and polyvinylpyrrolidone;

(3c) crystalline cellulose; and (4c) at least one kind of particular ingredient selected from the group consisting of low-substituted hydroxypropylcellulose, cornstarch and carmellose, wherein the blending ratio of each ingredient relative to 100 wt% of the disintegrating tablet is (1) 0.01 to 50 wt%, (2b) 20 to 86 wt%, the total of (3b) and (3c) 10 to 30 wt%, and at least two kinds of particular ingredients are contained in (4b) and (4c), the blending ratio of each particular ingredient is 1 to 20 wt% and the total of the particular ingredients to be blended 3 to 60 wt%, and the blending ratio of (5b) when blended is 0.5 to 10 wt%, and an assembly of (3b) crystalline cellulose and (3c) crystalline cellulose to be blended has a bulk density of not more than 0.18 g/cm$^3$.

[29] A process of preparing an orally disintegrating tablet, comprising mixing and forming; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;

(2b) mannitol; (3b) crystalline cellulose; (4b) at least two kinds of particular ingredients selected from the group consisting of low-substituted hydroxypropylcellulose, cornstarch and carmellose and, where necessary; (5b) at least one kind of water-soluble polymer selected from the group consisting of methylcellulose, hydroxypropylcellulose, polyvinyl alcohol, hypromellose and polyvinylpyrrolidone, wherein the blending ratio relative to 100 wt% of the disintegrating tablet is (1a) 0.1 to 10 wt%, the total of (2a) and (2b) 20 to 86 wt%, (3b) 10 to 30 wt%, each particular ingredient of (4b) 1 to 20 wt% and the total of the particular ingredients to be blended 3 - 60 wt%, and when (5a) and (5b) are blended, the total thereof 0.5 to 10 wt%, and an assembly of (3b) crystalline cellulose to be blended has a bulk density of not more than 0.18 g/cm$^3$.

[30] The process of the above-mentioned [29], comprising mixing and forming; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;

(2b) mannitol; (3b) crystalline cellulose; (4b) at least two kinds of particular ingredients selected from the group consisting of low-substituted hydroxypropylcellulose, cornstarch and carmellose; and (5b) at least one kind of water-soluble polymer selected from the group consisting of methylcellulose and hydroxypropylcellulose, wherein the blending ratio relative to 100 wt% of the disintegrating tablet is (1a) 0.1 to 10 wt%, the total of (2a) and (2b) 20 to 86 wt%, (3b) 10 to 30 wt%, each particular ingredient of (4b) 1 to 20 wt% and the total of the particular ingredients to be blended 3 - 60 wt%, the total of (5a) and (5b) to be blended is 0.5 to 10 wt%, and an assembly of (3b) crystalline cellulose to be blended has a bulk density of not more than 0.18 $g/cm^3$.

[31] A process of preparing an orally disintegrating tablet, comprising forming a mixture of: granules obtainable by granulating a mixture of; active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;

(2b) mannitol; (3b) crystalline cellulose; (4b) at least one kind of particular ingredient selected from the group consisting of low-substituted hydroxypropylcellulose, cornstarch and carmellose; and (5b) at least one kind of water-soluble polymer selected from the group consisting of methylcellulose, hydroxypropylcellulose, polyvinyl alcohol, hypromellose and polyvinylpyrrolidone:

(3c) crystalline cellulose: (4c) at least one kind of particular ingredient selected from the group consisting of low-substituted hydroxypropylcellulose, cornstarch and carmellose, wherein the blending ratio relative to 100 wt% of the disintegrating tablet is (1a) 0.1 to 10 wt%, the total of (2a) and (2b) 20 to 86 wt%, the total of (3b) and (3c) 10 to 30 wt%, at least two kinds of particular ingredients are contained in (4b) and (4c), the blending ratio of each particular ingredient is 1 to 20 wt% and the total of the particular ingredients to be blended 3 to 60 wt%, and the total of (5a) and (5b) is 0.5 to 10 wt%,

and an assembly of (3b) crystalline cellulose and (3c) crystalline cellulose to be blended in combination has a bulk density of not more than 0.18 $g/cm^3$.

[32] A process of preparing an orally disintegrating tablet, comprising forming a mixture of: granules obtainable by granulating a mixture of; active ingredient-containing particles obtainable by mixing and particulating (1a) compound A or a pharmaceutically acceptable salt thereof, (2a) mannitol and (5a) methylcellulose or methylcellulose and hydroxypropylcellulose;

(2b) mannitol; (3b) crystalline cellulose; (4.1b) low-substituted hydroxypropylcellulose; (4.3b) carmellose; and (5b) at least one kind of water-soluble polymer selected from the group consisting of methylcellulose and hydroxypropylcellulose:

(3c) crystalline cellulose: (4.1c) low-substituted hydroxypropylcellulose: and (4.2c) cornstarch, wherein the blending ratio relative to 100 wt% of the disintegrating tablet is (1a) 0.1 to 10 wt%, the total of (2a) and (2b) 20 to 86 wt%, the total of (3b) and (3c) 10 to 30 wt%, the total of (4.1b) and (4.1c) 1 to 20 wt%, (4.2c) 1 to 20 wt%, (4.3b) 1 to 20 wt% and the total of (5a) and (5b) is 0.5 to 10 wt%, and an assembly of (3b) crystalline cellulose and (3c) crystalline cellulose to be blended in combination has a bulk density of not more than 0.18 $g/cm^3$.

Effect of the Invention

According to the present invention, an orally disintegrating tablet having superior disintegrability and adequate hardness, which maintains rapid oral disintegrability even under moist conditions, and hardness of not less than a predetermined level necessary for using an automatic packaging machine can be provided. In addition, an orally disintegrating tablet which is easy to take can be provided.

Embodiment for Practicing the Invention

In the present invention, the "orally disintegrating tablet" means a tablet rapidly disintegrating in oral cavity without ingesting water to take a tablet, and specifically means a tablet which is disintegrated within about 40 sec, preferably about 30 sec, in oral cavity in a disintegrating test mainly with saliva or a disintegrating test using a device, and the like.

In the present specification and Claims, the blending ratio indicates a proportion (wt%) of each ingredient to the total tablet weight when the total weight of the orally disintegrating tablet is 100 wt%.

The "average particle size" in the present specification and Claims is shown by the values measured by, for example, a laser diffraction particle size measurement apparatus (HELOS&RODOS manufactured by SYMPATEC GmbH), or a laser diffraction particle size distribution measurement apparatus (SALD3000) manufactured by SHIMADZU Corporation.

The "bulk density" in the present specification and Claims is shown by the values measured by the constant mass method (method 1) described in the Japanese Pharmacopoeia, 15th Edition. It is a numerical value represented by the following formula

bulk density $(g/cm^3)$= weight of sample (g)/x$(cm^3)$ wherein x $cm^3$ is a volume of a sample (generally about 30 g) when it is allowed to freely fall into a 100-mL $(cm^3)$ measuring flask. When the sample outpours from the measuring flask, the weight of the sample is appropriately reduced before measurement.

The orally disintegrating tablet of the present invention characteristically contains

(1) an active ingredient,
(2) mannitol,
(3) crystalline cellulose and
(4) at least two kinds of particular ingredients selected from the group consisting of low-substituted hydroxypropyl-cellulose, cornstarch and carmellose,

wherein the blending ratio relative to 100 wt% of the disintegrating tablet is

(1) 0.01 to 50 wt%,
(2) 20 to 86 wt%,
(3) 10 to 30 wt% and
(4) the blending ratio of each particular ingredient is 1 to 20 wt%, and the total thereof to be blended is 3 to 60 wt%,

and further,

assembly of (3) crystalline cellulose to be blended has a bulk density of not more than 0.18 $g/cm^3$.

More specifically, the orally disintegrating tablet of the present invention is obtainable by formulating a composition containing the following ingredients to meet the following blending ratio per 100 wt% of the orally disintegrating tablet:

(1) 0.01 to 50 wt% of an active ingredient,
(2) 20 to 86 wt% of mannitol,
(3) 10 to 30 wt% of crystalline cellulose whose assembly has a bulk density of not more than 0.18 $g/cm^3$ and
(4) at least two kinds of particular ingredients selected from the group consisting of low-substituted hydroxypropyl-cellulose, cornstarch and carmellose, each of which at a blending ratio of 1 to 20 wt%, in total 3 to 60 wt%.

The present invention is explained in more detail in the following.

(1) Active ingredient

The active ingredient used in the orally disintegrating tablet of the present invention is not particularly limited, as far as it can be subjected to the treatment or prophylaxis of disease as a pharmaceutical active ingredient, and permits oral administration. For example, nutritious tonics; antipyretic analgesic anti-inflammatory drugs; antpsychotic drugs; hypnotic sedatives; antispasmodic drugs; central nervous system drugs; brain metabolism ameliorators; brain circulation ameliorators; anti-epileptic drugs; sympathomimetic drugs; stomachic digestants; anti-ulcer agents; gastrointestinal tract movement ameliorators; antacids; antitussive expectorants; intestinal movement suppressants; antiemetics; respiratory stimulants; bronchodilators; anti-allergic drugs; antihistaminic agents; cardiotonics; anti-arrhythmic agents; diuretics; ACE inhibitors; Ca antagonists; AII antagonists; vasoconstrictors; coronary vasodilators; vasodilators; peripheral vasodilators; antihyperlipidemic agents; choleretic drugs; cephem antibiotics; oral antibacterial drugs; chemotherapeutic agents; sulfonylurea drugs; α glucosidase inhibitors; insulin resistance ameliorators; rapid-acting insulin secretagogues; DPPIV inhibitors; diabetic complication remedies; anti-osteoporosis agents; antirheumatic agents; skeletal muscle relaxants; alkaloid narcotics; sulfa drugs; goat remedies; blood coagulation inhibitors; anti-malignancy agents, and the like can be mentioned.

The active ingredient in the present invention may be in the form of a salt or a free form, as far as it is pharmaceutically acceptable. It may also be in the form of a solvate such as alcohol solvate, or a hydrate and the like. Furthermore, the active ingredient mentioned above may be used singly, or may be used in combination of two kinds or more.

The amount of the active ingredient to be blended of (1) in the present invention is generally 0.01 to 50 wt%, preferably 0.1 to 45 wt%, more preferably 0.1 to 25 wt%, relative to 100 wt% of the orally disintegrating tablet. The "blending ratio of the active ingredient" in the present invention is based on the form of a "pharmaceutical active ingredient" generally adopted as a medicament. In other words, when a drug takes the form of a salt, the amount to be the basis contains an acid or base forming the salt. When a drug contains crystal water and the like, the amount to be the basis does not include an amount corresponding to the crystal water.

The average particle size of the active ingredient to be used in the present invention may be any as long as gritty feel is absent in the oral cavity. It is generally 1 to 250 $\mu$m, preferably 1 to 150 $\mu$m, more preferably 1 to 100 $\mu$m. To achieve a desired particle size, the active ingredient is appropriately pulverized as necessary before use. Examples of the pulverization method include a method using a jet mill or hammer mill.

The active ingredient may be subjected to masking of uncomfortable tastes such as a bitter taste and the like by a known formulation method, conferred with controlled release ability such as enteric or sustained-release and the like, or subjected to a treatment for stabilization or improving productivity. Specifically, for example, an active ingredient may be granulated or coated to give particles (or granules). While the method is not particularly limited, the active ingredient may be granulated by, for example, as taught in WO 2005/055989, mixing and particulating (1) a drug with an uncomfortable taste, (2) methylcellulose and (3) mannitol to give "drug-containing particles with reduced uncomfortable taste".

Moreover, as exemplary coating of an active ingredient, for example, the method described in JP-A-3-130214 may be used.

As mentioned herein, coating includes to coating all or part of the surface of an efficacy ingredient with a coating ingredient. As apparatuses for this coating, ordinary fluidized-bed granulating machine (including rotor fluidized-bed granulating machine, Wurster fluidized-bed granulating machine and the like) can be mentioned; to suppress particle coarsening in a step, preference is given to improved Wurster fluidized-bed granulating machines equipped with an apparatus for forced circulation from side (e.g., SPC, manufactured by POWREX CORPORATION, and the like), hybrid fluidized-bed granulating machines equipped with a grinding mechanism (screen impeller type, blade stator type, cross-screws, lump breakers and the like) (e.g., super fine particle coating and granulating processor SFP-01, manufactured by POWREX CORPORATION, and the like), and rotary fluidized-bed granulating machines (e.g., OMNITECS, manufactured by NARA MACHINERY CO. LTD., and the like). As apparatuses for spray drying, ordinary spray dryers (manufactured by OKAWARA MFG. CO.,LTD, manufactured by OHKAWARA KAKOHKI CO. LTD., manufactured by Yamato, manufactured by Niro, and the like) can be used.

As mentioned above, when the active ingredient is granulated or coated, it is recommended to finely divide the active ingredient to have an average particle size of 1 to 150 $\mu$m, preferably 1 to 50 $\mu$m, more preferably 1 to 30 $\mu$m. Thus, when the active ingredient is granulated or coated etc. for a pretreatment to confer a function, average particle sizes of the particles containing the active ingredient, which represent the minimum unit for expression of the object function (active ingredient-containing particles) varies depending on the characteristics of the particles, for example, easiness of dissolution and the like. It is, for example, about 1 to 500 $\mu$m, preferably about 1 to 400 $\mu$m, more preferably about 10 to 300 $\mu$m. When the particles are slightly soluble, it may be, for example, not more than 250 $\mu$m, preferably about 1 to 150 $\mu$m. The particles containing the active ingredient, which represent the minimum unit for expression of the object function, mean the minimum constitution unit containing the active ingredient, which shows a newly conferred property that the active ingredient cannot exhibit, such as bitter taste masking, sustained release, enteric dissolution and the like, in a tablet. For example, they take the form of bitter taste-masked particles, sustained-release particles, enteric particles and the like, each containing the active ingredient. The amount of the particles containing the active ingredient (active ingredient-containing particles)to be blended, which represent the function expression minimum unit, per 100 wt% of the orally disintegrating tablet is generally 0.5 to 60 wt%, preferably 1 to 50 wt%, more preferably about 2 to 30 wt%, most preferably about 3 to 20 wt%.

In addition, when the ingredients described in (2) to (4) (further (5)) below are used in a treatment as mentioned above, the amount thereof to be used is contained in the blending ratio of the following ingredients.

(2) Mannitol

While mannitol, one of the essential ingredients in the present invention, is not particularly limited, D-mannitol is preferable. Those described in "the Japanese Pharmacopoeia" or "Japanese Pharmaceutical Excipients" can be generally used. The crystal form thereof is not particularly limited, and any of $\alpha$ type, $\beta$ type and $\delta$ type may be used, or it may be an amorphous form obtained by spray drying. On the other hand, mannitol which is spherical and has high density, as described in JP-A-11-92403, may be used.

While the average particle size of mannitol to be blended is not particularly limited, it is preferably 10 to 300 $\mu$m, more preferably 10 to 250 $\mu$m, further preferably 30 to 200 $\mu$m. To achieve a desired particle size, the active ingredient is appropriately pulverized as necessary before use. Examples of the pulverization method include a method using a jet mill or hammer mill.

The amount of mannitol to be blended per 100 wt% of the orally disintegrating tablet in the present invention is generally 20 to 86 wt%, preferably 20 to 75 wt%, more preferably 30 to 75 wt%.

(3) Crystalline cellulose

As for crystalline cellulose, which is one of the essential ingredients in the present invention, the bulk density of its assembly when blending to form the orally disintegrating tablet is important. That is, crystalline cellulose whose assembly has a bulk density of generally not more than 0.18 $g/cm^3$ is used. Preferred are those having a bulk density of not more than 0.17 $g/cm^3$, more preferably not more than 0.15 $g/cm^3$.

The effect desired in the present invention can be exhibited by a combination of crystalline cellulose having a particular bulk density and two or more kinds of particular ingredients described in the following (4). In other words, even if crystalline cellulose whose assembly has a bulk density satisfying the above-mentioned range is added in any large amounts, the desired effect cannot be achieved when it is not combined with the following particular ingredients (see Comparative Examples 1 to 5). Moreover, even if two or more kinds of the following particular ingredients (4) are used, the desired effect cannot be achieved when they are combined with crystalline cellulose whose assembly has a bulk density not satisfying the above-mentioned range (see Comparative Examples 18 to 23). Namely, it has been found that the desired effect can be achieved by the combination of the present invention.

When the crystalline cellulose used in the present invention has a large average particle diameter of the crystalline cellulose, gritty feel to the tongue is felt after disintegration in the oral cavity; therefore, from the viewpoint of the easiness of ingestion, the average particle diameter of the crystalline cellulose to be used as a starting material is preferably 150 μm or less, more preferably 120 μm or less, still more preferably 100 μm or less.

As the crystalline cellulose having a "bulk density" of not more than 0.18 $g/cm^3$ in the present invention, a commercially available one, for example, CEOLUS KG-1000 (manufactured by ASAHI KASEI CORPORATION) can be used. While one kind of crystalline cellulose may be used, two or more kinds of crystalline celluloses having different bulk densities and different average particle sizes may be used in combination, such that an assembly thereof has a bulk density of not more than 0.18 $g/cm^3$. For example, the "bulk density" of CEOLUS KG-802 (manufactured by ASAHI KASEI CORPORATION) is 0.22 $g/cm^3$, and a single use thereof for the present invention fails to achieve the effect of the invention, as shown in the following Comparative Examples (21 to 23). However, for example, a 1:1 (weight ratio) mixture with CEOLUS KG-1000 having a bulk density of 0.14 $g/cm^3$ can afford crystalline cellulose having a "bulk density" of not more than 0.18 $g/cm^3$, which affords the desired effect (Example 45). By appropriately mixing in other manner to adjust the bulk density of the assembly, the desired effect can be obtained similarly (Examples 44 and 46). Moreover, two or more different kinds of crystalline celluloses affording a "bulk density" of not more than 0.18 $g/cm^3$ in the present invention by mixing as mentioned above may be used in combination without previous mixing. For example, they may be respectively blended in the present invention by granulation, addition at the final stage and the like without mixing.

From the aspects of hardness and disintegration time, the amount of the above-mentioned crystalline cellulose to be blended is generally 10 to 30 wt%, preferably 15 to 25 wt%, per 100 wt% of the orally disintegrating tablet. An assembly of crystalline cellulose to be blended preferably has a bulk density of not more than 0.18 $g/cm^3$ and a blending ratio of 18 to 30 wt%. When an assembly of crystalline cellulose to be blended has a bulk density of 0.15 $g/cm^3$ or below, the blending ratio is preferably 10 to 30 wt%.

(4) Particular ingredient

The present invention is characterized by containing at least two kinds of particular ingredients selected from the group consisting of low-substituted hydroxypropylcellulose, cornstarch and carmellose. In the combination with the above-mentioned particular crystalline cellulose, a desired effect is obtainable when two or more kinds from the three kinds of particular ingredients are blended. In other words, it was found that when the particular ingredient is not blended or only one kind is blended, a desired effect is not obtainable (see Comparative Examples 1 to 5 and Comparative Examples 6 to 10), and the object of the present invention can be achieved by blending two or three kinds thereof.

(4.1) Low-substituted hydroxypropylcellulose

The low-substituted hydroxypropylcellulose to be used in the present invention is not particularly limited with respect to the substitution ratio of a hydroxypropoxy group, as long as it is compatible with the Japanese Pharmacopoeia. It is generally 7.0 to 12.9%. While the average particle size thereof is not particularly limited, since a gritty feel is left in the oral cavity after disintegration when the average particle size of the low-substituted hydroxypropylcellulose to be used is large, the average particle size of the low-substituted hydroxypropylcellulose to be used as a starting material is preferably 10 to 200 $\mu$m, more preferably 10 to 150 $\mu$m, further preferably 10 to 100 $\mu$m, from the aspect of easiness of ingestion. The low-substituted hydroxypropylcellulose may be appropriately pulverized as necessary to achieve a desired particle size. Examples of the pulverization method include a method using a jet mill or hammer mill. The amount of the low-substituted hydroxypropylcellulose to be blended is generally 1 to 20 wt%, preferably 1 to 16 wt%, more preferably 1 to 10 wt%, further preferably 1 to 6 wt%, per 100 wt% of the orally disintegrating tablet.

(4.2) Cornstarch

While the average particle size of cornstarch to be used in the present invention is not particularly limited, since a gritty feel is left in the oral cavity after disintegration when the average particle size of the cornstarch to be used is large, the average particle size of the cornstarch to be used as a starting material is preferably 10 to 200 $\mu$m, more preferably 10 to 100 $\mu$m, further preferably 10 to 50 $\mu$m, from the aspect of easiness of ingestion. The cornstarch may be appropriately pulverized as necessary to achieve a desired particle size. Examples of the pulverization method include a method using a jet mill or hammer mill. The blending ratio of cornstarch is generally 1 to 20 wt%, preferably 5 to 20 wt%, per 100 wt% of the orally disintegrating tablet.

(4.3) Carmellose

While the carmellose to be used in the present invention is not particularly limited, one compatible with the Japanese Pharmacopoeia is generally used. While the average particle size thereof is not particularly limited, since a gritty feel is left in the oral cavity after disintegration when the average particle size of the carmellose to be used is large, the average particle size of the carmellose to be used as a starting material is preferably 10 to 200 $\mu$m, more preferably 10 to 150 $\mu$m, further preferably 10 to 100 $\mu$m, from the aspect of easiness of ingestion comfortable use. The carmellose may be appropriately pulverized as necessary to achieve a desired particle size. Examples of the pulverization method include a method using a jet mill or hammer mill. The amount of the carmellose to be blended is generally 1 to 20 wt%, preferably 1 to 10 wt%, more preferably 1 to 5 wt%, most preferably 1 to 3 wt%, per 100 wt% of the orally disintegrating tablet.

As for the above-mentioned three kinds of particular ingredients, any two kinds or three kinds may be used, and the total amount thereof to be blended is 3 to 60 wt% per 100 wt% of the orally disintegrating tablet. When two kinds of particular ingredients alone are blended, the total amount thereof to be blended is 3 to 40 wt%. The total blending ratio of (4) particular ingredients is preferably 5 to 40 wt%, more preferably 5 to 35 wt%, whether two kinds thereof are used or 3 kinds thereof are used.

(5) Water-soluble polymer

In the present invention, a water-soluble polymer may be added as a binder for granulation, so as to further improve mixing property or content uniformity. As the water-soluble polymer to be used in the present invention, for example, methylcellulose, hydroxypropylcellulose, polyvinyl alcohol, hypromellose and polyvinylpyrrolidone and the like can be mentioned. Preferred are methylcellulose and hydroxypropylcellulose. A water-soluble polymer is not generally added or, if at all added, the blending ratio is extremely small, since its binding force adversely influences the disintegration property. In the present invention, however, 0.5 to 10 wt% of a water-soluble polymer can be added per 100 wt% of the orally disintegrating tablet. From the aspects of disintegration property, it is preferably 0.5 to 8 wt%, more preferably 0.5 to 5 wt%.

Other ingredients for formulation

The orally disintegrating tablet of the present invention is generally formulated by addition of other formulation ingredients besides the above-mentioned ingredients. The "other formulation ingredient" to be sometimes used in the present invention may be any as long as it does not influence the initial hardness and the hardness after humidification and the disintegration time of the medicament, or influences extremely less frequently and only to the extent that formulation is not interrupted. Examples thereof include excipient, disintegrant, binder, sweetening agent, taste correctives/odor correctives, stabilizer, surfactant, fluidizer, antistatic agent, coating agent, lubricant, colorant, flavor and the like. The amount of the "other formulation ingredient" to be blended is 0.01 to 25 wt% per 100 wt% of the orally disintegrating tablet. When such formulation ingredients are contained in a tablet, the constituting blending ratio is such that the amount thereof to be blended is subtracted from the amount of the above-mentioned ingredients.

Excipient

Examples of the excipient include xylitol, sorbitol, erythritol, trehalose, glucose, white soft sugar, lactose hydrates, calcium sulfate, calcium carbonate and the like.

Disintegrant

Examples of the disintegrant include croscarmellose sodium; sodium carboxymethyl starch; starches such as partly pregelatinized starch, potato starch, rice starch and the like; carmellose sodium, carmellose calcium, crospovidone and the like. The amount of the disintegrant to be blended may be any as long as it does not prevent maintenance of the hardness of the tablet.

Binder

Examples of the binder include gum arabic, gum arabic powder, partially gelatinized starch, gelatin, agar, dextrin, pullulan, povidone, ethylcellulose, carboxymethylethylcellulose, carmellose sodium, hydroxyethylcellulose, hydroxyethylmethylcellulose and the like. The amount of the binder to be blended may be any as long as it does not prevent maintenance of the hardness of the tablet and disintegration in the oral cavity.

Sweetening agent

Examples of the sweetening agent include aspartame, neotame, acesulfame potassium, fructose, reduced maltose syrup, dipotassium glycyrrhizinate, saccharin, saccharin sodium, sucralose, stevia, thaumatin and the like.

Taste correctives/odor correctives

Examples of the taste corrective/odor corrective include various amino acids and salts thereof such as sodium aspartate, alanine, arginine, glycine, glutamine, glutamic acid, glutamic acid hydrochloride, sodium glutamate and the like, organic acids such as adipic acid, ascorbic acid, citric acid, succinic acid, tartaric acid, malic acid and the like, licorice, triethyl citrate, taurine, tannic acid and the like.

Stabilizer

Examples of the stabilizer include sodium edetate, tocopherol and the like.

Surfactant

Examples of the surfactant include sodium lauryl sulfate, polysorbate, hydrogenated oil and the like.

Fluidizer

Examples of the fluidizer include hydrated silicon dioxide, light anhydrous silicic acid, heavy anhydrous silicic acid, titanium dioxide, magnesium alumino metasilicate, calcium silicate and the like.

Antistatic agent

Examples of the antistatic agent include hydrated silicon dioxide, light anhydrous silicic acid, talc and the like.

Coating agent

Examples of the coating agent include ethyl acrylatemethyl methacrylate copolymer dispersion liquids, aminoalkyl methacrylate copolymer, gum arabic powder, ethylcellulose, Opadry, carnauba wax, carboxyvinyl polymer, carboxymethylethylcellulose, carmellose sodium, dry methacrylic acid copolymer, stearyl alcohol, cetanol, shellac, gelatin, hydroxypropylmethylcellulose acetate succinate, pullulan, povidone, hydroxyethylcellulose, hydroxyethylmethylcellulose, polyvinyl alcohol copolymer, dimethylaminoethyl methacrylate-methyl methacrylate copolymer, hydroxypropylmethylcellulose phthalate, fumaric acid-stearic acid-polyvinylacetal diethylaminoacetate-hydroxypropylmethylcellulose mixture, polyvinylacetal diethylaminoacetate, methacrylic acid copolymer, 2-methyl-5-vinylpyridine methylacrylate-methacrylic acid copolymer and the like.

Lubricant

Examples of the lubricant include stearic acid, stearic acid metal salt, sodium stearyl fumarate, sucrose ester of fatty acid, talc, hydrogenated oil, macrogol and the like. Examples of the stearic acid metal salt include magnesium stearate, calcium stearate, aluminum stearate and the like. Among the lubricants, stearic acid or stearic acid metal salt, particularly magnesium stearate, is preferable. The average particle size of the lubricant before formulation is 0.5 to 50 $\mu$m, preferably 1 to 30 $\mu$m. The blending ratio of the lubricant is generally 0.01 to 3.0 wt%, preferably 0.1 to 2.0 wt%, more preferably 0.3 to 1.2 wt%, per 100 wt% of the orally disintegrating tablet.

Colorant

Examples of the colorant include food colors such as Food Color Red No. 3, Food Color Yellow No. 5, and Food Color Blue No. 1, yellow ferric oxide, red ferric oxide, brown iron oxide, black iron oxide, copper chlorophyll, copper chlorophyll sodium, riboflavin, green powdered tea and the like.

Flavor

Examples of the flavor include orange essence, orange oil, caramel, camphor, cassia oil, mint oil such as peppermint and spearmint and the like, strawberry essence, chocolate essence, cherry flavor, spruce oil, pine oil, peppermint oil, vanilla flavor, bitter essence, fruit flavor, yogurt flavor, mint powder and mint flavor such as peppermint and spearmint and the like, mixed flavor, menthol, lemon powder, lemon oil, rose oil and the like.

Preparation method of orally disintegrating tablet

The orally disintegrating tablet of the present invention can be prepared according to a method conventionally used in the pharmaceutical field. It can be obtained by formulating a composition containing each ingredient of the above-mentioned (1) to (4) (and as necessary, ingredient (5)) and, as necessary, the above-mentioned other formulation ingredient by a known means. For example, the above-mentioned various ingredients are uniformly mixed, and the mixture is compression molded by tableting using a conventional tablet press and the like, and the like to give tablets. In addition, the above-mentioned ingredients are mixed (where necessary, granulated to afford granules), a lubricant is added and the mixture is compression molded to give tablets. Alternatively, the active ingredient (1) is pre-treated in advance with a part of each ingredient of (2) to (4), and other formulation ingredients by a known means such as granulation, coating etc., uniformly mixed with the rest of the formulation ingredients, and the mixture is compression molded by a known means to give tablets.

For example, by mixing and forming active ingredient-containing particles which are obtainable by mixing and particulating (1a) an active ingredient, (2a) mannitol and (5a) at least one kind of water-soluble polymer selected from the group consisting of methylcellulose and hydroxypropylcellulose, (2b) mannitol, (3b) crystalline cellulose, (4b) at least two kinds of particular ingredients selected from the group consisting of low-substituted hydroxypropylcellulose, cornstarch and carmellose and, where necessary, (5b) at least one kind of water-soluble polymer selected from the group consisting of methylcellulose, hydroxypropylcellulose, polyvinyl alcohol, hypromellose and polyvinylpyrrolidone, an orally disintegrating tablet can be obtained. The blending ratio relative to 100 wt% of the disintegrating tablet is (1a) 0.01 to 50 wt%, the total of (2a) and (2b) 20 to 86 wt%, (3b) 10 to 30 wt%, each particular ingredient of (4b) 1 to 20 wt% and the total of the particular ingredients to be blended 3 - 60 wt%, and when (5a) and (5b) are blended, the total thereof 0.5 to 10 wt%.

In addition, by forming a mixture of granules which are obtainable by granulating a mixture of (1) an active ingredient, (2b) mannitol, (3b) crystalline cellulose and (4b) at least one kind of particular ingredient selected from the group consisting of low-substituted hydroxypropylcellulose, cornstarch and carmellose and, where necessary, (5b) at least one kind of water-soluble polymer selected from the group consisting of methylcellulose, hydroxypropylcellulose, polyvinyl alcohol, hypromellose and polyvinylpyrrolidone, (3c) crystalline cellulose and (4c) at least one kind of particular ingredient selected from the group consisting of low-substituted hydroxypropylcellulose, cornstarch and carmellose, an orally disintegrating tablet can be obtained. The blending ratio of each ingredient relative to 100 wt% of the disintegrating tablet is (1) 0.01 to 50 wt%, (2b) 20 to 86 wt%, the total of (3b) and (3c) 10 to 30 wt%, and at least two kinds of particular ingredients are contained in (4b) and (4c), the blending ratio of each particular ingredient is 1 to 20 wt% and the total of the particular ingredients to be blended 3 to 60 wt%, and the blending ratio of (5b) when blended is 0.5 to 10 wt%.

The method of shaping the orally disintegrating tablet of the present invention is not particularly limited, and compression molding methods with the use of known tablet press, for example, a rotary tablet press, a single punch tablet press, a press machine and the like is used. Compression force is not particularly limited, as far as it confers sufficient hardness and appropriate disintegration time to the tablet of the present invention, and can be and appropriately determined.

Application to compound A

Particularly, the present invention is preferably applied to the use, as an active ingredient, of 4-amino-5-chloro-2-ethoxy-N-[[4-(4-fluorobenzyl)-2-morpholinyl]methyl]benzamide (compound A) or a pharmaceutically acceptable salt thereof, which is a drug having an uncomfortable taste. The compound (or an acid addition salt thereof and hydrates thereof) are selective serotonin 4 receptor agonists, and show good gastric motility promoting action (see US Patent No. 4,870,074). The compound can be prepared, for example, by the method described in US Patent No. 4,870,074 or a method analogous thereto. The compound A may be a racemate, or one of the optically active forms. The compound may be a free form, or a pharmaceutically acceptable salt thereof. As the salt, an acid addition salt is preferable. For example, an addition salt with organic acid includes formate, acetate, lactate, adipate, citrate, tartrate, fumarate, methanesulfonate, maleate and the like, and an addition salt with inorganic acid includes hydrochloride, sulfate, nitrate, phosphate and the like. Among these, citrate is particularly preferable. Moreover, the active ingredient or a pharmaceutically acceptable salt thereof may also be a solvate, hydrate or non-hydrate. Preferred is a hydrate of citrate, and citrate dihydrate is particularly preferable.

However, since the compound by itself has an uncomfortable taste, masking of the taste thereof is desired to afford an orally disintegrating tablet. As the method therefor, various methods are known, and drug-containing particles which are obtainable by the method described in WO2005/055989 are preferable. That is, compound A or a pharmaceutically acceptable salt thereof, mannitol, and at least one kind of water-soluble polymer selected from the group consisting of methylcellulose and hydroxypropylcellulose are preferably mixed and particulated to give active ingredient-containing particles. The water-soluble polymer is essentially preferably methylcellulose, more preferably methylcellulose and hydroxypropylcellulose. Here, methylcellulose does not completely cover the active ingredient but the active ingredient is also present on the particle surface.

Active ingredient-containing particles

Preferable active ingredient-containing particles using compound A are obtainable by mixing a composition containing (1a) compound A or a pharmaceutically acceptable salt thereof, (2a) mannitol and (5a) at least one kind of water-soluble polymer selected from the group consisting of methylcellulose and hydroxypropylcellulose, and particulating the mixture.

The blending ratio of (1a) compound A or a pharmaceutically acceptable salt thereof contained in the active ingredient-containing particles is the same as that of the above-mentioned (1) the active ingredient, and preferably 0.1 to 10 wt%, preferably about 1 to 8 wt%, more preferably about 1 to 5 wt%, per 100 wt% of the tablet.

As (2a) mannitol necessary for particulation, a part of the above-mentioned (2) mannitol, which is one of the essential ingredients of the present invention, is used. The blending ratio thereof is within the range described in WO2005/055989, preferably about 1 to 30 wt%, more preferably about 1 to 20 wt%, per 100 wt% of the tablet.

(5a) water-soluble polymer necessary for particulation preferably contains at least methylcellulose for masking of an uncomfortable taste. Alternatively, methylcellulose and hydroxypropylcellulose may be used in combination. Other water-soluble polymer may also be added. The amount of (5a) water-soluble polymer to be blended is within the range described in WO2005/055989, preferably about 0.5 to 9.9 wt%, more preferably about 0.5 - 6 wt%, per 100 wt% of the tablet.

The active ingredient-containing particles are obtainable by mixing each ingredient of the above-mentioned (1a), (2a) and (5a), and particulating the mixture. Specifically, for example, they are obtainable by adding water and a water-containing solvent to a mixture of the above, and particulating the mixture. The method also includes mixing each ingredient, adding water or a water-containing solvent thereto and particulating the mixture, or dissolving the whole or a part of methylcellulose (where necessary, hydroxypropylcellulose is further added) in water, adding same to the mixture and particulating the mixture. Moreover, the method also includes mixing each ingredient, adding water or a water-containing solvent, which contains other conventional binder to a level uninfluential on the effect of the present invention, and particulating the mixture. Examples of the particulation method include conventionally-used granulation methods such as an agitation granulation method, an extrusion granulation method, a fluidized bed granulation method, a dry granulation method and the like. The active ingredient-containing particles may further contain corrigent, fluidizer, stabilizer, surfactant, disintegrant, colorant and the like within the particles. Specific examples of these ingredients include those exemplified for the above-mentioned other formulation ingredients.

Orally disintegrating tablet with masking of uncomfortable taste of compound A

By applying the above-mentioned active ingredient-containing particles to the orally disintegrating tablet of the present invention, an orally disintegrating tablet having superior disintegrability and adequate hardness, which masks an uncomfortable taste, maintains rapid oral disintegrability even under moist conditions, and hardness of not less than a predetermined level necessary for packing by an automatic packaging machine can be provided.

That is, an orally disintegrating tablet is obtainable by tableting a mixture of active ingredient-containing particles which are obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose, (2b) mannitol, (3b) crystalline cellulose, at least two kinds of the above-mentioned (4b) particular ingredients and, where necessary, (5b) at least one kind of water-soluble polymer selected from the group consisting of methylcellulose, hydroxypropylcellulose, polyvinyl alcohol, hypromellose and polyvinylpyrrolidone. The preparation method thereof can be the above-mentioned method. The blending ratio relative to 100 wt% of the disintegrating tablet is (1a) 0.1 to 10 wt%, the total of (2a) and (2b) 20 to 86 wt%, (3b) 10 to 30 wt%, each particular ingredient of (4b) 1 to 20 wt% and the total thereof to be blended 3 to 60 wt%, and the total of (5a) and (5b) 0.5 to 10 wt%. In (5b), methylcellulose or hydroxypropylcellulose is preferable.

In general, when the blending ratio of each ingredient contained in a tablet is not less than 1 wt%, the problem of failure to meet the content uniformity of each ingredient in the obtainable orally disintegrating tablet is rare. Nevertheless, when the above-mentioned active ingredient-containing particles are mixed with each ingredient of (2) to (4) at a blending ratio of not less than 1 wt%, it was found that the content uniformity may not be satisfied, though a desired effect of the present invention can be achieved.

Therefore, the present inventors have found that the above-mentioned problem of content uniformity can be solved by granulating the above-mentioned active ingredient-containing particles using a part of ingredients (2) to (4). That is, the problem can be solved by forming a mixture of granules obtainable by granulating a mixture of active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;

(2b) mannitol; (3b) crystalline cellulose; (4b) at least one kind of particular ingredient selected from the group consisting of low-substituted hydroxypropylcellulose, cornstarch and carmellose; and (5b) at least one kind of water-soluble polymer selected from the group consisting of methylcellulose, hydroxypropylcellulose, polyvinyl alcohol, hypromellose and polyvinylpyrrolidone:

(3c) crystalline cellulose: (4c) at least one kind of particular ingredient selected from the group consisting of low-substituted hydroxypropylcellulose, cornstarch and carmellose. The blending ratio relative to 100 wt% of the disintegrating tablet is (1a) 0.1 to 10 wt%, the total of (2a) and (2b) 20 to 86 wt%, the total of (3b) and (3c) 10 to 30 wt%, at least two kinds of particular ingredients are contained in (4b) and (4c), the blending ratio of each particular ingredient is 1 to 20 wt% and the total of the particular ingredients to be blended 3 to 60 wt%, and the total of (5a) and (5b) is 0.5 to 10 wt%.

Particularly, it is preferable to form a mixture of granules obtainable by granulating a mixture of active ingredient-containing particles obtainable by mixing and particulating (1a) compound A or a pharmaceutically acceptable salt thereof, (2a) mannitol and (5a) at least one kind of water-soluble polymer selected from the group consisting of methylcellulose and hydroxypropylcellulose;

(2b) mannitol; (3b) crystalline cellulose; (4.1b) low-substituted hydroxypropylcellulose; (4.3b) carmellose; and (5b) at least one kind of water-soluble polymer selected from the group consisting of methylcellulose and hydroxypropylcellulose:

(3c) crystalline cellulose: (4.1c) low-substituted hydroxypropylcellulose: and (4.2c) cornstarch. The blending ratio relative to 100 wt% of the disintegrating tablet is (1a) 0.1 to 10 wt%, the total of (2a) and (2b) 20 to 86 wt%, the total of (3b) and (3c) 10 to 30 wt%, the total of (4.1b) and (4.1c) 1 to 20 wt%, (4.2c) 1 to 20 wt%, (4.3b) 1 to 20 wt% and the total of (5a) and (5b) is 0.5 to 10 wt%. Preferably, the total of (2a) and (2b) is 20 to 75 wt%, (more preferably 30 to 75 wt%), and (4.2c) is 5 to 20 wt%.

The blending ratios of (3b) crystalline cellulose and particular ingredients (4.1b), (4.2b) and (4.3b) to be used for granules can be freely adjusted as long as the above-mentioned conditions are met. The weight ratio of crystalline celluloses (3b) and (3c) is preferably 1:0.5 to 1:2. Low-substituted hydroxypropylcellulose (4.1b) and carmellose (4.3b) can be freely selected. Cornstarch (4.2b) is preferably added as a powder rather than as granules, which affords easiness of ingestion as mentioned below. The bulk density of an assembly of (3b) and (3c) is as described in the aforementioned (3).

Orally disintegrating tablet

The thus-obtainable orally disintegrating tablet of the present invention means one showing rapid disintegration property in the oral cavity without ingesting water for taking a formulation. Specifically, the orally disintegrating tablet of the present invention is a formulation that is disintegrated in the oral cavity mainly with saliva in about 40 sec, generally within 40 sec, preferably within 30 sec.

In addition, the orally disintegrating tablet of the present invention has sufficient hardness to avoid crack and breaking during ordinary operation. The ordinary operation includes transport and storage in a package state, picking up from PTP, one dose packaging by an automatic packaging machine, transport and storage in a one dose package state and the like. Specifically, the orally disintegrating tablet of the present invention has an absolute hardness of 2.0N/mm$^2$ or above, preferably 3.0N/mm$^2$ or above. When the tablet has a separating line, the absolute hardness thereof is 2.0 to 10.0N/mm$^2$, preferably 2.5 to 7.0N/mm$^2$, in consideration of easiness of splitting.

The absolute hardness is a value obtained by dividing the hardness measured by a tablet hardness meter by a cross-sectional area of tablet divided into two along the longitudinal direction (for example, in the case of a flat tablet, tablet diameter (mm) ×tablet thickness (mm)), which is shown by the following formula.

$$\text{absolute hardness } (N/mm^2) = \text{hardness } (N)/\text{cross-sectional area } (mm^2)$$

Furthermore, the orally disintegrating tablet of the present invention can maintain rapid disintegration property and hardness even when stored under moist conditions. For example, the oral disintegration time of the orally disintegrating tablet of the present invention after preservation under the conditions of 25°C, relative humidity 75% (75% RH) for 3 days does not differ from that of initial (for example, difference in disintegration time before and after preservation is within 5 sec), and the absolute hardness can be maintained at generally 1.5N/mm$^2$ or above, preferably 1.6N/mm2 or above.

Moreover, easiness of ingestion of an orally disintegrating tablet is an important factor for the patients under medication, and it is important that the tablet start to disintegrate early with saliva without the feeling of water content of saliva being absorbed by the tablet in the oral cavity, without swelling of the tablet in the oral cavity, and without tasting sour and the like due to the ingredients. In other words, the orally disintegrating tablet of the present invention is characterized in that it is free of powdery feeling on ingestion (easy absorption of water content in the mouth by the tablet, long time required to achieve a suspension state, and comparatively large amount of remaining powder), bloated feeling (swelling of tablet due to absorption of water content in the mouth by the tablet, which is irrelevant to tablet disintegration) and acid taste (taste sour in the mouth), and initial disintegration property is good (see Tables 55 - 62).

Among the formulations meeting the preferable ranges of hardness and disintegration time after the aforementioned humidification in the present invention, when the above-mentioned easiness of ingestion is taken into consideration, the preferable range of numerical value of each ingredient of (3) and (4) in the present invention is as shown below. That is, the preferable range of numerical value per 100 wt% of the orally disintegrating tablet is (3) crystalline cellulose having a particular bulk density 15 to 25 wt%, (4.1) low-substituted hydroxypropylcellulose 1 to 6 wt%, (4.2) cornstarch 5 to 20 wt%, and (4.3) carmellose 1 to 5 wt%, more preferably 1 to 3 wt%. When the active ingredient-containing particles containing compound A are used for orally disintegrating tablets, three kinds of the above-mentioned particular ingredients (4.1), (4.2) and (4.3) are preferably contained. In a different case, high easiness of ingestion can also be obtained by using two or three kinds thereof.

Examples

The present invention is explained in more detail in the following by referring to Examples, which are not to be construed as limitative.

As the active ingredient of (1), the following 5 kinds were used. That is, racemate of compound A was defined as compound A-a and citrate of compound A-a was defined as compound A-b (active ingredient), and A-b·dihydrate was used (manufactured by Dainippon Sumitomo Pharma Co., Ltd., average particle size about 5 μm). As caffeine, anhydrous caffeine manufactured by BASF was used, as acetaminophen, one manufactured by YAMAMOTO CORPORATION CO., LTD. was used, as zonisamide, one manufactured by Dainippon Sumitomo Pharma Co., Ltd. (average particle size about 7 μm) was used, and as cimetidine, one manufactured by Sumitomo Chemical Co., Ltd. was used.

As mannitol of (2), the following 14 kinds were used. To be specific, unless particularly specified in the Examples and Tables, "mannitol" means PEARLITOL 160C (average particle size about 60 μm) of ROQUETTE Corporation, which is D-mannitol, mannitol 50C means PEARLITOL 50C (average particle size about 30 μm) of ROQUETTE Corporation, which is D-mannitol, and mannitol 25C means PEARLITOL 25C of ROQUETTE Corporation, which is D-mannitol. In addition, mannitol S means mannit S (average particle size about 70 μm) of TOWA-KASEI Co., Ltd., which is D-mannitol, and mannitol P means mannit P (average particle size about 30 μm) of TOWA-KASEI Co., Ltd., which is D-mannitol. Mannitol M means Parteck delta M of Merck & Co., Inc, which is D-mannitol. Mannitol 100SD means PEARLITOL 100SD of ROQUETTE Corporation, which is D-mannitol, mannitol 200SD means PEARLITOL 200SD of ROQUETTE Corporation, which is D-mannitol, mannitol Marine Crystal means Marine Crystal of Mitsubishi Shoji Foodtech Co., Ltd., which is D-mannitol, and mannitol 108 means Nonpareil-108 of Freund Corporation, which is D-mannitol. Mannitol M100 means Parteck M100 of Merck & Co., Inc., which is D-mannitol, mannitol M200 means Parteck M200 of Merck & Co., Inc., which is D-mannitol, and mannitol M300 means Parteck M300 of Merck & Co., Inc., which is D-mannitol. Mannitol 1.059 means mannitol 1.05980 of Merck & Co., Inc., which is D-mannitol.

As crystalline cellulose of (3), the following were used. Crystalline cellulose KG-1000 means CEOLUS KG-1000 (bulk density, 0.14 g/cm$^3$) of ASAHI KASEI CORPORATION, crystalline cellulose PH-101 means CEOLUS PH-101 (bulk density, 0.31 g/cm$^3$) of ASAHI KASEI CORPORATION, and crystalline cellulose KG-802 means CEOLUS KG-802 (bulk density, 0.22 g/cm$^3$) of ASAHI KASEI CORPORATION.

As for the particular ingredients of (4), as low-substituted hydroxypropylcellulose, the following were used. Unless particularly specified in the Examples and Tables, "low-substituted hydroxypropylcellulose" means LH-21 of Shin-Etsu Chemical Co., Ltd., low-substituted hydroxypropylcellulose 22 means LH-22 of Shin-Etsu Chemical Co., Ltd., low-substituted hydroxypropylcellulose 32 means LH-32 of Shin-Etsu Chemical Co., Ltd., low-substituted hydroxypropylcellulose 11 means LH-11 of Shin-Etsu Chemical Co., Ltd., and low-substituted hydroxypropylcellulose 31 means LH-31 of Shin-Etsu Chemical Co., Ltd.. As carmellose, carmellose NS-300 of Gotoku Chemical Company Ltd. was used, and as cornstarch, cornstarch (XX16)W of Nihon Shokuhinkako Co., Ltd. was used.

As crospovidone, Kollidon CL of BASF Japan was used, as carmellose sodium, Serogen PR-S of San-Ei Gen F.F.I., Inc. was used, as carmellose calcium, ECG-505 of Gotoku Chemical Company Ltd. was used, as sodium carboxymethyl starch, Primojel of GOKYO TRADING CO., LTD. was used, and as croscarmellose sodium, Ac-Di-Sol of ASAHI KASEI CHEMICALS CORPORATION was used. As magnesium stearate, magnesium stearate (plant-derived) of Taihei Chemical Industrial Co., Ltd. was used, as methylcellulose, Metolose SM-25 (viscosity 2.53 mm$^2$/s (2% water-soluble viscosity at 20°C (the Japanese Pharmacopoeia))) of Shin-Etsu Chemical Co., Ltd. was used, and as hydroxypropylcellulose, HPC-L of NIPPON SODA CO., LTD. was used. As light anhydrous silicic acid, AEROSIL 200 manufactured by NIPPON AEROSIL was used, as aspartame, aspartame of Ajinomoto Co., Inc. was used, as stevia, stevi MZ of Maruzen Pharmaceuticals Co., Ltd. was used, as spearmint, spearmint Micron 20 of TAKASAGO INTERNATIONAL CORPORATION was used, and as menthol, menthol coaton DL47155 of Ogawa & Co., Ltd. was used. As finely granulated lactose, lactose G manufactured by Freund Corporation was used, as yellow ferric oxide, yellow ferric oxide manufactured by Kishi Kasei Co., Ltd. was used, and as anhydride citric acid, anhydride citric acid of Nacalai Tesque was used.

Comparative Examples 1 - 10

[Table 1]

Table 1 orally disintegrating tablet (Comparative Examples 1 - 10) formulation (amount blended) (unit: g)

| ingredient | | Comparative Example | | | | | | | | | |
|---|---|---|---|---|---|---|---|---|---|---|---|
| | | 1 | 2 | 3 | 4 | 5 | 6 | 7 | 8 | 9 | 10 |
| (1) | compound A-b·dihydrate | 0.529 | | | | | | | | | |
| | (as compound A-b) | (0.5) | | | | | | | | | |
| (2) | mannitol | 17.271 | 15.271 | 11.271 | 7.271 | 3.271 | 14.471 | 13.671 | 13.671 | 13.271 | 11.271 |
| (3) | crystalline cellulose KG-1000 | 2 | 4 | 8 | 12 | 16 | 4 | 4 | 4 | 4 | 4 |
| (4) | carmellose | - | | | | | 0.8 | 1.6 | - | - | - |
| | low-substituted hydroxypropylcellulose | - | | | | | - | - | 1.6 | - | - |
| | cornstarch | - | | | | | - | - | - | 2 | 4 |
| | magnesium stearate | 0.2 | | | | | | | | | |
| | total | 20 | | | | | | | | | |

[Table 2]

Table 2 orally disintegrating tablet (Comparative Examples 1 - 10) blending ratio (unit: wt%)

| ingredient | | Comparative Example | | | | | | | | | |
|---|---|---|---|---|---|---|---|---|---|---|---|
| | | 1 | 2 | 3 | 4 | 5 | 6 | 7 | 8 | 9 | 10 |
| (1) | compound A-b·dihydrate | 2.645 | | | | | | | | | |
| | (as compound A-b) | (2.5) | | | | | | | | | |
| (2) | mannitol | 86.355 | 76.355 | 56.355 | 36.355 | 16.355 | 72.355 | 68.355 | 68.355 | 66.355 | 56.355 |
| (3) | crystalline cellulose KG-1000 | 10 | 20 | 40 | 60 | 80 | 20 | 20 | 20 | 20 | 20 |
| (4) | carmellose | - | | | | | 4 | 8 | - | - | - |
| | low-substituted hydroxypropylcellulose | - | | | | | - | - | 8 | - | - |
| | cornstarch | - | | | | | - | - | - | 10 | 20 |
| | magnesium stearate | 1 | | | | | | | | | |
| | total | 100 | | | | | | | | | |

According to the formulations described in Table 1, orally disintegrating tablets having blending ratios shown in Table 2 were prepared. That is, respective ingredients other than magnesium stearate, which constitute the orally disintegrating tablet, were mixed. Then, magnesium stearate was added to the mixture, and the obtained mixture was tableted by a tablet press (manufactured by RIKEN, hydraulic press machine) to give tablets (weight 200 mg, diameter 8 mm per tablet). During compression, the compression force was adjusted such that the oral disintegration time was 25 sec $\pm$5 sec. The compression force at that time is shown in Table 52 in the Experimental Example below.

The orally disintegrating tablet is different from that of the present invention in that it does not contain the particular ingredient of (4) in the present invention, or contains only one kind thereof.

Examples 1 - 8

<When carmellose and cornstarch are blended as particular ingredients of (4)>

[Table 3]

Table 3 orally disintegrating tablet (Examples 1 - 8) formulation (amount blended) (unit: g)

| | ingredient | Example | | | | | | | |
|---|---|---|---|---|---|---|---|---|---|
| | | 1 | 2 | 3 | 4 | 5 | 6 | 7 | 8 |
| (1) | compound A-b·dihydrate | 0.529 | | | | | | | |
| | (as compound A-b) | (0.5) | | | | | | | |
| (2) | mannitol | 14.571 | 14.071 | 14.371 | 13.871 | 12.871 | 12.271 | 11.271 | 9.271 |
| (3) | crystalline cellulose KG-1000 | 4 | | | | | | | |
| (4) | carmellose | 0.2 | 0.2 | 0.4 | 0.4 | 0.4 | 1 | 2 | 2 |
| | low-substituted hydroxypropylcellulose | - | | | | | | | |
| | cornstarch | 0.5 | 1 | 0.5 | 1 | 2 | 2 | 2 | 4 |
| | magnesium stearate | 0.2 | | | | | | | |
| | total | 20 | | | | | | | |

[Table 4]

Table 4 orally disintegrating tablet (Examples 1 - 8) blending ratio (unit: wt%)

| | ingredient | Example | | | | | | | |
|---|---|---|---|---|---|---|---|---|---|
| | | 1 | 2 | 3 | 4 | 5 | 6 | 7 | 8 |
| (1) | compound A-b·dihydrate | 2.645 | | | | | | | |
| | (as compound A-b) | (2.5) | | | | | | | |
| (2) | mannitol | 72.855 | 70.355 | 71.855 | 69.355 | 64.355 | 61.355 | 56.355 | 46.355 |
| (3) | crystalline cellulose KG-1000 | 20 | | | | | | | |
| (4) | carmellose | 1 | 1 | 2 | 2 | 2 | 5 | 10 | 10 |
| | low-substituted hydroxypropylcellulose | - | | | | | | | |
| | cornstarch | 2.5 | 5 | 2.5 | 5 | 10 | 10 | 10 | 20 |
| | magnesium stearate | 1 | | | | | | | |

(continued)

| ingredient | | Example | | | | | | | |
|---|---|---|---|---|---|---|---|---|---|
| | | 1 | 2 | 3 | 4 | 5 | 6 | 7 | 8 |
| | total | 100 | | | | | | | |

According to the formulations described in Table 3, orally disintegrating tablets having blending ratios shown in Table 4 were prepared. That is, respective ingredients other than magnesium stearate, which constitute the orally disintegrating tablet, were mixed. Then, magnesium stearate was added to the mixture, and the obtained mixture was tableted by a tablet press (manufactured by RIKEN, hydraulic press machine) to give tablets (weight 200 mg, diameter 8 mm per tablet). During compression, the compression force was adjusted such that the oral disintegration time was 25 sec ± 5 sec. The compression force at that time is shown in Table 47 in the Experimental Example below.

Examples 9 - 16

<When low-substituted hydroxypropylcellulose and cornstarch are blended as particular ingredients of (4)>

[Table 5]

Table 5 orally disintegrating tablet (Examples 9 - 16) formulation (amount blended) (unit: g)

| ingredient | | Example | | | | | | | |
|---|---|---|---|---|---|---|---|---|---|
| | | 9 | 10 | 11 | 12 | 13 | 14 | 15 | 16 |
| (1) | compound A-b·dihydrate | 0.529 | | | | | | | |
| | (as compound A-b) | (0.5) | | | | | | | |
| (2) | mannitol | 14.571 | 14.071 | 13.871 | 12.871 | 12.471 | 11.671 | 10.671 | 9.071 |
| (3) | crystalline cellulose KG-1000 | 4 | | | | | | | |
| (4) | carmellose | - | | | | | | | |
| | low-substituted hydroxypropylcellulose | 0.2 | 0.2 | 0.4 | 0.4 | 0.8 | 1.6 | 1.6 | 3.2 |
| | cornstarch | 0.5 | 1 | 1 | 2 | 2 | 2 | 3 | 3 |
| | magnesium stearate | 0.2 | | | | | | | |
| | total | 20 | | | | | | | |

[Table 6]

Table 6 orally disintegrating tablet (Examples 9 - 16) blending ratio (unit: wt%)

| ingredient | | Example | | | | | | | |
|---|---|---|---|---|---|---|---|---|---|
| | | 9 | 10 | 11 | 12 | 13 | 14 | 15 | 16 |
| (1) | compound A-b·dihydrate | 2.645 | | | | | | | |
| | (as compound A-b) | (2.5) | | | | | | | |
| (2) | mannitol | 72.855 | 70.355 | 69.355 | 64.355 | 62.355 | 58.355 | 53.355 | 45.355 |
| (3) | crystalline cellulose KG-1000 | 20 | | | | | | | |

(continued)

| ingredient | | Example | | | | | | | |
|---|---|---|---|---|---|---|---|---|---|
| | | 9 | 10 | 11 | 12 | 13 | 14 | 15 | 16 |
| (4) | carmellose | - | | | | | | | |
| | low-substituted hydroxypropylcellulose | 1 | 1 | 2 | 2 | 4 | 8 | 8 | 16 |
| | cornstarch | 2.5 | 5 | 5 | 10 | 10 | 10 | 15 | 15 |
| | magnesium stearate | 1 | | | | | | | |
| | total | 100 | | | | | | | |

In the same manner as in Examples 1 - 8 and according to the formulations shown in Table 5, orally disintegrating tablets having blending ratios shown in Table 6 were prepared. The compression force at that time is shown in Table 47 in the Experimental Example below.

Examples 17 - 23

<When low-substituted hydroxypropylcellulose and carmellose are blended as particular ingredients of (4)>

[Table 7]

Table 7 orally disintegrating tablet (Examples 17 - 23) formulation (amount blended) (unit: g)

| ingredient | | Example | | | | | | |
|---|---|---|---|---|---|---|---|---|
| | | 17 | 18 | 19 | 20 | 21 | 22 | 23 |
| (1) | compound A-b·dihydrate | 0.529 | | | | | | |
| | (as compound A-b) | (0.5) | | | | | | |
| (2) | mannitol | 14.671 | 14.271 | 14.471 | 14.071 | 12.871 | 10.471 | 8.871 |
| (3) | crystalline cellulose KG-1000 | 4 | | | | | | |
| (4) | carmellose | 0.2 | 0.2 | 0.4 | 0.4 | 0.8 | 1.6 | 3.2 |
| | low-substituted hydroxypropylcellulose | 0.4 | 0.8 | 0.4 | 0.8 | 1.6 | 3.2 | 3.2 |
| | cornstarch | - | | | | | | |
| | magnesium stearate | 0.2 | | | | | | |
| | total | 20 | | | | | | |

[Table 8]

Table 8 orally disintegrating tablet (Examples 17 - 23) blending ratio (unit: wt%)

| ingredient | | Example | | | | | | |
|---|---|---|---|---|---|---|---|---|
| | | 17 | 18 | 19 | 20 | 21 | 22 | 23 |
| (1) | compound A-b·dihydrate | 2.645 | | | | | | |
| | (as compound A-b) | (2.5) | | | | | | |
| (2) | mannitol | 73.355 | 71.355 | 72.355 | 70.355 | 64.355 | 52.355 | 44.355 |
| (3) | crystalline cellulose KG-1000 | 20 | | | | | | |

(continued)

| ingredient | | Example | | | | | | |
|---|---|---|---|---|---|---|---|---|
| | | 17 | 18 | 19 | 20 | 21 | 22 | 23 |
| (4) | carmellose | 1 | 1 | 2 | 2 | 4 | 8 | 16 |
| | low-substituted hydroxypropylcellulose | 2 | 4 | 2 | 4 | 8 | 16 | 16 |
| | cornstarch | - | | | | | | |
| | magnesium stearate | 1 | | | | | | |
| | total | 100 | | | | | | |

In the same manner as in Examples 1 - 8 and according to the formulations shown in Table 7, orally disintegrating tablets having blending ratios shown in Table 8 were prepared. The compression force at that time is shown in Table 47 in the Experimental Example below.

Comparative Examples 11 - 13 and Examples 24 - 39

<Blending rate of total particular ingredients of (4)>

[Table 9]

Table 9 orally disintegrating tablet (Comparative Examples 11 - 13 and Examples 24 - 27) formulation (amount blended) (unit: g)

| ingredient | | Comparative Example | | | Example | | | |
|---|---|---|---|---|---|---|---|---|
| | | 11 | 12 | 13 | 24 | 25 | 26 | 27 |
| (1) | compound A-b·dihydrate | 0.529 | | | | | | |
| | (as compound A-b) | (0.5) | | | | | | |
| (2) | mannitol | 14.871 | 14.871 | 14.871 | 14.671 | 13.671 | 13.971 | 13.471 |
| (3) | crystalline cellulose KG-1000 | 4 | | | | | | |
| (4) | carmellose | 0.2 | - | 0.2 | 0.2 | 0.2 | 0.4 | 0.4 |
| | low-substituted hydroxypropylcellulose | - | 0.2 | 0.2 | 0.2 | 0.4 | 0.4 | 0.4 |
| | cornstarch | 0.2 | 0.2 | - | 0.2 | 1 | 0.5 | 1 |
| | magnesium stearate | 0.2 | | | | | | |
| | total | 20 | | | | | | |

[Table 10]

Table 10 orally disintegrating tablet (Comparative Examples 11 - 13 and Examples 24 - 27) blending ratio (unit: wt%)

| ingredient | | Comparative Example | | | Example | | | |
|---|---|---|---|---|---|---|---|---|
| | | 11 | 12 | 13 | 24 | 25 | 26 | 27 |
| (1) | compound A-b·dihydrate | 2.645 | | | | | | |
| | (as compound A-b) | (2.5) | | | | | | |
| (2) | mannitol | 74.355 | 74.355 | 74.355 | 73.355 | 68.355 | 69.855 | 67.355 |
| (3) | crystalline cellulose KG-1000 | 20 | | | | | | |

(continued)

| ingredient | | Comparative Example | | | Example | | | |
|---|---|---|---|---|---|---|---|---|
| | | 11 | 12 | 13 | 24 | 25 | 26 | 27 |
| (4) | carmellose | 1 | - | 1 | 1 | 1 | 2 | 2 |
| | low-substituted hydroxypropylcellulose | - | 1 | 1 | 1 | 2 | 2 | 2 |
| | cornstarch | 1 | 1 | - | 1 | 5 | 2.5 | 5 |
| | magnesium stearate | 1 | | | | | | |
| | total | 100 | | | | | | |

[Table 11]

Table 11 orally disintegrating tablet (Examples 28 - 33) formulation (amount blended) (unit: g)

| ingredient | | Example | | | | | |
|---|---|---|---|---|---|---|---|
| | | 28 | 29 | 30 | 31 | 32 | 33 |
| (1) | compound A-b·dihydrate | 0.529 | | | | | |
| | (as compound A-b) | (0.5) | | | | | |
| (2) | mannitol | 12.271 | 13.071 | 12.471 | 12.071 | 9.671 | 10.871 |
| (3) | crystalline cellulose KG-1000 | 4 | | | | | |
| (4) | carmellose | 0.2 | 0.4 | 0.4 | 0.4 | 0.8 | 0.8 |
| | low-substituted hydroxypropylcellulose | 0.8 | 0.8 | 0.4 | 0.8 | 0.8 | 1.6 |
| | cornstarch | 2 | 1 | 2 | 2 | 4 | 2 |
| | magnesium stearate | 0.2 | | | | | |
| | total | 20 | | | | | |

[Table 12]

Table 12 orally disintegrating tablet (Examples 28 - 33) blending ratio (unit: wt%)

| ingredient | | Example | | | | | |
|---|---|---|---|---|---|---|---|
| | | 28 | 29 | 30 | 31 | 32 | 33 |
| (1) | compound A-b·dihydrate | 2.645 | | | | | |
| | (as compound A-b) | (2.5) | | | | | |
| (2) | mannitol | 61.355 | 65.355 | 62.355 | 60.355 | 48.355 | 54.355 |
| (3) | crystalline cellulose KG-1000 | 20 | | | | | |
| (4) | carmellose | 1 | 2 | 2 | 2 | 4 | 4 |
| | low-substituted hydroxypropylcellulose | 4 | 4 | 2 | 4 | 4 | 8 |
| | cornstarch | 10 | 5 | 10 | 10 | 20 | 10 |
| | magnesium stearate | 1 | | | | | |
| | total | 100 | | | | | |

[Table 13]

Table 13 orally disintegrating tablet (Examples 34 - 39) formulation (amount blended) (unit: g)

| ingredient | | Example | | | | | |
|---|---|---|---|---|---|---|---|
| | | 34 | 35 | 36 | 37 | 38 | 39 |
| (1) | compound A-b·dihydrate | 0.529 | | | | | |
| | (as compound A-b) | (0.5) | | | | | |
| (2) | mannitol | 8.871 | 10.071 | 8.071 | 8.471 | 6.471 | 4.871 |
| (3) | crystalline cellulose KG-1000 | 4 | | | | | |
| (4) | carmellose | 0.8 | 1.6 | 1.6 | 1.6 | 1.6 | 3.2 |
| | low-substituted hydroxypropylcellulose | 1.6 | 1.6 | 1.6 | 3.2 | 3.2 | 3.2 |
| | cornstarch | 4 | 2 | 4 | 2 | 4 | 4 |
| | magnesium stearate | 0.2 | | | | | |
| | total | 20 | | | | | |

[Table 14]

Table 14 orally disintegrating tablet (Examples 34 - 39) blending ratio (unit: wt%)

| ingredient | | Example | | | | | |
|---|---|---|---|---|---|---|---|
| | | 34 | 35 | 36 | 37 | 38 | 39 |
| (1) | compound A-b·dihydrate | 2.645 | | | | | |
| | (as compound A-b) | (2.5) | | | | | |
| (2) | mannitol | 44.355 | 50.355 | 40.355 | 42.355 | 32.355 | 24.355 |
| (3) | crystalline cellulose KG-1000 | 20 | | | | | |
| (4) | carmellose | 4 | 8 | 8 | 8 | 8 | 16 |
| | low-substituted hydroxypropylcellulose | 8 | 8 | 8 | 16 | 16 | 16 |
| | cornstarch | 20 | 10 | 20 | 10 | 20 | 20 |
| | magnesium stearate | 1 | | | | | |
| | total | 100 | | | | | |

In the same manner as in Examples 1 - 8 and according to the formulations shown in Table 9, Table 11 and Table 13, orally disintegrating tablets having blending ratios shown in Table 10, Table 12 and Table 14 were prepared. The orally disintegrating tablets of Comparative Examples 11 - 13 are different from that of the present invention in that the total of the blending ratios of particular ingredients of (4) is 2 wt% and does not satisfy the range of the present invention. The compression force at that time is shown in Table 48 and Table 52 in the Experimental Example below.

Comparative Examples 14 - 17 and Examples 40 - 43

<Blending rate of crystalline cellulose>

[Table 15]

28

Table 15 orally disintegrating tablet (Comparative Examples 14 - 17 and Examples 40 - 43) formulation (amount blended) (unit:g)

| | ingredient | Comparative Example | | | | Example | | | |
|---|---|---|---|---|---|---|---|---|---|
| | | 14 | 15 | 16 | 17 | 40 | 41 | 42 | 43 |
| (1) | compound A-b·dihydrate | 0.529 | | | | | | | |
| | (as compound A-b) | (0.5) | | | | | | | |
| (2) | mannitol | 13.871 | 6.871 | 11.871 | 4.871 | 12.871 | 8.871 | 10.871 | 6.871 |
| (3) | crystalline cellulose KG-1000 | 1 | 8 | 1 | 8 | 2 | 6 | 2 | 6 |
| (4) | carmellose | 0.8 | 0.8 | 0.8 | 0.8 | 0.8 | 0.8 | 0.8 | 0.8 |
| | low-substituted hydroxypropylcellulose | 1.6 | 1.6 | 1.6 | 1.6 | 1.6 | 1.6 | 1.6 | 1.6 |
| | cornstarch | 2 | 2 | 4 | 4 | 2 | 2 | 4 | 4 |
| | magnesium stearate | 0.2 | | | | | | | |
| | total | 20 | | | | | | | |

[Table 16]

Table 16 orally disintegrating tablet (Comparative Examples 14 - 17 and Examples 40 - 43) blending ratio (unit: wt%)

| | ingredient | Comparative Example | | | | Example | | | |
|---|---|---|---|---|---|---|---|---|---|
| | | 14 | 15 | 16 | 17 | 40 | 41 | 42 | 43 |
| (1) | compound A-b·dihydrate | 2.645 | | | | | | | |
| | (as compound A-b) | (2.5) | | | | | | | |
| (2) | mannitol | 69.355 | 34.355 | 59.355 | 24.355 | 64.355 | 44.355 | 54.355 | 34.355 |
| (3) | crystalline cellulose KG-1000 | 5 | 40 | 5 | 40 | 10 | 30 | 10 | 30 |
| (4) | carmellose | 4 | 4 | 4 | 4 | 4 | 4 | 4 | 4 |
| | low-substituted hydroxypropylcellulose | 8 | 8 | 8 | 8 | 8 | 8 | 8 | 8 |
| | cornstarch | 10 | 10 | 20 | 20 | 10 | 10 | 20 | 20 |
| | magnesium stearate | 1 | | | | | | | |
| | total | 100 | | | | | | | |

In the same manner as in Examples 1 - 8 and according to the formulations shown in Table 15, orally disintegrating tablets having blending ratios shown in Table 16 were prepared. The orally disintegrating tablets of Comparative Examples 14 - 17 are different from that of the present invention in that the blending ratio of the crystalline cellulose of (3) is 5 wt% or 40 wt% and does not satisfy the range of the present invention. The compression force at that time is shown in Table 48 and Table 52 in the Experimental Example below.

Examples 44 - 46 and Comparative Examples 18 - 23

<Bulk density of crystalline cellulose (3) to be blended>

[Table 17]

Table 17 orally disintegrating tablet (Examples 44 - 46 and Comparative Examples 18 - 23) formulation (amount blended) (unit: g)

| | ingredient | Example | | | Comparative Example | | | | | |
|---|---|---|---|---|---|---|---|---|---|---|
| | | 44 | 45 | 46 | 18 | 19 | 20 | 21 | 22 | 23 |
| (1) | compound A-b·dihydrate | 0.529 | | | | | | | | |
| | (as compound A-b) | (0.5) | | | | | | | | |
| (2) | mannitol | 10.871 | 10.871 | 8.871 | 12.071 | 10.871 | 10.071 | 12.071 | 10.871 | 10.071 |
| (3) | crystalline cellulose KG-1000 | 3 | 2 | 3 | - | - | - | - | - | - |
| | crystalline cellulose PH-101 | - | - | 1 | 4 | 4 | 4 | - | - | - |
| | crystalline cellulose KG-802 | 1 | 2 | - | - | - | - | 4 | 4 | 4 |
| (4) | carmellose | 0.8 | 0.8 | 0.8 | 0.4 | 0.8 | 1.6 | 0.4 | 0.8 | 1.6 |
| | low-substituted hydroxypropylcellulose | 1.6 | 1.6 | 1.6 | 0.8 | 1.6 | 1.6 | 0.8 | 1.6 | 1.6 |
| | cornstarch | 2 | 2 | 4 | 2 | 2 | 2 | 2 | 2 | 2 |
| | magnesium stearate | 0.2 | | | | | | | | |
| | total | 20 | | | | | | | | |

[Table 18]

Table 18 orally disintegrating tablet (Examples 44 - 46 and Comparative Examples 18 - 23) blending ratio (unit: wt%)

| ingredient | | Example | | | Comparative Example | | | | | |
|---|---|---|---|---|---|---|---|---|---|---|
| | | 44 | 45 | 46 | 18 | 19 | 20 | 21 | 22 | 23 |
| (1) | compound A-b·dihydrate | 2.645 | | | | | | | | |
| | (as compound A-b) | (2.5) | | | | | | | | |
| (2) | mannitol | 54.355 | 54.355 | 44.355 | 60.355 | 54.355 | 50.355 | 60.355 | 54.355 | 50.355 |
| (3) | crystalline cellulose KG-1000 | 15 | 10 | 15 | - | - | - | - | - | - |
| | crystalline cellulose PH-101 | - | - | 5 | 20 | 20 | 20 | - | - | - |
| | crystalline cellulose KG-802 | 5 | 10 | - | - | - | - | 20 | 20 | 20 |
| (4) | carmellose | 4 | 4 | 4 | 2 | 4 | 8 | 2 | 4 | 8 |
| | low-substituted hydroxypropylcellulose | 8 | 8 | 8 | 4 | 8 | 8 | 4 | 8 | 8 |
| | cornstarch | 10 | 10 | 20 | 10 | 10 | 10 | 10 | 10 | 10 |
| | magnesium stearate | 1 | | | | | | | | |
| | total | 100 | | | | | | | | |

Using crystalline celluloses having different bulk densities, in the same manner as in Examples 1 - 8 and according to the formulations shown in Table 17, orally disintegrating tablets having blending ratios shown in Table 18 were prepared. To be specific, in Examples 44 - 46, crystalline cellulose KG-1000 having a bulk density of 0.14 $g/cm^3$ was mixed with crystalline cellulose PH-101 having a bulk density of 0.31 $g/cm^3$ or crystalline cellulose KG-802 having a bulk density of 0.22 $g/cm^3$, and the bulk density of an assembly of crystalline celluloses to be blended was adjusted to 0.17 $g/cm^3$ (Example 44), 0.18 $g/cm^3$ (Example 45) and 0.17 $g/cm^3$ (Example 46). Using the assembly, orally disintegrating tablets were prepared. The orally disintegrating tablets of Comparative Examples 18 - 23 are different from that of the present invention in that (3) crystalline cellulose to be blended has a bulk density of 0.31 $g/cm^3$ or 0.22 $g/cm^3$ and does not satisfy the range of the present invention. The compression force at that time is shown in Table 48 and Table 52 in the Experimental Example below.

Examples 47 - 50

<Kinds of mannitol>

[Table 19]

Table 19 orally disintegrating tablet (Examples 47 - 50) formulation (amount blended) and blending ratio

| ingredient | | amount blended (g) | | | | blending ratio (wt%) | | | |
| --- | --- | --- | --- | --- | --- | --- | --- | --- | --- |
| | | Example | | | | Example | | | |
| | | 47 | 48 | 49 | 50 | 47 | 48 | 49 | 50 |
| (1) | compound A-b·dihydrate | 0.529 | | | | 2.645 | | | |
| | (as compound A-b) | (0.5) | | | | (2.5) | | | |
| (2) | mannitol 50C | 12.071 | - | - | - | 60.355 | - | - | - |
| | mannitol 25C | - | 12.071 | - | - | - | 60.355 | - | - |
| | mannitol S | - | - | 12.071 | - | - | - | 60.355 | - |
| | mannitol P | - | - | - | 12.071 | - | - | - | 60.355 |
| (3) | crystalline cellulose KG-1000 | 4 | | | | 20 | | | |
| (4) | carmellose | 0.4 | | | | 2 | | | |
| | low-substituted hydroxypropylcellulose | 0.8 | | | | 4 | | | |
| | cornstarch | 2 | | | | 10 | | | |
| | magnesium stearate | 0.2 | | | | 1 | | | |
| | total | 20 | | | | 100 | | | |

Using various mannitols, in the same manner as in Examples 1 - 8 and according to the formulations shown in Table 19, orally disintegrating tablets having blending ratios shown in Table 19 were prepared. The compression force at that time is shown in Table 48 in the Experimental Example below.

Examples 51 - 56

<Blending rate of active ingredient>

[Table 20]

Table 20 orally disintegrating tablet (Examples 51 - 56) formulation (amount blended) (unit: g)

| ingredient | | Example | | | | | |
|---|---|---|---|---|---|---|---|
| | | 51 | 52 | 53 | 54 | 55 | 56 |
| (1) | compound A-b·dihydrate | 0.02116 | 0.1058 | 0.2116 | 1.058 | 2.116 | 4.232 |
| | (as compound A-b) | (0.02) | (0.1) | (0.2) | (1) | (2) | (4) |
| (2) | mannitol | 11.37884 | 11.2942 | 11.1884 | 10.342 | 9.284 | 7.168 |
| (3) | crystalline cellulose KG-1000 | 4 | | | | | |
| (4) | carmellose | 0.8 | | | | | |
| | low-substituted hydroxypropylcellulose | 1.6 | | | | | |
| | cornstarch | 2 | | | | | |
| | magnesium stearate | 0.2 | | | | | |
| | total | 20 | | | | | |

[Table 21]

Table 21 orally disintegrating tablet (Examples 51 - 56) blending ratio (unit: wt%)

| ingredient | | Example | | | | | |
|---|---|---|---|---|---|---|---|
| | | 51 | 52 | 53 | 54 | 55 | 56 |
| (1) | compound A-b·dihydrate | 0.1058 | 0.529 | 1.058 | 5.29 | 10.58 | 21.16 |
| | (as compound A-b) | (0.1) | (0.5) | (1) | (5) | (10) | (20) |
| (2) | mannitol | 56.8942 | 56.471 | 55.942 | 51.71 | 46.42 | 35.84 |
| (3) | crystalline cellulose KG-1000 | 20 | | | | | |
| (4) | carmellose | 4 | | | | | |
| | low-substituted hydroxypropylcellulose | 8 | | | | | |
| | cornstarch | 10 | | | | | |
| | magnesium stearate | 1 | | | | | |
| | total | 100 | | | | | |

In the same manner as in Examples 1 - 8 and according to the formulations shown in Table 20, orally disintegrating tablets having blending ratios shown in Table 21 were prepared. The compression force at that time is shown in Table 48 in the Experimental Example below.

Example 57 - 72

<Kinds and blending ratios of active ingredients>

[Table 22]

Table 22 orally disintegrating tablet (Examples 57 - 60) formulation (amount blended) and blending ratio

| ingredient | | amount blended (g) | | | | blending ratio (wt%) | | | |
|---|---|---|---|---|---|---|---|---|---|
| | | Example | | | | Example | | | |
| | | 57 | 58 | 59 | 60 | 57 | 58 | 59 | 60 |
| (1) | caffeine | 6 | 10 | | | 30 | 50 | | |
| (2) | mannitol | 5.4 | 4.2 | | | 27 | 21 | | |

(continued)

| | | amount blended (g) | | | | blending ratio (wt%) | | | |
|---|---|---|---|---|---|---|---|---|---|
| | ingredient | Example | | | | Example | | | |
| | | 57 | 58 | 59 | 60 | 57 | 58 | 59 | 60 |
| (3) | crystalline cellulose KG-1000 | 4 | 3 | | | 20 | 15 | | |
| (4) | carmellose | 0.8 | 0.8 | | | 4 | 4 | | |
| | low-substituted hydroxypropylcellulose | 1.6 | 0.8 | | | 8 | 4 | | |
| | cornstarch | 2 | 1 | | | 10 | 5 | | |
| | magnesium stearate | 0.2 | | | | 1 | | | |
| | total | 20 | | | | 100 | | | |

[Table 23]

Table 23 orally disintegrating tablet (Examples 61 - 64) formulation (amount blended) and blending ratio

| | | amount blended (g) | | | | blending ratio (wt%) | | | |
|---|---|---|---|---|---|---|---|---|---|
| | ingredient | Example | | | | Example | | | |
| | | 61 | 62 | 63 | 64 | 61 | 62 | 63 | 64 |
| (1) | acetaminophen | 6 | 10 | | | 30 | 50 | | |
| (2) | mannitol | 5.4 | 4.2 | | | 27 | 21 | | |
| (3) | crystalline cellulose KG-1000 | 4 | 3 | | | 20 | 15 | | |
| (4) | carmellose | 0.8 | 0.8 | | | 4 | 4 | | |
| | low-substituted hydroxypropylcellulose | 1.6 | 0.8 | | | 8 | 4 | | |
| | cornstarch | 2 | 1 | | | 10 | 5 | | |
| | magnesium stearate | 0.2 | | | | 1 | | | |
| | total | 20 | | | | 100 | | | |

[Table 24]

Table 24 orally disintegrating tablet (Examples 65 - 68) formulation (amount blended) and blending ratio

| | | amount blended (g) | | | | blending ratio (wt%) | | | |
|---|---|---|---|---|---|---|---|---|---|
| | ingredient | Example | | | | Example | | | |
| | | 65 | 66 | 67 | 68 | 65 | 66 | 67 | 68 |
| (1) | cimetidine | 6 | 10 | | | 30 | 50 | | |
| (2) | mannitol | 5.4 | 4.2 | | | 27 | 21 | | |
| (3) | crystalline cellulose KG-1000 | 4 | 3 | | | 20 | 15 | | |
| (4) | carmellose | 0.8 | 0.8 | | | 4 | 4 | | |
| | low-substituted hydroxypropylcellulose | 1.6 | 0.8 | | | 8 | 4 | | |
| | cornstarch | 2 | 1 | | | 10 | 5 | | |

(continued)

| ingredient | | amount blended (g) | | | | blending ratio (wt%) | | | |
|---|---|---|---|---|---|---|---|---|---|
| | | Example | | | | Example | | | |
| | | 65 | 66 | 67 | 68 | 65 | 66 | 67 | 68 |
| | magnesium stearate | 0.2 | | | | 1 | | | |
| | total | 20 | | | | 100 | | | |

[Table 25]

Table 25 orally disintegrating tablet (Examples 69 - 72) formulation (amount blended) and blending ratio

| | ingredient | amount blended (g) | | | | blending ratio (wt%) | | | |
|---|---|---|---|---|---|---|---|---|---|
| | | Example | | | | Example | | | |
| | | 69 | 70 | 71 | 72 | 69 | 70 | 71 | 72 |
| (1) | zonisamide | 6 | 10 | | | 30 | 50 | | |
| (2) | mannitol | 5.4 | 4.2 | | | 27 | 21 | | |
| (3) | crystalline cellulose KG-1000 | 4 | 3 | | | 20 | 15 | | |
| (4) | carmellose | 0.8 | 0.8 | | | 4 | 4 | | |
| | low-substituted hydroxypropylcellulose | 1.6 | 0.8 | | | 8 | 4 | | |
| | cornstarch | 2 | 1 | | | 10 | 5 | | |
| | magnesium stearate | 0.2 | | | | 1 | | | |
| | total | 20 | | | | 100 | | | |

Using various active ingredients, in the same manner as in Examples 1 - 8 and according to the formulations shown in Tables 22 - 25, orally disintegrating tablets having blending ratios shown in Tables 22 - 25 were prepared. The compression force at that time is shown in Table 49 in the Experimental Example below. As the active ingredients, caffeine was used in Examples 57 - 60, acetaminophen was used in Examples 61 - 64, cimetidine was used in Examples 65 - 68, and zonisamide was used in Examples 69 - 72.

The same mixture was used in Examples 58 - 60, Examples 62 - 64, Examples 66 - 68 and Examples 70 - 72. Tablets (weight 100 mg and diameter 7 mm per tablet) were prepared in Examples 58, 62, 66 and 70, tablets (weight 200 mg and diameter 8 mm per tablet) were prepared in Examples 59, 63, 67 and 71, and tablets (weight 300 mg and diameter 9 mm per tablet) were prepared in Examples 60, 64, 68 and 72.

Examples 73 - 80

<Active ingredient-containing particles obtained by particulation of active ingredient>

[Table 26]

Table 26 orally disintegrating tablet (Examples 73 - 80)
formulation (amount blended) (unit: g)

| | | ingredient | Example | | | | | | | |
|---|---|---|---|---|---|---|---|---|---|---|
| | | | 73 | 74 | 75 | 76 | 77 | 78 | 79 | 80 |
| active ingredient containing particles | (1a) | compound A-b·dihydrate | 52.9 | 52.9 | 105.8 | 52.9 | 52.9 | 211.6 | 105.8 | 211.6 |
| | | (as compound A-b) | (50) | (50) | (100) | (50) | (50) | (200) | (100) | (200) |
| | (2a) | mannitol | 347.1 | – | 294.2 | 392.1 | 347.1 | 568.4 | 284.2 | 568.4 |
| | | mannitol M | – | 347.1 | – | – | – | – | – | – |
| | (5a) | methylcellulose | 90 | 90 | 90 | 50 | 90 | 200 | 100 | 200 |
| | | hydroxypropylcellulose | 10 | 10 | 10 | 5 | 10 | 20 | 10 | 20 |
| orally disintegrating tablet — granules | | active ingredient containing particles | 375 | 375 | 187.5 | 375 | 375 | 250 | 187.5 | 250 |
| | (2b) | mannitol | 637.5 | – | 829.5 | 637.5 | 487.5 | 1040 | – | 1120 |
| | | mannitol M | – | 637.5 | – | – | – | – | 840 | – |
| | (3b) | crystalline cellulose KG-1000 | 150 | 150 | 150 | 150 | 150 | 200 | 150 | 200 |
| | | crystalline cellulose KG-802 | – | – | – | – | 150 | – | – | – |
| | (4.3b) | carmellose | 45 | 45 | 30 | 45 | 45 | 40 | 30 | 40 |
| | (4.2b) | cornstarch | 45 | 45 | 45 | 45 | 45 | – | 45 | 60 |
| | | light anhydrous silicic acid | 7.5 | 7.5 | 7.5 | 7.5 | 7.5 | 10 | 7.5 | 10 |
| | | aspartame | – | – | – | – | 6 | – | – | – |
| | | stevia | – | – | – | – | 6 | – | – | – |
| | (5b) | hydroxypropylcellulose | 15 | 15 | 15 | 15 | 15 | 20 | 15 | 20 |
| orally disintegrating tablet — powder ingredient | (3c) | crystalline cellulose KG-1000 | 150 | 150 | 150 | 150 | 150 | 200 | 150 | 200 |
| | (4.2c) | cornstarch | 45 | 45 | 45 | 45 | 45 | 200 | 45 | 70 |
| | | light anhydrous silicic acid | 1.5 | 1.5 | 1.5 | 1.5 | 1.5 | 2 | 1.5 | 2 |
| | | aspartame | 6 | 6 | 15 | 6 | – | 8 | 6 | 4 |
| | | stevia | 6 | 6 | 7.5 | 6 | – | 8 | 6 | 4 |
| | | spearmint | 1.5 | 1.5 | 1.5 | 1.5 | 1.5 | 2 | 1.5 | – |
| | | magnesium stearate | 15 | 15 | 15 | 15 | 15 | 20 | 15 | 20 |
| | | total | 1500 | 1500 | 1500 | 1500 | 1500 | 2000 | 1500 | 2000 |

Table 27  orally disintegrating tablet (Examples 73 - 80) blending ratio (unit: wt%)

EP 2 251 005 B1

| | | | ingredient | Example | | | | | | | |
|---|---|---|---|---|---|---|---|---|---|---|---|
| | | | | 73 | 74 | 75 | 76 | 77 | 78 | 79 | 80 |
| active ingredient containing particles | | (1a) | compound A-b·dihydrate | 2.645 | 2.645 | 2.645 | 2.645 | 2.645 | 2.645 | 2.645 | 2.645 |
| | | | (as compound A-b) | (2.5) | (2.5) | (2.5) | (2.5) | (2.5) | (2.5) | (2.5) | (2.5) |
| | | (2a) | mannitol | 17.355 | – | 7.355 | 19.605 | 17.355 | 7.105 | 7.105 | 7.105 |
| | | | mannitol M | – | 17.355 | – | – | – | – | – | – |
| | | (5a) | methylcellulose | 4.5 | 4.5 | 2.25 | 2.5 | 4.5 | 2.5 | 2.5 | 2.5 |
| | | | hydroxypropylcellulose | 0.5 | 0.5 | 0.25 | 0.25 | 0.5 | 0.25 | 0.25 | 0.25 |
| orally disintegrating tablet | granules | | active ingredient containing particles | 25 | 25 | 12.5 | 25 | 25 | 12.5 | 12.5 | 12.5 |
| | | (2b) | mannitol | 42.5 | – | 55.3 | 42.5 | 32.5 | 52 | – | 56 |
| | | | mannitol M | – | 42.5 | – | – | – | – | 56 | – |
| | | (3b) | crystalline cellulose KG-1000 | 10 | 10 | 10 | 10 | 10 | 10 | 10 | 10 |
| | | | crystalline cellulose KG-802 | – | – | – | – | 10 | – | – | – |
| | | (4.3b) | carmellose | 3 | 3 | 2 | 3 | 3 | 2 | 2 | 2 |
| | | (4.2b) | cornstarch | 3 | 3 | 3 | 3 | 3 | – | 3 | 3 |
| | | | light anhydrous silicic acid | 0.5 | 0.5 | 0.5 | 0.5 | 0.5 | 0.5 | 0.5 | 0.5 |
| | | | aspartame | – | – | – | – | 0.4 | – | – | – |
| | | | stevia | – | – | – | – | 0.4 | – | – | – |
| | | (5b) | hydroxypropylcellulose | 1 | 1 | 1 | 1 | 1 | 1 | 1 | 1 |
| | powder ingredient | (3c) | crystalline cellulose KG-1000 | 10 | 10 | 10 | 10 | 10 | 10 | 10 | 10 |
| | | (4.2c) | cornstarch | 3 | 3 | 3 | 3 | 3 | 10 | 3 | 3.5 |
| | | | light anhydrous silicic acid | 0.1 | 0.1 | 0.1 | 0.1 | 0.1 | 0.1 | 0.1 | 0.1 |
| | | | aspartame | 0.4 | 0.4 | 1 | 0.4 | – | 0.4 | 0.4 | 0.2 |
| | | | stevia | 0.4 | 0.4 | 0.5 | 0.4 | – | 0.4 | 0.4 | 0.2 |
| | | | spearmint | 0.1 | 0.1 | 0.1 | 0.1 | 0.1 | 0.1 | 0.1 | – |
| | | | magnesium stearate | 1 | 1 | 1 | 1 | 1 | 1 | 1 | 1 |
| | | | total | 100 | 100 | 100 | 100 | 100 | 100 | 100 | 100 |

Preparation of active ingredient-containing particles

The particles were prepared according to the formulations shown in Table 26 and at blending ratios shown in Table 27. That is, mannitol (2a) and methylcellulose (5a) were sieved through a 30 mesh sieve, and granulated while spraying a suspension of compound A-b·dihydrate (1a) in a binding solution (4 wt% aqueous solution of hydroxypropyl-cellulose (5a)), and dried in a fluid bed granulator (POWREX, MP-01). The obtained granules were sieved through a 22 mesh sieve to give active ingredient-containing particles having an average particle size of about 100 μm.

Preparation of orally disintegrating tablets

The tablets were prepared according to the formulations shown in Table 26 and at blending ratios shown in Table 27. That is, active ingredient-containing particles, mannitol (2b), crystalline cellulose (3b), carmellose (4.3b), cornstarch (4.2b), and a ingredient in the formulation from light anhydrous silicic acid, aspartame and stevia were mixed, and the mixture was sieved through a 30 mesh sieve, and granulated while spraying a binding solution (4 wt% aqueous solution of hydroxypropylcellulose (5b)), and dried in a fluid bed granulator (POWREX, MP-01). The obtained granules were sieved through a 22 mesh sieve to give granules having an average particle size of about 100 μm. The granules were mixed with powder ingredients recited in the formulation other than magnesium stearate. Then, magnesium stearate was added to the mixture, and the obtained mixture was compressed in a tablet press (Kikusui Seisakusho Ltd., VIRGO) to give tablets (weight 200 mg, diameter 8 mm per tablet). During compression, the compression force was adjusted such that the initial absolute hardness was 2.5 to 3.0N/mm$^2$. The compression force at that time is shown in Table 50 in the Experimental Example below.

Examples 81 - 88

<Active ingredient-containing particles obtained by particulation of active ingredient>

[Table 28]

```
Table 28 orally disintegrating tablet (Examples 81 - 88)
formulation (amount blended) (unit: g)
```

EP 2 251 005 B1

| | | | Example | | | | | | | |
|---|---|---|---|---|---|---|---|---|---|---|
| | | ingredient | 81 | 82 | 83 | 84 | 85 | 86 | 87 | 88 |
| active ingredient containing particles | (1a) | compound A-b·dihydrate | 211.6 | 211.6 | 253.92 | 253.92 | 211.6 | 211.6 | 211.6 | 105.8 |
| | | (as compound A-b) | (200) | (200) | (240) | (240) | (200) | (200) | (200) | (100) |
| | (2a) | mannitol | 568.4 | 568.4 | 682.08 | 682.08 | 568.4 | 568.4 | 568.4 | 498.2 |
| | (5a) | methylcellulose | 200 | 200 | 240 | 240 | 200 | 200 | 200 | 140 |
| | | hydroxypropylcellulose | 20 | 20 | 24 | 24 | 20 | 20 | 20 | 16 |
| orally disintegrating tablet | granules | | active ingredient containing particles | 187.5 | 187.5 | 187.5 | 187.5 | 187.5 | 187.5 | 187.5 | 285 |
| | | (2b) | mannitol | 730.5 | 798 | 798 | 768 | 730.5 | 715.5 | 723 | 625.5 |
| | | (3b) | crystalline cellulose KG-1000 | 150 | 150 | 150 | 150 | 150 | 150 | 150 | 150 |
| | | (4.3b) | carmellose | 30 | — | — | — | 30 | 30 | 30 | 30 |
| | | (4.2b) | cornstarch | — | 45 | 45 | 45 | — | — | — | — |
| | | (4.1b) | low-substituted hydroxypropylcellulose | 45 | — | — | — | 45 | 45 | 45 | 45 |
| | | | low-substituted hydroxypropylcellulose 22 | — | 45 | — | 75 | — | — | — | — |
| | | | low-substituted hydroxypropylcellulose 32 | — | — | 45 | — | — | — | — | — |
| | | | light anhydrous silicic acid | 15 | 7.5 | 7.5 | 7.5 | 15 | 15 | 15 | 15 |
| | | (5b) | hydroxypropylcellulose | 12 | 12 | 12 | 12 | 12 | 12 | 12 | 12 |
| | powder ingredient | (3c) | crystalline cellulose KG-1000 | 150 | 150 | 150 | 150 | 150 | 150 | 150 | 150 |
| | | (4.2c) | cornstarch | 150 | 45 | 45 | 45 | 150 | 150 | 150 | 150 |
| | | (4.3c) | carmellose | — | 30 | 30 | 30 | — | — | — | — |
| | | (4.1c) | low-substituted hydroxypropylcellulose | — | — | — | — | — | 15 | 15 | 15 |
| | | | light anhydrous silicic acid | 1.5 | 1.5 | 1.5 | 1.5 | 1.5 | 1.5 | 1.5 | 1.5 |
| | | | aspartame | 6 | 6 | 6 | 6 | 6 | 6 | 3 | 3 |
| | | | stevia | 6 | 6 | 6 | 6 | 6 | 6 | 1.5 | 1.5 |
| | | | spearmint | 1.5 | 1.5 | 1.5 | 1.5 | 1.5 | 1.5 | — | — |
| | | | menthol | — | — | — | — | — | — | 1.5 | 1.5 |
| | | | magnesium stearate | 15 | 15 | 15 | 15 | 15 | 15 | 15 | 15 |
| | | | total | 1500 | 1500 | 1500 | 1500 | 1500 | 1500 | 1500 | 1500 |

[Table 29]

Table 29 orally disintegrating tablet (Examples 81 – 88) blending ratio (unit: wt%)

EP 2 251 005 B1

| | | | ingredient | Example | | | | | | | |
|---|---|---|---|---|---|---|---|---|---|---|---|
| | | | | 81 | 82 | 83 | 84 | 85 | 86 | 87 | 88 |
| active ingredient containing particles | | (1a) | compound A-b·dihydrate | 2.645 | 2.645 | 2.645 | 2.645 | 2.645 | 2.645 | 2.645 | 2.645 |
| | | | (as compound A-b) | (2.5) | (2.5) | (2.5) | (2.5) | (2.5) | (2.5) | (2.5) | (2.5) |
| | | (2a) | mannitol | 7.105 | 7.105 | 7.105 | 7.105 | 7.105 | 7.105 | 7.105 | 12.455 |
| | | (5a) | methylcellulose | 2.5 | 2.5 | 2.5 | 2.5 | 2.5 | 2.5 | 2.5 | 3.5 |
| | | | hydroxypropylcellulose | 0.25 | 0.25 | 0.25 | 0.25 | 0.25 | 0.25 | 0.25 | 0.4 |
| orally disintegrating tablet | granules | | active ingredient containing particles | 12.5 | 12.5 | 12.5 | 12.5 | 12.5 | 12.5 | 12.5 | 19 |
| | | (2b) | mannitol | 48.7 | 53.2 | 53.2 | 51.2 | 48.7 | 47.7 | 48.2 | 41.7 |
| | | (3b) | crystalline cellulose KG-1000 | 10 | 10 | 10 | 10 | 10 | 10 | 10 | 10 |
| | | (4.3b) | carmellose | 2 | — | — | — | 2 | 2 | 2 | 2 |
| | | (4.2b) | cornstarch | — | 3 | 3 | 3 | — | — | — | — |
| | | (4.1b) | low-substituted hydroxypropylcellulose | 3 | — | — | — | 3 | 3 | 3 | 3 |
| | | | low-substituted hydroxypropylcellulose22 | — | 3 | — | 5 | — | — | — | — |
| | | | low-substituted hydroxypropylcellulose32 | — | — | 3 | — | — | — | — | — |
| | | | light anhydrous silicic acid | 1 | 0.5 | 0.5 | 0.5 | 1 | 1 | 1 | 1 |
| | | (5b) | hydroxypropylcellulose | 0.8 | 0.8 | 0.8 | 0.8 | 0.8 | 0.8 | 0.8 | 0.8 |
| | powder ingredient | (3c) | crystalline cellulose KG-1000 | 10 | 10 | 10 | 10 | 10 | 10 | 10 | 10 |
| | | (4.2c) | cornstarch | 10 | 3 | 3 | 3 | 10 | 10 | 10 | 10 |
| | | (4.3c) | carmellose | — | 2 | 2 | 2 | — | — | — | — |
| | | (4.1c) | low-substituted hydroxypropylcellulose | — | — | — | — | — | 1 | 1 | 1 |
| | | | light anhydrous silicic acid | 0.1 | 0.1 | 0.1 | 0.1 | 0.1 | 0.1 | 0.1 | 0.1 |
| | | | aspartame | 0.4 | 0.4 | 0.4 | 0.4 | 0.4 | 0.4 | 0.2 | 0.2 |
| | | | stevia | 0.4 | 0.4 | 0.4 | 0.4 | 0.4 | 0.4 | 0.1 | 0.1 |
| | | | spearmint | 0.1 | 0.1 | 0.1 | 0.1 | 0.1 | 0.1 | — | — |
| | | | menthol | — | — | — | — | — | — | 0.1 | 0.1 |
| | | | magnesium stearate | 1 | 1 | 1 | 1 | 1 | 1 | 1 | 1 |
| | | | total | 100 | 100 | 100 | 100 | 100 | 100 | 100 | 100 |

43

Preparation of active ingredient-containing particles

The particles were prepared according to the formulations shown in Table 28 and at blending ratios shown in Table 29. That is, mannitol (2a) and methylcellulose (5a) were sieved through a 30 mesh sieve, and granulated while spraying a suspension of compound A-b·dihydrate (1a) in a binding solution (4 wt% aqueous solution of hydroxypropyl-cellulose (5a)), and dried in a fluid bed granulator (POWREX, MP-01). The obtained granules were sieved through a 22 mesh sieve to give active ingredient-containing particles having an average particle size of about 100 μm.

Preparation of orally disintegrating tablets

The tablets were prepared according to the formulations shown in Table 28 and at blending ratios shown in Table 29. That is, ingredients recited in the formulation from among active ingredient-containing particles, mannitol (2b), crystalline cellulose (3b), carmellose (4.3b), cornstarch (4.2b), various low-substituted hydroxypropylcelluloses (4.1b) and light anhydrous silicic acid were mixed, and the mixture was sieved through a 30 mesh sieve, and granulated while spraying a binding solution (4 wt% aqueous solution of hydroxypropylcellulose (5b)), and dried in a fluid bed granulator (POWREX, MP-01). The obtained granules were sieved through a 22 mesh sieve to give granules having an average particle size of about 100 μm. The granules were mixed with powder ingredients recited in the formulation other than magnesium stearate. Then, magnesium stearate was added to the mixture, and the obtained mixture was compressed in a tablet press (Kikusui Seisakusho Ltd., VIRGO) to give tablets (weight 200 mg, diameter 8 mm per tablet). During compression, the compression force was adjusted such that the initial absolute hardness was 2.5 to 3.0N/mm$^2$. The compression force at that time is shown in Table 50 in the Experimental Example below.

Examples 89 - 99

<Kinds of low-substituted hydroxypropylcellulose and mannitol>

[Table 30]

Table 30 orally disintegrating tablet (Examples 89 - 92) formulation (amount blended) and blending ratio

| ingredient | | amount blended (g) | | | | blending ratio (wt%) | | | |
|---|---|---|---|---|---|---|---|---|---|
| | | Example | | | | Example | | | |
| | | 89 | 90 | 91 | 92 | 89 | 90 | 91 | 92 |
| (1) | compound A-b·dihydrate | 0.529 | | | | 2.645 | | | |
| | (as compound A-b) | (0.5) | | | | (2.5) | | | |
| (2) | mannitol | 10.871 | | | | 54.355 | | | |
| (3) | crystalline cellulose KG-1000 | 4 | | | | 20 | | | |
| (4) | carmellose | 0.8 | | | | 4 | | | |
| | low-substituted hydroxypropylcellulose 11 | 1.6 | - | - | - | 8 | - | - | - |
| | low-substituted hydroxypropylcellulose 31 | - | 1.6 | - | - | - | 8 | - | - |
| | low-substituted hydroxypropylcellulose 22 | - | - | 1.6 | - | - | - | 8 | - |
| | low-substituted hydroxypropylcellulose 32 | - | - | - | 1.6 | - | - | - | 8 |
| | cornstarch | 2 | | | | 10 | | | |

(continued)

| ingredient | | amount blended (g) | | | | blending ratio (wt%) | | | |
|---|---|---|---|---|---|---|---|---|---|
| | | Example | | | | Example | | | |
| | | 89 | 90 | 91 | 92 | 89 | 90 | 91 | 92 |
| | magnesium stearate | 0.2 | | | | 1 | | | |
| | total | 20 | | | | 100 | | | |

[Table 31]

Table 31 orally disintegrating tablet (Examples 93 - 96) formulation (amount blended) and blending ratio

| ingredient | | | amount blended (g) | | | | blending ratio (wt%) | | | |
|---|---|---|---|---|---|---|---|---|---|---|
| | | | Example | | | | Example | | | |
| | | | 93 | 94 | 95 | 96 | 93 | 94 | 95 | 96 |
| (1) | compound A-b·dihydrate | | 0.529 | | | | 2.645 | | | |
| | (as compound A-b) | | (0.5) | | | | (2.5) | | | |
| (2) | mannitol 100SD | | 12.071 | - | - | - | 60.355 | - | - | - |
| | mannitol 200SD | | - | 12.071 | - | - | - | 60.355 | - | - |
| | mannitol Marine Crystal | | - | - | 12.071 | - | - | - | 60.355 | - |
| | mannitol 108 | | - | - | - | 12.071 | - | - | - | 60.355 |
| (3) | crystalline cellulose KG-1000 | | 4 | | | | 20 | | | |
| (4) | carmellose | | 0.4 | | | | 2 | | | |
| | low-substituted hydroxypropylcellulose | | 0.8 | | | | 4 | | | |
| | cornstarch | | 2 | | | | 10 | | | |
| | magnesium stearate | | 0.2 | | | | 1 | | | |
| | total | | 20 | | | | 100 | | | |

[Table 32]

Table 32 orally disintegrating tablet (Examples 97 - 99) formulation (amount blended) and blending ratio

| ingredient | | amount blended (g) | | | blending ratio (wt%) | | |
|---|---|---|---|---|---|---|---|
| | | Example | | | Example | | |
| | | 97 | 98 | 99 | 97 | 98 | 99 |
| (1) | compound A-b·dihydrate | 0.529 | | | 2.645 | | |
| | (as compound A-b) | (0.5) | | | (2.5) | | |
| (2) | mannitol M100 | 12.071 | - | - | 60.355 | - | - |
| | mannitol M200 | - | 12.071 | - | - | 60.355 | - |
| | mannitol M300 | - | - | 12.071 | - | - | 60.355 |
| (3) | crystalline cellulose KG-1000 | 4 | | | 20 | | |

(continued)

| | | amount blended (g) | | | blending ratio (wt%) | | |
|---|---|---|---|---|---|---|---|
| | ingredient | Example | | | Example | | |
| | | 97 | 98 | 99 | 97 | 98 | 99 |
| (4) | carmellose | 0.4 | | | 2 | | |
| | low-substituted hydroxypropylcellulose | 0.8 | | | 4 | | |
| | cornstarch | 2 | | | 10 | | |
| | magnesium stearate | 0.2 | | | 1 | | |
| | total | 20 | | | 100 | | |

In the same manner as in Examples 1 - 8 and according to the formulations shown in Tables 30 - 32, orally disintegrating tablets having blending ratios shown in Tables 30 - 32 were prepared. The compression force at that time is shown in Table 51 in the Experimental Example below.

Comparative Examples 24 - 35

<Blending rate of each particular ingredient (4)>

[Table 33]

Table 33 orally disintegrating tablet (Comparative Examples 24 - 29) formulation (amount blended) (unit: g)

| | ingredient | Comparative Example | | | | | |
|---|---|---|---|---|---|---|---|
| | | 24 | 25 | 26 | 27 | 28 | 29 |
| (1) | compound A-b·dihydrate | 0.529 | | | | | |
| | (as compound A-b) | (0.5) | | | | | |
| (2) | mannitol | 14.371 | 9.471 | 14.371 | 9.471 | 13.571 | 8.671 |
| (3) | crystalline cellulose KG-1000 | 4 | | | | | |
| (4) | carmellose | 0.8 | 0.8 | 0.8 | 0.8 | 0.1 | 5.0 |
| | low-substituted hydroxypropylcellulose | 0.1 | 5.0 | - | - | 1.6 | 1.6 |
| | cornstarch | - | - | 0.1 | 5.0 | - | - |
| | magnesium stearate | 0.2 | | | | | |
| | total | 20 | | | | | |

[Table 34]

Table 34 orally disintegrating tablet (Comparative Examples 24 - 29) blending ratio (unit: wt%)

| | ingredient | Comparative Example | | | | | |
|---|---|---|---|---|---|---|---|
| | | 24 | 25 | 26 | 27 | 28 | 29 |
| (1) | compound A-b·dihydrate | 2.645 | | | | | |
| | (as compound A-b) | (2.5) | | | | | |
| (2) | mannitol | 71.855 | 47.355 | 71.855 | 47.355 | 67.855 | 43.355 |
| (3) | crystalline cellulose KG-1000 | 20 | | | | | |

(continued)

| | ingredient | Comparative Example | | | | | |
|---|---|---|---|---|---|---|---|
| | | 24 | 25 | 26 | 27 | 28 | 29 |
| (4) | carmellose | 4 | 4 | 4 | 4 | 0.5 | 25 |
| | low-substituted hydroxypropylcellulose | 0.5 | 25 | - | - | 8 | 8 |
| | cornstarch | - | - | 0.5 | 25 | - | - |
| | magnesium stearate | 1 | | | | | |
| | total | 100 | | | | | |

[Table 35]

Table 35 orally disintegrating tablet (Comparative Examples 30 - 35) formulation (amount blended) (unit: g)

| | ingredient | Comparative Example | | | | | |
|---|---|---|---|---|---|---|---|
| | | 30 | 31 | 32 | 33 | 34 | 35 |
| (1) | compound A-b·dihydrate | 0.529 | | | | | |
| | (as compound A-b) | (0.5) | | | | | |
| (2) | mannitol | 13.571 | 8.671 | 13.171 | 8.271 | 13.171 | 8.271 |
| (3) | crystalline cellulose KG-1000 | 4 | | | | | |
| (4) | carmellose | - | - | 0.1 | 5.0 | - | - |
| | low-substituted hydroxypropylcellulose | 1.6 | 1.6 | - | - | 0.1 | 5.0 |
| | cornstarch | 0.1 | 5.0 | 2 | 2 | 2 | 2 |
| | magnesium stearate | 0.2 | | | | | |
| | total | 20 | | | | | |

[Table 36]

Table 36 orally disintegrating tablet (Comparative Examples 30 - 35) blending ratio (unit: wt%)

| | ingredient | Comparative Example | | | | | |
|---|---|---|---|---|---|---|---|
| | | 30 | 31 | 32 | 33 | 34 | 35 |
| (1) | compound A-b·dihydrate | 2.645 | | | | | |
| | (as compound A-b) | (2.5) | | | | | |
| (2) | mannitol | 67.855 | 43.355 | 65.855 | 41.355 | 65.855 | 41.355 |
| (3) | crystalline cellulose KG-1000 | 20 | | | | | |
| (4) | carmellose | - | - | 0.5 | 25 | - | - |
| | low-substituted hydroxypropylcellulose | 8 | 8 | - | - | 0.5 | 25 |
| | cornstarch | 0.5 | 25 | 10 | 10 | 10 | 10 |
| | magnesium stearate | 1 | | | | | |
| | total | 100 | | | | | |

In the same manner as in Examples 1 - 8 and according to the formulations shown in Tables 33 and 35, orally disintegrating tablets having blending ratios shown in Tables 34 and 36 were prepared. The orally disintegrating tablets of Comparative Examples 24 - 35 are different from that of the present invention in that the blending ratio of one kind of particular ingredients of (4) does not satisfy the range of the present invention. The compression force at that time is shown in Table 53 in the Experimental Example below.

Comparative Examples 36 - 65

<When disintegrant different from the particular ingredient of (4) in the present invention is added>

[Table 37]

Table 37 orally disintegrating tablet (Comparative Examples 36 - 41) formulation (amount blended) (unit: g)

| ingredient | | Comparative Example | | | | | |
|---|---|---|---|---|---|---|---|
| | | 36 | 37 | 38 | 39 | 40 | 41 |
| (1) | compound A-b·dihydrate | 0.529 | | | | | |
| | (as compound A-b) | (0.5) | | | | | |
| (2) | mannitol | 12.871 | 12.871 | 12.471 | 12.471 | 11.671 | 11.671 |
| (3) | crystalline cellulose KG-1000 | 4 | | | | | |
| (4) | carmellose | 0.8 | - | 0.8 | - | - | - |
| | low-substituted hydroxypropylcellulose | - | 1.6 | - | - | 1.6 | - |
| | cornstarch | - | - | - | 2 | - | 2 |
| | crospovidone | 1.6 | 0.8 | 2 | 0.8 | 2 | 1.6 |
| | magnesium stearate | 0.2 | | | | | |
| | total | 20 | | | | | |

[Table 38]

Table 38 orally disintegrating tablet (Comparative Examples 36 - 41) blending ratio (unit: wt%)

| ingredient | | Comparative Example | | | | | |
|---|---|---|---|---|---|---|---|
| | | 36 | 37 | 38 | 39 | 40 | 41 |
| (1) | compound A-b·dihydrate | 2.645 | | | | | |
| | (as compound A-b) | (2.5) | | | | | |
| (2) | mannitol | 64.355 | 64.355 | 62.355 | 62.355 | 58.355 | 58.355 |
| (3) | crystalline cellulose KG-1000 | 20 | | | | | |
| (4) | carmellose | 4 | - | 4 | - | - | - |
| | low-substituted hydroxypropylcellulose | - | 8 | - | - | 8 | - |
| | cornstarch | - | - | - | 10 | - | 10 |
| | crospovidone | 8 | 4 | 10 | 4 | 10 | 8 |
| | magnesium stearate | 1 | | | | | |
| | total | 100 | | | | | |

[Table 39]

Table 39 orally disintegrating tablet (Comparative Examples 42 - 47) formulation (amount blended) (unit: g)

| ingredient | | Comparative Example | | | | | |
|---|---|---|---|---|---|---|---|
| | | 42 | 43 | 44 | 45 | 46 | 47 |
| (1) | compound A-b·dihydrate | 0.529 | | | | | |
| | (as compound A-b) | (0.5) | | | | | |
| (2) | mannitol | 12.871 | 12.871 | 12.471 | 12.471 | 11.671 | 11.671 |
| (3) | crystalline cellulose KG-1000 | 4 | | | | | |
| (4) | carmellose | 0.8 | - | 0.8 | - | - | - |
| | low-substituted hydroxypropylcellulose | - | 1.6 | - | - | 1.6 | - |
| | cornstarch | - | - | - | 2 | - | 2 |
| | carmellose sodium | 1.6 | 0.8 | 2 | 0.8 | 2 | 1.6 |
| | magnesium stearate | 0.2 | | | | | |
| | total | 20 | | | | | |

[Table 40]

Table 40 orally disintegrating tablet (Comparative Examples 42 - 47) blending ratio (unit: wt%)

| ingredient | | Comparative Example | | | | | |
|---|---|---|---|---|---|---|---|
| | | 42 | 43 | 44 | 45 | 46 | 47 |
| (1) | compound A-b·dihydrate | 2.645 | | | | | |
| | (as compound A-b) | (2.5) | | | | | |
| (2) | mannitol | 64.355 | 64.355 | 62.355 | 62.355 | 58.355 | 58.355 |
| (3) | crystalline cellulose KG-1000 | 20 | | | | | |
| (4) | carmellose | 4 | - | 4 | - | - | - |
| | low-substituted hydroxypropylcellulose | - | 8 | - | - | 8 | - |
| | cornstarch | - | - | - | 10 | - | 10 |
| | carmellose sodium | 8 | 4 | 10 | 4 | 10 | 8 |
| | magnesium stearate | 1 | | | | | |
| | total | 100 | | | | | |

[Table 41]

Table 41 orally disintegrating tablet (Comparative Examples 48 - 53) formulation (amount blended) (unit: g)

| ingredient | | Comparative Example | | | | | |
|---|---|---|---|---|---|---|---|
| | | 48 | 49 | 50 | 51 | 52 | 53 |
| (1) | compound A-b·dihydrate | 0.529 | | | | | |
| | (as compound A-b) | (0.5) | | | | | |
| (2) | mannitol | 12.871 | 12.871 | 12.471 | 12.471 | 11.671 | 11.671 |
| (3) | crystalline cellulose KG-1000 | 4 | | | | | |

(continued)

| ingredient | | Comparative Example | | | | | |
|---|---|---|---|---|---|---|---|
| | | 48 | 49 | 50 | 51 | 52 | 53 |
| (4) | carmellose | 0.8 | - | 0.8 | - | - | - |
| | low-substituted hydroxypropylcellulose | - | 1.6 | - | - | 1.6 | - |
| | cornstarch | - | - | - | 2 | - | 2 |
| | carmellose calcium | 1.6 | 0.8 | 2 | 0.8 | 2 | 1.6 |
| magnesium stearate | | 0.2 | | | | | |
| total | | 20 | | | | | |

[Table 42]

Table 42 orally disintegrating tablet (Comparative Examples 48 - 53) blending ratio (unit: wt%)

| ingredient | | Comparative Example | | | | | |
|---|---|---|---|---|---|---|---|
| | | 48 | 49 | 50 | 51 | 52 | 53 |
| (1) | compound A-b·dihydrate | 2.645 | | | | | |
| | (as compound A-b) | (2.5) | | | | | |
| (2) | mannitol | 64.355 | 64.355 | 62.355 | 62.355 | 58.355 | 58.355 |
| (3) | crystalline cellulose KG-1000 | 20 | | | | | |
| (4) | carmellose | 4 | - | 4 | - | - | - |
| | low-substituted hydroxypropylcellulose | - | 8 | - | - | 8 | - |
| | cornstarch | - | - | - | 10 | - | 10 |
| | carmellose calcium | 8 | 4 | 10 | 4 | 10 | 8 |
| magnesium stearate | | 1 | | | | | |
| total | | 100 | | | | | |

[Table 43]

Table 43 orally disintegrating tablet (Comparative Examples 54 - 59) formulation (amount blended) (unit: g)

| ingredient | | Comparative Example | | | | | |
|---|---|---|---|---|---|---|---|
| | | 54 | 55 | 56 | 57 | 58 | 59 |
| (1) | compound A-b·dihydrate | 0.529 | | | | | |
| | (as compound A-b) | (0.5) | | | | | |
| (2) | mannitol | 12.871 | 12.871 | 12.471 | 12.471 | 11.671 | 11.671 |
| (3) | crystalline cellulose KG-1000 | 4 | | | | | |
| (4) | carmellose | 0.8 | - | 0.8 | - | - | - |
| | low-substituted hydroxypropylcellulose | - | 1.6 | - | - | 1.6 | - |
| | cornstarch | - | - | - | 2 | - | 2 |
| | sodium carboxymethyl starch | 1.6 | 0.8 | 2 | 0.8 | 2 | 1.6 |
| magnesium stearate | | 0.2 | | | | | |

(continued)

| ingredient | | Comparative Example | | | | | |
|---|---|---|---|---|---|---|---|
| | | 54 | 55 | 56 | 57 | 58 | 59 |
| | total | 20 | | | | | |

[Table 44]

Table 44 orally disintegrating tablet (Comparative Examples 54 - 59) blending ratio (unit: wt%)

| ingredient | | Comparative Example | | | | | |
|---|---|---|---|---|---|---|---|
| | | 54 | 55 | 56 | 57 | 58 | 59 |
| (1) | compound A-b·dihydrate | 2.645 | | | | | |
| | (as compound A-b) | (2.5) | | | | | |
| (2) | mannitol | 64.355 | 64.355 | 62.355 | 62.355 | 58.355 | 58.355 |
| (3) | crystalline cellulose KG-1000 | 20 | | | | | |
| (4) | carmellose | 4 | - | 4 | - | - | - |
| | low-substituted hydroxypropylcellulose | - | 8 | - | - | 8 | - |
| | cornstarch | - | - | - | 10 | - | 10 |
| | sodium carboxymethyl starch | 8 | 4 | 10 | 4 | 10 | 8 |
| | magnesium stearate | 1 | | | | | |
| | total | 100 | | | | | |

[Table 45]

Table 45 orally disintegrating tablet (Comparative Examples 60 - 65) formulation (amount blended) (unit: g)

| ingredient | | Comparative Example | | | | | |
|---|---|---|---|---|---|---|---|
| | | 60 | 61 | 62 | 63 | 64 | 65 |
| (1) | compound A-b·dihydrate | 0.529 | | | | | |
| | (as compound A-b) | (0.5) | | | | | |
| (2) | mannitol | 12.871 | 12.871 | 12.471 | 12.471 | 11.671 | 11.671 |
| (3) | crystalline cellulose KG-1000 | 4 | | | | | |
| (4) | carmellose | 0.8 | - | 0.8 | - | - | - |
| | low-substituted hydroxypropylcellulose | - | 1.6 | - | - | 1.6 | - |
| | cornstarch | - | - | - | 2 | - | 2 |
| | croscarmellose sodium | 1.6 | 0.8 | 2 | 0.8 | 2 | 1.6 |
| | magnesium stearate | 0.2 | | | | | |
| | total | 20 | | | | | |

[Table 46]

Table 46 orally disintegrating tablet (Comparative Examples 60 - 65) blending ratio (unit: wt%)

| ingredient | | Comparative Example | | | | | |
|---|---|---|---|---|---|---|---|
| | | 60 | 61 | 62 | 63 | 64 | 65 |
| (1) | compound A-b·dihydrate | 2.645 | | | | | |
| | (as compound A-b) | (2.5) | | | | | |
| (2) | mannitol | 64.355 | 64.355 | 62.355 | 62.355 | 58.355 | 58.355 |
| (3) | crystalline cellulose KG-1000 | 20 | | | | | |
| (4) | carmellose | 4 | - | 4 | - | - | - |
| | low-substituted hydroxypropylcellulose | - | 8 | - | - | 8 | - |
| | cornstarch | - | - | - | 10 | - | 10 |
| | croscarmellose sodium | 8 | 4 | 10 | 4 | 10 | 8 |
| | magnesium stearate | 1 | | | | | |
| | total | 100 | | | | | |

In the same manner as in Examples 1 - 8 and according to the formulations shown in Tables 37, 39, 41, 43 and 45, orally disintegrating tablets having blending ratios shown in Tables 38, 40, 42, 44 and 46 were prepared. The orally disintegrating tablets of Comparative Examples 36 - 65 are different from that of the present invention in that only one kind of particular ingredients of (4) was used and a disintegrant different from the particular ingredients of (4), such as crospovidone, carmellose sodium and the like, was further blended. The compression force at that time is shown in Table 53 and Table 54 in the Experimental Example below.

Experimental Example 1

The tablets prepared in each Example and Comparative Example were measured for the disintegration time and hardness of initial and after humidification, and the results of Tables 47 - 54 were obtained.
[Table 47]

Table 47 disintegration time and hardness of initial and after humidification

| | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 1 | 6.0 | 26 | 25 | 3.6 | 1.9 |
| Ex. 2 | 6.0 | 28 | 24 | 3.1 | 1.8 |
| Ex. 3 | 5.0 | 26 | 21 | 3.0 | 1.6 |
| Ex. 4 | 6.0 | 21 | 22 | 3.5 | 1.8 |
| Ex. 5 | 8.0 | 24 | 26 | 4.2 | 2.5 |
| Ex. 6 | 10 | 25 | 26 | 3.7 | 2.0 |
| Ex. 7 | 10 | 25 | 24 | 3.7 | 2.0 |
| Ex. 8 | 10 | 26 | 26 | 3.7 | 2.1 |
| Ex. 9 | 6.0 | 25 | 24 | 3.6 | 1.9 |
| Ex. 10 | 5.0 | 23 | 23 | 3.2 | 1.6 |
| Ex. 11 | 6.0 | 25 | 24 | 3.5 | 1.9 |
| Ex. 12 | 6.0 | 25 | 25 | 3.9 | 1.9 |
| Ex. 13 | 7.0 | 25 | 28 | 3.8 | 2.2 |
| Ex. 14 | 7.0 | 26 | 28 | 3.6 | 1.9 |

(continued)

|  | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 15 | 7.0 | 25 | 26 | 3.6 | 1.8 |
| Ex. 16 | 6.0 | 28 | 27 | 3.5 | 1.8 |
| Ex. 17 | 5.0 | 26 | 23 | 2.8 | 1.6 |
| Ex. 18 | 5.0 | 24 | 23 | 3.3 | 1.6 |
| Ex. 19 | 6.0 | 26 | 23 | 3.7 | 1.8 |
| Ex. 20 | 7.0 | 24 | 25 | 3.8 | 2.2 |
| Ex. 21 | 7.0 | 22 | 24 | 3.5 | 1.8 |
| Ex. 22 | 6.0 | 27 | 28 | 3.5 | 2.1 |
| Ex. 23 | 6.0 | 25 | 25 | 3.8 | 1.8 |

[Table 48]

Table 48 disintegration time and hardness of initial and after humidification

|  | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 24 | 5.0 | 23 | 23 | 3.0 | 1.6 |
| Ex. 25 | 6.0 | 23 | 25 | 3.1 | 1.8 |
| Ex. 26 | 6.0 | 25 | 23 | 3.5 | 1.8 |
| Ex. 27 | 7.0 | 25 | 26 | 3.8 | 1.9 |
| Ex. 28 | 6.0 | 25 | 22 | 3.6 | 1.9 |
| Ex. 29 | 6.0 | 22 | 24 | 3.6 | 1.8 |
| Ex. 30 | 7.0 | 26 | 23 | 3.9 | 2.1 |
| Ex. 31 | 7.0 | 23 | 24 | 3.9 | 2.4 |
| Ex. 32 | 6.0 | 25 | 24 | 3.6 | 1.8 |
| Ex. 33 | 7.0 | 25 | 26 | 3.9 | 2.0 |
| Ex. 34 | 7.0 | 23 | 26 | 4.0 | 2.1 |
| Ex. 35 | 7.0 | 23 | 25 | 4.2 | 2.0 |
| Ex. 36 | 8.0 | 26 | 25 | 4.0 | 2.2 |
| Ex. 37 | 6.0 | 26 | 26 | 4.0 | 2.0 |
| Ex. 38 | 5.0 | 25 | 26 | 3.5 | 1.8 |
| Ex. 39 | 5.0 | 24 | 27 | 3.1 | 1.6 |
| Ex. 40 | 10 | 26 | 24 | 3.5 | 1.5 |
| Ex. 41 | 4.0 | 24 | 28 | 4.2 | 1.9 |
| Ex. 42 | 9.0 | 26 | 21 | 3.3 | 1.6 |
| Ex. 43 | 5.0 | 26 | 28 | 5.2 | 2.8 |
| Ex. 44 | 7.0 | 26 | 24 | 3.7 | 2.1 |
| Ex. 45 | 7.0 | 25 | 23 | 3.4 | 1.8 |

(continued)

|  | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 46 | 7.0 | 26 | 25 | 3.4 | 1.9 |
| Ex. 47 | 8.0 | 26 | 23 | 4.9 | 2.6 |
| Ex. 48 | 7.0 | 23 | 24 | 5.1 | 2.8 |
| Ex. 49 | 7.0 | 23 | 23 | 3.1 | 1.7 |
| Ex. 50 | 8.0 | 23 | 23 | 4.2 | 2.3 |
| Ex. 51 | 8.0 | 25 | 27 | 3.6 | 1.6 |
| Ex. 52 | 8.0 | 24 | 26 | 3.6 | 1.6 |
| Ex. 53 | 8.0 | 24 | 25 | 3.4 | 1.6 |
| Ex. 54 | 7.0 | 25 | 27 | 3.6 | 1.6 |
| Ex. 55 | 7.0 | 26 | 26 | 4.5 | 1.9 |
| Ex. 56 | 6.0 | 25 | 25 | 4.1 | 1.7 |

[Table 49]

Table 49 disintegration time and hardness of initial and after humidification

|  | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 57 | 5.0 | 26 | 26 | 4.0 | 2.9 |
| Ex. 58 | 5.0 | 25 | 27 | 5.9 | 4.4 |
| Ex. 59 | 4.0 | 24 | 24 | 3.5 | 2.8 |
| Ex. 60 | 3.0 | 24 | 25 | 2.2 | 1.5 |
| Ex. 61 | 5.0 | 25 | 21 | 2.7 | 1.6 |
| Ex. 62 | 10 | 19 | 18 | 4.1 | 3.0 |
| Ex. 63 | 10 | 23 | 23 | 2.7 | 2.1 |
| Ex. 64 | 9.0 | 24 | 24 | 2.5 | 1.9 |
| Ex. 65 | 8.0 | 25 | 23 | 4.6 | 2.6 |
| Ex. 66 | 10 | 18 | 17 | 5.0 | 3.2 |
| Ex. 67 | 10 | 20 | 20 | 4.4 | 2.8 |
| Ex. 68 | 10 | 27 | 27 | 4.2 | 2.5 |
| Ex. 69 | 8 | 25 | 24 | 4.7 | 3.2 |
| Ex. 70 | 10 | 23 | 20 | 4.9 | 3.7 |
| Ex. 71 | 10 | 24 | 24 | 3.9 | 3.0 |
| Ex. 72 | 10 | 26 | 25 | 3.7 | 2.8 |

[Table 50]

Table 50 disintegration time and hardness of initial and after humidification

|  | compression force (kN)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 73 | 5.5 | 21 | 21 | 2.2 | 1.6 |
| Ex. 74 | 4.7 | 17 | 16 | 2.5 | 1.9 |
| Ex. 75 | 6.0 | 22 | 22 | 2.5 | 1.6 |
| Ex. 76 | 5.5 | 22 | 22 | 2.6 | 1.6 |
| Ex. 77 | 5.0 | 27 | 26 | 2.7 | 1.6 |
| Ex. 78 | 5.7 | 24 | 25 | 2.8 | 2.0 |
| Ex. 79 | 5.1 | 21 | 19 | 2.9 | 1.9 |
| Ex. 80 | 5.5 | 21 | 19 | 2.9 | 1.8 |
| Ex. 81 | 5.5 | 22 | 23 | 2.9 | 2.0 |
| Ex. 82 | 6.4 | 21 | 19 | 2.6 | 1.6 |
| Ex. 83 | 5.7 | 21 | 20 | 2.8 | 1.9 |
| Ex. 84 | 5.6 | 22 | 24 | 2.9 | 1.8 |
| Ex. 85 | 5.8 | 20 | 18 | 2.5 | 1.6 |
| Ex. 86 | 5.9 | 20 | 22 | 2.7 | 1.7 |
| Ex. 87 | 5.7 | 20 | 19 | 2.7 | 1.7 |
| Ex. 88 | 5.5 | 17 | 18 | 2.8 | 1.7 |

[Table 51]

Table 51 disintegration time and hardness of initial and after humidification

|  | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Ex. 89 | 6.0 | 24 | 25 | 4.1 | 1.6 |
| Ex. 90 | 6.0 | 26 | 25 | 4.1 | 1.8 |
| Ex. 91 | 6.0 | 23 | 20 | 3.7 | 1.5 |
| Ex. 92 | 6.0 | 27 | 24 | 4.1 | 1.8 |
| Ex. 93 | 3.0 | 29 | 29 | 3.6 | 1.8 |
| Ex. 94 | 4.0 | 27 | 30 | 3.1 | 1.7 |
| Ex. 95 | 7.0 | 28 | 24 | 4.6 | 2.2 |
| Ex. 96 | 5.0 | 29 | 26 | 3.0 | 1.6 |
| Ex. 97 | 3.0 | 29 | 26 | 4.0 | 2.1 |
| Ex. 98 | 3.0 | 29 | 29 | 4.2 | 2.1 |
| Ex. 99 | 3.0 | 28 | 27 | 3.5 | 1.6 |

[Table 52]

Table 52 disintegration time and hardness of initial and after humidification

| | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Com. Ex. 1 | 8.0 | 29 | - | 1.2 | - |
| Com. Ex. 2 | 7.0 | 25 | 32 | 2.9 | 1.2 |
| Com. Ex. 3 | 6.0 | 28 | 35 | 4.6 | 2.3 |
| Com. Ex. 4 | 3.0 | 25 | 37 | 3.8 | 2.3 |
| Com. Ex. 5 | 2.0 | 24 | 40 | 4.0 | 2.5 |
| Com. Ex. 6 | 7.0 | 24 | 28 | 2.0 | 0.9 |
| Com. Ex. 7 | 9.0 | 22 | 24 | 2.0 | 0.8 |
| Com. Ex. 8 | 6.0 | 27 | - | 1.8 | - |
| Com. Ex. 9 | 9.0 | 27 | 30 | 2.1 | 1.1 |
| Com. Ex. 10 | 8.0 | 23 | - | 1.6 | - |
| Com. Ex. 11 | 5.0 | 24 | 24 | 2.8 | 1.3 |
| Com. Ex. 12 | 4.0 | 25 | 23 | 2.3 | 0.8 |
| Com. Ex. 13 | 5.0 | 23 | 23 | 2.9 | 0.9 |
| Com. Ex. 14 | 10 | 21 | 23 | 2.4 | 0.9 |
| Com. Ex. 15 | 3.0 | 27 | 33 | 4.2 | 2.1 |
| Com. Ex. 16 | 10 | 24 | 21 | 2.6 | 1.2 |
| Com. Ex. 17 | 3.0 | 26 | 32 | 4.2 | 2.3 |
| Com. Ex. 18 | 8.0 | 21 | - | 1.3 | - |
| Com. Ex. 19 | 8.0 | 27 | 19 | 2.1 | 0.8 |
| Com. Ex. 20 | 9.0 | 27 | 18 | 2.1 | 0.8 |
| Com. Ex. 21 | 8.0 | 23 | - | 1.9 | - |
| Com. Ex. 22 | 7.0 | 24 | 21 | 2.5 | 1.1 |
| Com. Ex. 23 | 8.0 | 26 | 21 | 2.4 | 0.9 |
| -: not measured | | | | | |

[Table 53]

Table 53 disintegration time and hardness of initial and after humidification

| | compression force (MPa)[*1] | disintegration time (s)[*2] | disintegration time (s)[*3] | hardness (N/mm$^2$)[*4] | hardness after humidification (N/mm$^2$)[*5] |
|---|---|---|---|---|---|
| Com. Ex. 24 | 7.0 | 25 | 28 | 3.5 | 1.4 |
| Com. Ex. 25 | 3.0 | 25 | 27 | 2.5 | 1.0 |
| Com. Ex. 26 | 5.0 | 25 | 23 | 2.9 | 1.1 |
| Com. Ex. 27 | 6.0 | 25 | 20 | 3.3 | 1.4 |
| Com. Ex. 28 | 5.0 | 26 | 23 | 3.2 | 1.3 |
| Com. Ex. 29 | 4.0 | 26 | 22 | 2.3 | 0.9 |
| Com. Ex. 30 | 6.0 | 24 | 24 | 2.8 | 1.3 |

(continued)

| | compression force (MPa)*1 | disintegration time (s)*2 | disintegration time (s)*3 | hardness (N/mm²)*4 | hardness after humidification (N/mm²)*5 |
|---|---|---|---|---|---|
| Com. Ex. 31 | 5.0 | 25 | 20 | 2.9 | 1.4 |
| Com. Ex. 32 | 6.0 | 24 | 20 | 2.3 | 1.1 |
| Com. Ex. 33 | 5.0 | 26 | 19 | 2.3 | 1.1 |
| Com. Ex. 34 | 5.0 | 24 | 21 | 2.9 | 1.4 |
| Com. Ex. 35 | 3.0 | 26 | 25 | 2.0 | 1.0 |
| Com. Ex. 36 | 9.0 | 24 | 19 | 2.7 | 0.9** |
| Com. Ex. 37 | 9.0 | 25 | 19 | 3.1 | 1.2** |
| Com. Ex. 38 | 9.0 | 24 | 19 | 2.7 | 0.8** |
| Com. Ex. 39 | 9.0 | 23 | 19 | 3.0 | 1.2** |
| Com. Ex. 40 | 9.0 | 25 | 19 | 2.7 | 0.9** |
| Com. Ex. 41 | 10 | 24 | 18 | 3.0 | 1.0** |
| Com. Ex. 42 | 2.0 | >60 | - | - | - |
| Com. Ex. 43 | 2.0 | >60 | - | - | - |
| Com. Ex. 44 | 2.0 | >60 | - | - | - |
| Com. Ex. 45 | 2.0 | >60 | - | - | - |
| Com. Ex. 46 | 2.0 | >60 | - | - | - |

-: not measured,

**: Phenomenon of convex and concaves on tablet surface was observed.

[Table 54]

Table 54 disintegration time and hardness of initial and after humidification

| | compression force (MPa)*1 | disintegration time (s)*2 | disintegration time (s)*3 | hardness (N/mm²)*4 | hardness after humidification (N/mm²)*5 |
|---|---|---|---|---|---|
| Com. Ex. 47 | 2.0 | >60 | - | - | - |
| Com. Ex. 48 | 4.0 | 25 | - | 1.6 | - |
| Com. Ex. 49 | 4.0 | 24 | 24 | 2.1 | 0.9 |
| Com. Ex. 50 | 4.0 | 24 | - | 1.3 | - |
| Com. Ex. 51 | 4.0 | 25 | 20 | 2.1 | 0.9 |
| Com. Ex. 52 | 3.0 | 26 | - | 1.6 | - |
| Com. Ex. 53 | 4.0 | 23 | - | 1.8 | - |
| Com. Ex. 54 | 3.0 | 23 | - | 1.2 | - |
| Com. Ex. 55 | 3.0 | 22 | - | 1.3 | - |
| Com. Ex. 56 | 3.0 | 25 | - | 1.1 | - |
| Com. Ex. 57 | 4.0 | 23 | - | 1.8 | - |
| Com. Ex. 58 | 3.0 | 26 | - | 1.5 | - |
| Com. Ex. 59 | 3.0 | 23 | - | 1.3 | - |

(continued)

| | compression force (MPa)[*1] | disintegration time (s)[*2] | disintegration time (s)[*3] | hardness (N/mm²)[*4] | hardness after humidification (N/mm²)[*5] |
|---|---|---|---|---|---|
| Com. Ex. 60 | 4.0 | 24 | - | 1.8 | - |
| Com. Ex. 61 | 4.0 | 23 | 23 | 2.2 | 0.9 |
| Com. Ex. 62 | 3.0 | 25 | - | 1.2 | - |
| Com. Ex. 63 | 5.0 | 25 | 23 | 2.7 | 1.1 |
| Com. Ex. 64 | 2.0 | >60 | - | - | - |
| Com. Ex. 65 | 2.0 | 23 | - | 0.8 | - |
| -: not measured | | | | | |

*1: The pressure (MPa) applied onto the whole punch during compression was measured using a pressure gauge provided on a hydraulic press machine manufactured by RIKEN (for Table 50 alone, Kikusui Seisakusho Ltd., VIRGO was used, pressure unit was kN).
*2: Using three healthy male test subjects, an average time (sec) from placing a tablet in the oral cavity to disintegration thereof was calculated.
*3: Using three healthy male test subjects, an average time (sec) from placing a tablet preserved under conditions of 25°C and 75% RH for 3 days in the oral cavity to disintegration thereof was calculated.
*4: The tablet thickness was measured by a dial thickness gauge (manufactured by Niigataseiki, DS-3010S), the hardness (kgf) was measured using a tablet hardness meter (manufactured by Toyama Sangyo Co., Ltd., TH-203CP), and the absolute hardness was calculated from the following formula. absolute hardness = measured hardness (kgf) $\times$ 9.8/cross-sectional area (mm²)
*5: After preservation at 25°C, 75% RH for 3 days, the tablet thickness was measured by a dial thickness gauge (manufactured by Niigataseiki, DS-3010S), the hardness (kgf) was measured using a tablet hardness meter (manufactured by Toyama Sangyo Co., Ltd., TH-203CP), and the absolute hardness was calculated from the formula of *4.

All the orally disintegrating tablets prepared in each Example disintegrated within 30 sec under the both conditions of before and after humidification, and were shown to be superior in disintegration property. In addition, they had absolute hardness of not less than 2.0N/mm² and hardness after humidification of not less than 1.5N/mm². On the other hand, the orally disintegrating tablets prepared in Comparative Example did not satisfy all of the disintegration property, absolute hardness and hardness after humidification.

Experimental Example 2

The tablets prepared in each Example and Comparative Example were evaluated for easiness of ingestion (sensory evaluation) to obtain the results of Tables 55 - 62.
[Table 55]

Table 55 evaluation of comfortable during use

| | powdery feeling[*1] | bloated feeling[*1] | acid taste[*1] | initial disintegration property[*2] |
|---|---|---|---|---|
| Ex. 1 | ⊙ | ⊙ | ⊙ | ○ |
| Ex. 2 | ⊙ | ⊙ | ⊙ | ⊙ |
| Ex. 3 | ⊙ | ⊙ | ⊙ | ○ |
| Ex. 4 | ⊙ | ⊙ | ⊙ | ⊙ |
| Ex. 5 | ⊙ | ⊙ | ⊙ | ⊙ |
| Ex. 6 | ⊙ | ⊙ | ○ | ⊙ |
| Ex. 7 | ⊙ | ⊙ | Δ | ⊙ |

(continued)

| | powdery feeling[*1] | bloated feeling[*1] | acid taste[*1] | initial disintegration property[*2] |
|---|---|---|---|---|
| Ex. 8 | ⊙ | ⊙ | Δ | ⊙ |
| Ex. 9 | ⊙ | ⊙ | ⊙ | ○ |
| Ex. 10 | ⊙ | ⊙ | ⊙ | ⊙ |
| Ex. 11 | ⊙ | ⊙ | ⊙ | ⊙ |
| Ex. 12 | ⊙ | ⊙ | ⊙ | ⊙ |
| Ex. 13 | ⊙ | ⊙ | ⊙ | ⊙ |
| Ex. 14 | ○ | ⊙ | ⊙ | ⊙ |
| Ex. 15 | ○ | ⊙ | ⊙ | ⊙ |
| Ex. 16 | Δ | ⊙ | ⊙ | ⊙ |
| Ex. 17 | ⊙ | ⊙ | ⊙ | ○ |
| Ex. 18 | ⊙ | ⊙ | ⊙ | ○ |
| Ex. 19 | ⊙ | ⊙ | ⊙ | ○ |
| Ex. 20 | ⊙ | ⊙ | ⊙ | ○ |
| Ex. 21 | ○ | ⊙ | ○ | ○ |
| Ex. 22 | Δ | ⊙ | Δ | ○ |
| Ex. 23 | Δ | ⊙ | × | ○ |

[Table 56]

Table 56 evaluation of comfortable during use

| | powdery feeling[*1] | bloated feeling[*1] | acid taste[1] | initial disintegration property[*2] |
|---|---|---|---|---|
| Ex. 24 | ⊙ | ⊙ | ⊙ | ○ |
| Ex. 25 | ⊙ | ⊙ | ⊙ | ○ |
| Ex. 26 | ⊙ | ⊙ | ⊙ | ○ |
| Ex. 27 | ⊙ | ⊙ | ⊙ | ⊙ |
| Ex. 28 | ○ | ⊙ | ⊙ | ⊙ |
| Ex. 29 | ⊙ | ⊙ | ⊙ | ⊙ |
| Ex. 30 | ⊙ | ⊙ | ⊙ | ⊙ |
| Ex. 31 | ⊙ | ⊙ | ⊙ | ⊙ |
| Ex. 32 | ⊙ | ⊙ | ○ | ⊙ |
| Ex. 33 | ○ | ⊙ | ○ | ⊙ |
| Ex. 34 | ○ | ⊙ | ○ | ⊙ |
| Ex. 35 | ○ | ⊙ | Δ | ⊙ |
| Ex. 36 | ○ | ⊙ | Δ | ⊙ |
| Ex. 37 | Δ | ⊙ | Δ | ⊙ |
| Ex. 38 | Δ | ⊙ | Δ | ⊙ |
| Ex. 39 | Δ | ⊙ | × | ⊙ |
| Ex. 40 | ○ | ⊙ | ○ | ⊙ |
| Ex. 41 | ○ | ○ | ○ | ○ |

(continued)

|  | powdery feeling[*1] | bloated feeling[*1] | acid taste[1] | initial disintegration property[*2] |
|---|---|---|---|---|
| Ex. 42 | ○ | ⊙ | ○ | ⊙ |
| Ex. 43 | ○ | ○ | ○ | ⊙ |
| Ex. 44 | ○ | ⊙ | ○ | ⊙ |
| Ex. 45 | ○ | ⊙ | ○ | ⊙ |
| Ex. 46 | ○ | ⊙ | ○ | ⊙ |
| Ex. 47 | ⊙ | ⊙ | ⊙ | ⊙ |
| Ex. 48 | ⊙ | ⊙ | ⊙ | ⊙ |
| Ex. 49 | ⊙ | ⊙ | ⊙ | ⊙ |
| Ex. 50 | ⊙ | ⊙ | ⊙ | ⊙ |
| Ex. 51 | ○ | ⊙ | ○ | ⊙ |
| Ex. 52 | ○ | ⊙ | ○ | ⊙ |
| Ex. 53 | ○ | ⊙ | ○ | ⊙ |
| Ex. 54 | ○ | ⊙ | ○ | ⊙ |
| Ex. 55 | ○ | ⊙ | ○ | ⊙ |
| Ex. 56 | ○ | ⊙ | ○ | ⊙ |

[Table 57]

Table 57 evaluation of comfortable during use

|  | powdery feeling[*1] | bloated feeling[*1] | acid taste[1] | initial disintegration property[*2] |
|---|---|---|---|---|
| Ex. 57 | ○ | ⊙ | ○ | ⊙ |
| Ex. 58 | ⊙ | ⊙ | ○ | ⊙ |
| Ex. 59 | ⊙ | ⊙ | ○ | ⊙ |
| Ex. 60 | ⊙ | ⊙ | ○ | ⊙ |
| Ex. 61 | ○ | ⊙ | ○ | ⊙ |
| Ex. 62 | ⊙ | ⊙ | ○ | ⊙ |
| Ex. 63 | ⊙ | ⊙ | ○ | ⊙ |
| Ex. 64 | ⊙ | ⊙ | ○ | ⊙ |
| Ex. 65 | ○ | ⊙ | ○ | ⊙ |
| Ex. 66 | ⊙ | ⊙ | ○ | ⊙ |
| Ex. 67 | ⊙ | ⊙ | ○ | ⊙ |
| Ex. 68 | ⊙ | ⊙ | ○ | ⊙ |
| Ex. 69 | ○ | ⊙ | ○ | ⊙ |
| Ex. 70 | ⊙ | ⊙ | ○ | ⊙ |
| Ex. 71 | ⊙ | ⊙ | ○ | ⊙ |
| Ex. 72 | ⊙ | ⊙ | ○ | ⊙ |

[Table 58]

Table 58 evaluation of comfortable during use

|  | powdery feeling[*1] | bloated feeling[*1] | acid taste[*1] | initial disintegration property[*2] |
|---|---|---|---|---|
| Ex. 73 | ☉ | ☉ | ☉ | ○ |
| Ex. 74 | ☉ | ☉ | ☉ | ○ |
| Ex. 75 | ☉ | ☉ | ☉ | ○ |
| Ex. 76 | ☉ | ☉ | ☉ | ○ |
| Ex. 77 | ☉ | ○ | ☉ | ○ |
| Ex. 78 | ☉ | ☉ | ☉ | ☉ |
| Ex. 79 | ☉ | ☉ | ☉ | ○ |
| Ex. 80 | ☉ | ☉ | ☉ | ○ |
| Ex. 81 | ☉ | ☉ | ☉ | ☉ |
| Ex. 82 | ☉ | ☉ | ☉ | ○ |
| Ex. 83 | ☉ | ☉ | ☉ | ○ |
| Ex. 84 | ☉ | ☉ | ☉ | ○ |
| Ex. 85 | ☉ | ☉ | ☉ | ☉ |
| Ex. 86 | ☉ | ☉ | ☉ | ☉ |
| Ex. 87 | ☉ | ☉ | ☉ | ☉ |
| Ex. 88 | ☉ | ☉ | ☉ | ☉ |

[Table 59]

Table 59 evaluation of comfortable during use

|  | powdery feeling[*1] | bloated feeling[*1] | acid taste[*1] | initial disintegration property[*2] |
|---|---|---|---|---|
| Ex. 89 | ○ | ☉ | ○ | ☉ |
| Ex. 90 | ○ | ☉ | ○ | ☉ |
| Ex. 91 | ○ | ☉ | ○ | ☉ |
| Ex. 92 | ○ | ☉ | ○ | ☉ |
| Ex. 93 | ☉ | ☉ | ☉ | ☉ |
| Ex. 94 | ☉ | ☉ | ☉ | ☉ |
| Ex. 95 | ☉ | ☉ | ☉ | ☉ |
| Ex. 96 | ☉ | ☉ | ☉ | ☉ |
| Ex. 97 | ☉ | ☉ | ☉ | ☉ |
| Ex. 98 | ☉ | ☉ | ☉ | ☉ |
| Ex. 99 | ☉ | ☉ | ☉ | ☉ |

[Table 60]

Table 60 evaluation of comfortable during use

|  | powdery feeling[*1] | bloated feeling[*1] | acid taste[*1] | initial disintegration property[*2] |
|---|---|---|---|---|
| Com. Ex. 1 | ☉ | ☉ | ☉ | ○ |
| Com. Ex. 2 | ☉ | ☉ | ☉ | ○ |
| Com. Ex. 3 | ☉ | Δ | ☉ | Δ |

(continued)

|  | powdery feeling[*1] | bloated feeling[*1] | acid taste[*1] | initial disintegration property[*2] |
|---|---|---|---|---|
| Com. Ex. 4 | ⊙ | × | ⊙ | × |
| Com. Ex. 5 | ⊙ | × × | ⊙ | × |
| Com. Ex. 6 | ⊙ | ⊙ | ○ | ○ |
| Com. Ex. 7 | ⊙ | ⊙ | Δ | ○ |
| Com. Ex. 8 | ○ | ⊙ | ⊙ | ○ |
| Com. Ex. 9 | ⊙ | ⊙ | ⊙ | ⊙ |
| Com. Ex. 10 | ⊙ | ⊙ | ⊙ | ⊙ |
| Com. Ex. 11 | ⊙ | ⊙ | ⊙ | ○ |
| Com. Ex. 12 | ⊙ | ⊙ | ⊙ | ○ |
| Com. Ex. 13 | ⊙ | ⊙ | ⊙ | ○ |
| Com. Ex. 14 | ○ | ⊙ | ○ | ⊙ |
| Com. Ex. 15 | ○ | Δ | ○ | ○ |
| Com. Ex. 16 | ○ | ⊙ | ○ | ⊙ |
| Com. Ex. 17 | ○ | Δ | ○ | ○ |
| Com. Ex. 18 | ⊙ | ⊙ | ⊙ | ⊙ |
| Com. Ex. 19 | ○ | ⊙ | ○ | ⊙ |
| Com. Ex. 20 | ○ | ⊙ | Δ | ⊙ |
| Com. Ex. 21 | ⊙ | ⊙ | ⊙ | ⊙ |
| Com. Ex. 22 | ○ | ⊙ | ○ | ⊙ |
| Com. Ex. 23 | ○ | ⊙ | Δ | ⊙ |

[Table 61]

Table 61 evaluation of comfortable during use

|  | powdery feeling[*1] | bloated feeling[*1] | acid taste[*1] | initial disintegration property[*2] |
|---|---|---|---|---|
| Com. Ex. 24 | ⊙ | ⊙ | ○ | ○ |
| Com. Ex. 25 | × × | ⊙ | ○ | ○ |
| Com. Ex. 26 | ⊙ | ⊙ | ○ | ○ |
| Com. Ex. 27 | ○ | ⊙ | ○ | ⊙ |
| Com. Ex. 28 | ○ | ⊙ | ⊙ | ○ |
| Com. Ex. 29 | ○ | ⊙ | × × | ○ |
| Com. Ex. 30 | ○ | ⊙ | ⊙ | ○ |
| Com. Ex. 31 | ○ | ⊙ | ⊙ | ⊙ |
| Com. Ex. 32 | ⊙ | ⊙ | ⊙ | ⊙ |
| Com. Ex. 33 | ⊙ | ⊙ | × × | ⊙ |
| Com. Ex. 34 | ⊙ | ⊙ | ⊙ | ⊙ |
| Com. Ex. 35 | × × | ⊙ | ⊙ | ⊙ |
| Com. Ex. 36 | ⊙ | ⊙ | ⊙ | ⊙ |
| Com. Ex. 37 | ⊙ | ⊙ | ⊙ | ⊙ |

(continued)

| | powdery feeling[*1] | bloated feeling[*1] | acid taste[*1] | initial disintegration property[*2] |
|---|---|---|---|---|
| Com. Ex. 38 | ⊙ | ⊙ | ⊙ | ⊙ |
| Com. Ex. 39 | ⊙ | ⊙ | ⊙ | ⊙ |
| Com. Ex. 40 | ⊙ | ⊙ | ⊙ | ⊙ |
| Com. Ex. 41 | ⊙ | ⊙ | ⊙ | ⊙ |
| Com. Ex. 42 | ⊙ | ⊙ | ⊙ | ⊙ |
| Com. Ex. 43 | ⊙ | ⊙ | ⊙ | ⊙ |
| Com. Ex. 44 | ⊙ | ⊙ | ⊙ | ⊙ |
| Com. Ex. 45 | ⊙ | ⊙ | ⊙ | ⊙ |
| Com. Ex. 46 | ⊙ | ⊙ | ⊙ | ⊙ |

[Table 62]

Table 62 evaluation of comfortable during use

| | powdery feeling[*1] | bloated feeling[*1] | acid taste[*1] | initial disintegration property[*2] |
|---|---|---|---|---|
| Com. Ex. 47 | ⊙ | ⊙ | ⊙ | ⊙ |
| Com. Ex. 48 | ⊙ | ⊙ | ⊙ | ⊙ |
| Com. Ex. 49 | ⊙ | ⊙ | ⊙ | ⊙ |
| Com. Ex. 50 | ⊙ | ⊙ | ⊙ | ⊙ |
| Com. Ex. 51 | ⊙ | ⊙ | ⊙ | ⊙ |
| Com. Ex. 52 | ⊙ | ⊙ | ⊙ | ⊙ |
| Com. Ex. 53 | ⊙ | ⊙ | ⊙ | ⊙ |
| Com. Ex. 54 | ⊙ | ⊙ | ⊙ | ⊙ |
| Com. Ex. 55 | ⊙ | ⊙ | ⊙ | ⊙ |
| Com. Ex. 56 | ⊙ | ⊙ | ⊙ | ⊙ |
| Com. Ex. 57 | ⊙ | ⊙ | ⊙ | ⊙ |
| Com. Ex. 58 | ⊙ | ⊙ | ⊙ | ⊙ |
| Com. Ex. 59 | ⊙ | ⊙ | ⊙ | ⊙ |
| Com. Ex. 60 | ⊙ | ⊙ | ⊙ | ⊙ |
| Com. Ex. 61 | ⊙ | ⊙ | ⊙ | ⊙ |
| Com. Ex. 62 | ⊙ | ⊙ | ⊙ | ⊙ |
| Com. Ex. 63 | ⊙ | ⊙ | ⊙ | ⊙ |
| Com. Ex. 64 | ⊙ | ⊙ | ⊙ | ⊙ |
| Com. Ex. 65 | ⊙ | ⊙ | ⊙ | ⊙ |

*1: powdery feeling: state of easy absorption of water content in the mouth by the tablet, long time required to achieve a suspension state, and comparatively large amount of remaining powder.
bloated feeling: state of swelling of tablet due to absorption of water content in the mouth by the tablet, which is irrelevant to tablet disintegration.
acid taste: state of sour taste in the mouth.

Three healthy male test subjects maintained a tablet in the oral cavity, and evaluated easiness of ingestion with regard to the above-mentioned 3 items based on the following criteria.

The average evaluation of the three subjects is show in Tables 55 - 62.

⊙ not at all felt

○ scarcely felt

∆ slightly felt

× felt

×× water is required

*2: initial disintegration property: the time (sec) from placing a tablet in the oral cavity of three healthy male test subjects to the start of disintegration thereof was measured, and an average of the three was calculated and shown in Tables 55 - 62.

⊙ After placing, disintegration starts within 3 sec.

○ After placing, disintegration starts in 3 sec - 7 sec.

∆ After placing, disintegration starts in 8 sec - 12 sec.

× After placing, disintegration starts in not less than 13 sec.

The orally disintegrating tablets prepared in respective Examples all received good evaluation with respect to initial disintegration property. Although some of them prepared slightly powdery feeling and an acid taste, high easiness of ingestion was mostly afforded.

Comparative Examples 66 - 68

<Formulation disclosed in patent document 5 which is different from that of the present invention>

[Table 63]

Table 63 orally disintegrating tablet (Comparative Examples 66 - 68) granule B formulation (amount blended) and blending ratio

| ingredient | | amount blended (g) | blending ratio (wt%) |
|---|---|---|---|
| | | Comparative Example | Comparative Example |
| | | common to 66 - 68 | common to 66 - 68 |
| | compound A-b·dihydrate | 224 | 20.3 |
| | (as compound A-b) | (211.7) | (19.2) |
| | finely granulated lactose | 657.8 | 59.6 |
| | cornstarch | 33 | 3.0 |
| | low-substituted hydroxypropyl-cellulose 31 | 165 | 14.9 |
| | hydroxypropylcellulose | 22 | 2.0 |
| | yellow ferric oxide | 2.2 | 0.2 |
| | total | 1104 | 100 |

[Table 64]

Table 64 orally disintegrating tablet (Comparative Examples 66 - 68) granule C formulation (amount blended) and blending ratio

| ingredient | | amount blended (g) | blending ratio (wt%) |
|---|---|---|---|
| | | Comparative Example | Comparative Example |
| | | common to 66 - 68 | common to 66 - 68 |
| | mannitol S | 574.3 | 56.8 |
| | mannitol 1.059 | 300.9 | 29.7 |

(continued)

| ingredient | | amount blended (g) | blending ratio (wt%) |
|---|---|---|---|
| | | Comparative Example | Comparative Example |
| | | common to 66 - 68 | common to 66 - 68 |
| | low-substituted hydroxypropyl-cellulose 11 | 95.9 | 9.5 |
| | mannitol | 30.7 | 3.0 |
| | anhydride citric acid | 9.6 | 0.9 |
| | yellow ferric oxide | 0.77 | 0.1 |
| | total | 1012.17 | 100 |

[Table 65]

Table 65 orally disintegrating tablet (Comparative Examples 66 - 68) formulation (amount blended) and blending ratio

| ingredient | | amount blended (g) | | | blending ratio (wt%) | | |
|---|---|---|---|---|---|---|---|
| | | Comparative Example | | | Comparative Example | | |
| | | 66 | 67 | 68 | 66 | 67 | 68 |
| | granule B | 18 | | | 40.0 | | |
| | granule C | 23.76 | | | 52.8 | | |
| | crystalline cellulose KG-1000 | 2.48 | - | - | 5.5 | - | - |
| | crystalline cellulose KG-802 | - | 2.48 | - | - | 5.5 | - |
| | crystalline cellulose PH-101 | - | - | 2.48 | - | - | 5.5 |
| | aspartame | 0.09 | | | 0.2 | | |
| | magnesium stearate | 0.675 | | | 1.5 | | |
| | total | 45.005 | | | 100 | | |

Preparation of granule B

The granules were prepared according to the blending ratios shown in Table 63. That is, A-b·dihydrate, finely granulated lactose, cornstarch and low-substituted hydroxypropylcellulose 31 were granulated while spraying a suspension of yellow ferric oxide in a binding solution prepared by adding a 22-fold weight of purified water to hydroxypropylcellulose in a fluid bed granulator (manufactured by POWREX, MP-01), and dried. The obtained granules were sieved through a 22 mesh sieve to give granule B.

Preparation of granule C

The granules were prepared according to the blending ratios shown in Table 64. That is, mannitol S, mannitol 1.059 and low-substituted hydroxypropylcellulose 11 were charged in a fluid bed granulator (manufactured by POWREX, MP-01), granulated while spraying a suspension of D-mannitol, anhydride citric acid and yellow ferric oxide in purified water (306.6 g) and dried. The obtained granules were sieved through a 22 mesh sieve to give granule C.

Preparation of orally disintegrating tablets

The tablets were prepared according to the blending ratios shown in Table 65. That is, the powder ingredients (except magnesium stearate) of granule B, granule C, aspartame, magnesium stearate and a ingredient from crystalline cellulose KG-1000, crystalline cellulose KG-802 and crystalline cellulose PH-101, which is suitable for the formulation, were mixed. Then, magnesium stearate was added to the mixture, and the obtained mixture was compressed in a tablet press (manufactured by RIKEN, hydraulic press machine) to give tablets (weight 250 mg, diameter 9 mm per tablet). During compression, the compression force was adjusted such that the compression disintegration time was around 25 sec. The compression force at that time is shown in Table 69 in the Experimental Example below.

Comparative Examples 69 - 71

<Formulation disclosed in patent document 5, which is different from that of the present invention>

[Table 66]

Table 66 orally disintegrating tablet (Comparative Examples 69 - 71) mixed powder D formulation (amount blended) and blending ratio

| | | amount blended (g) | blending ratio (wt%) |
|---|---|---|---|
| ingredient | | Comparative Example | Comparative Example |
| | | common to 69 - 71 | common to 69 - 71 |
| | compound A-b·dihydrate | 22.4 | 20.3 |
| | (as compound A-b) | (21.17) | (19.2) |
| | finely granulated lactose | 65.78 | 59.6 |
| | cornstarch | 3.3 | 3.0 |
| | low-substituted hydroxypropyl-cellulose 31 | 16.5 | 14.9 |
| | hydroxypropylcellulose | 2.2 | 2.0 |
| | yellow ferric oxide | 0.22 | 0.2 |
| | total | 110.4 | 100 |

[Table 67]

Table 67 orally disintegrating tablet (Comparative Examples 69 - 71) mixed powder E formulation (amount blended) and blending ratio

| | | amount blended (g) | blending ratio (wt%) |
|---|---|---|---|
| ingredient | | Comparative Example | Comparative Example |
| | | common to 69 - 71 | common to 69 - 71 |
| | mannitol S | 57.43 | 56.8 |
| | mannitol 1.059 | 30.09 | 29.7 |
| | low-substituted hydroxypropyl-cellulose 11 | 9.59 | 9.5 |
| | mannitol | 3.07 | 3.0 |
| | anhydride citric acid | 0.96 | 0.9 |
| | yellow ferric oxide | 0.077 | 0.1 |
| | total | 101.217 | 100 |

[Table 68]

Table 68 orally disintegrating tablet (Comparative Examples 69 - 71) formulation (amount blended) and blending ratio

| ingredient | | amount blended (g) | | | blending ratio (wt%) | | |
|---|---|---|---|---|---|---|---|
| | | Comparative Example | | | Comparative Example | | |
| | | 69 | 70 | 71 | 69 | 70 | 71 |
| | mixed powder D | 18 | | | 40.0 | | |
| | mixed powder E | 23.76 | | | 52.8 | | |
| | crystalline cellulose KG-1000 | 2.48 | - | - | 5.5 | - | - |
| | crystalline cellulose KG-802 | - | 2.48 | - | - | 5.5 | - |
| | crystalline cellulose PH-101 | - | - | 2.48 | - | - | 5.5 |
| | aspartame | 0.09 | | | 0.2 | | |
| | magnesium stearate | 0.675 | | | 1.5 | | |
| | total | 45.005 | | | 100 | | |

Preparation of mixed powder D

The mixed powders were prepared according to the blending ratios shown in Table 66. That is, A-b·dihydrate, finely granulated lactose, cornstarch, low-substituted hydroxypropylcellulose 31, hydroxypropylcellulose and yellow ferric oxide were mixed, and the mixture was sieved through a 22 mesh sieve to give mixed powder D.

Preparation of mixed powder E

The mixed powders were prepared according to the blending ratios shown in Table 67. That is, mannitol S, mannitol 1.059, low-substituted hydroxypropylcellulose 11, D-mannitol, anhydride citric acid and yellow ferric oxide were mixed, and the mixture was sieved through a 22 mesh sieve to give mixed powder E.

Preparation of orally disintegrating tablets

The tablets were prepared according to the blending ratios shown in Table 68. That is, the powder ingredients (except magnesium stearate) of mixed powder D, mixed powder E, aspartame, magnesium stearate and a ingredient from crystalline cellulose KG-1000, crystalline cellulose KG-802 and crystalline cellulose PH-101, which is suitable for the formulation, were mixed. Then, magnesium stearate was added to the mixture, and the obtained mixture was compressed in a tablet press (manufactured by RIKEN, hydraulic press machine) to give tablets (weight 250 mg, diameter 9 mm per tablet). During compression, the compression force was adjusted such that the compression disintegration time was around 25 sec. The compression force at that time is shown in Table 69 in the Experimental Example below.

Experimental Example 3

The tablets prepared in the above-mentioned Comparative Examples 66 - 71 were measured for disintegration time and hardness of initial and after humidification to obtain the results of Table 69.
[Table 69]

| Disintegration time and hardness of initial and after humidification | | | | | |
|---|---|---|---|---|---|
| | compression force (MPa) [*1] | disintegration time (s) [*2] | disintegration time (s) [*3] | hardness (N/mm²) [*4] | hardness after humidification (N/mm) [*5] |
| Com. Ex. 66 | 3.4 | 25 | 26 | 1.3 | 0.7 |
| Com. Ex. 67 | 3.4 | 26 | 27 | 1.3 | 0.6 |
| Com. Ex. 68 | 3.4 | 30 | 25 | 1.1 | 0.7 |
| Com. Ex. 69 | 5.0 | 27 | 24 | 1.2 | 0.7 |
| Com. Ex. 70 | 5.0 | 27 | 23 | 1.2 | 0.7 |
| Com. Ex. 71 | 3.4 | 24 | 20 | 0.8 | 0.4 |

*1: The pressure (MPa) applied onto the whole punch during compression was measured using a pressure gauge provided on a hydraulic press machine manufactured by RIKEN.
*2: Using three healthy male test subjects, an average time (sec) from placing a tablet in the oral cavity to disintegration thereof was calculated.
*3: Using three healthy male test subjects, an average time (sec) from placing a tablet preserved under conditions of 25°C and 75% RH for 3 days in the oral cavity to disintegration thereof was calculated.
*4: The tablet thickness was measured by a dial thickness gauge (manufactured by Niigataseiki, DS-3010S), the hardness (kgf) was measured using a tablet hardness meter (manufactured by Toyama Sangyo Co., Ltd., TH-203CP), and the absolute hardness was calculated from the following formula.

$$\text{absolute hardness} = \text{measured hardness (kgf)} \times 9.8/\text{cross-sectional area (mm}^2)$$

*5: After preservation at 25°C, 75% RH for 3 days, the tablet thickness was measured by a dial thickness gauge (manufactured by Niigataseiki, DS-3010S), the hardness (kgf) was measured using a tablet hardness meter (manufactured by Toyama Sangyo Co., Ltd., TH-203CP), and the absolute hardness was calculated from the formula of *4.

In the orally disintegrating tablets prepared in the above-mentioned Comparative Examples, the disintegration time in oral cavity was within 30 sec both before and after humidification. However, the absolute hardness does not satisfy 2.0N/mm², and the hardness after humidification does not reach 1.5N/mm², either. Therefore, the tablets do not have sufficient hardness for performing operations such as one dose packaging and the like by an automatic packaging machine.

Industrial Applicability

The present invention can provide an orally disintegrating tablet permitting one dose packaging, that is, an orally disintegrating tablet having both suitable hardness and rapid disintegrability in oral cavity, which maintains orally disintegrability even under moist conditions, and hardness of not less than a predetermined level necessary for using in an automatic packaging machine.
While some of the embodiments of the present invention have been described in detail in the above, it is, however, possible for those of ordinary skill in the art to make various modifications and changes to the particular embodiments shown without substantially departing from the teaching and advantages of the present invention. Such modifications and changes are encompassed in the spirit and scope of the present invention as set forth in the appended claims.
The present invention is based on JP application Nos. 2008-32490 and 2008-113249 filed in Japan, the contents of which are encompassed in full herein.

**EP 2 251 005 B1**

**Claims**

1. An orally disintegrating tablet comprising (1) an active ingredient, (2) mannitol, (3) crystalline cellulose and (4) at least two kinds of particular ingredients selected from the group consisting of low-substituted hydroxypropylcellulose, cornstarch and carmellose, wherein the blending ratio of each ingredient relative to 100 wt% of the disintegrating tablet is (1) 0.01 to 50 wt%, (2) 20 to 86 wt%, (3) 10 to 30 wt%, and (4) 1 to 20 wt% for each particular ingredient and 3 to 60 wt% as the total of the particular ingredients to be blended, and an assembly of (3) crystalline cellulose to be blended has a bulk density of not more than 0.18 g/cm$^3$.

2. The orally disintegrating tablet according to claim 1, wherein (4) the particular ingredients comprise carmellose and cornstarch.

3. The orally disintegrating tablet according to claim 1 or 2, wherein the blending ratio of (2) mannitol is 20 to 75 wt%, relative to 100 wt% of the disintegrating tablet.

4. The orally disintegrating tablet according to claim 3, wherein, in (4) the particular ingredients, the blending ratio of carmellose is 1 to 10 wt% and that of cornstarch is 5 to 20 wt%, relative to 100 wt% of the disintegrating tablet.

5. The orally disintegrating tablet according to claim 1, wherein (4) the particular ingredients comprise low-substituted hydroxypropylcellulose, carmellose and cornstarch.

6. The orally disintegrating tablet according to claim 5, wherein (4) the particular ingredient comprise the blending ratio of low-substituted hydroxypropylcellulose is 1 to 10 wt%, that of carmellose is 1 to 10 wt% and that of cornstarch is 5 to 20 wt%, relative to 100 wt% of the disintegrating tablet.

7. The orally disintegrating tablet according to any one of claims 1 to 6, wherein (1) the active ingredient is 4-amino-5-chloro-2-ethoxy-N-[[4-(4-fluorobenzyl)-2-morpholinyl]methyl]benzamide (hereinafter to be referred to as "compound A") or a pharmaceutically acceptable salt thereof.

8. The orally disintegrating tablet according to any one of claims 1 to 7, further comprising (5) at least one kind of water-soluble polymer selected from the group consisting of methylcellulose, hydroxypropylcellulose, polyvinyl alcohol, hypromellose and polyvinylpyrrolidone in a total blending ratio of (5) 0.5 to 10 wt% relative to 100 wt% of the disintegrating tablet.

9. The orally disintegrating tablet according to claim 8, wherein (5) the water-soluble polymer is methylcellulose or hydroxypropylcellulose, and the total blending ratio thereof is 0.5 to 5 wt%, relative to 100 wt% of the disintegrating tablet.

10. An orally disintegrating tablet according to any one of claims 1 to 9 comprising; active ingredient-containing particles obtainable by mixing and particulating (1a) an active ingredient, (2a) mannitol and (5a) at least one kind of water-soluble polymer selected from the group consisting of methylcellulose and hydroxypropylcellulose; (2b) mannitol; (3b) crystalline cellulose; (4b) at least two kinds of particular ingredients selected from the group consisting of low-substituted hydroxypropylcellulose, cornstarch and carmellose and, where necessary; (5b) at least one kind of water-soluble polymer selected from the group consisting of methylcellulose, hydroxypropylcellulose, polyvinyl alcohol, hypromellose and polyvinylpyrrolidone, wherein the blending ratio relative to 100 wt% of the disintegrating tablet is (1a) 0.01 to 50 wt%, the total of (2a) and (2b) 20 to 86 wt%, (3b) 10 to 30 wt%, each particular ingredient of (4b) 1 to 20 wt% and the total of the particular ingredients to be blended 3 to 60 wt%, and when (5a) and (5b) are blended, the total thereof 0.5 to 10 wt%, and an assembly of (3b) crystalline cellulose to be blended has a bulk density of not more than 0.18 g/cm$^3$.

11. An orally disintegrating tablet according to any one of claims 1 to 9, obtainable by forming a mixture of; granules obtainable by granulating a mixture of (1) an active ingredient, (2b) mannitol, (3b) crystalline cellulose and (4b) at least one kind of particular ingredient selected from the group consisting of low-substituted hydroxypropylcellulose, cornstarch and carmellose and, where necessary, (5b) at least one kind of water-soluble polymer selected from the group consisting of methylcellulose, hydroxypropylcellulose, polyvinyl alcohol, hypromellose and polyvinylpyrrolidone; (3c) crystalline cellulose; (4c) at least one kind of particular ingredient selected from the group consisting of low-substituted hydroxypropylcellulose, cornstarch and carmellose, wherein the blending ratio of each ingredient relative to 100 wt% of the disintegrating tablet is (1) 0.01 to 50 wt%, (2b) 20 to 86 wt%, the total of (3b) and (3c) 10 to 30 wt%, and at least two kinds of particular ingredients are contained in (4b) and (4c), the blending ratio of each particular ingredient is 1 to 20 wt% and the total of the particular ingredients to be blended is 3 to 60 wt% and the blending ratio of (5b) when blended is 0.5 to 10 wt%, and an assembly of (3b) crystalline cellulose and (3c) crystalline cellulose to be blended has a bulk density of not more than 0.18 g/cm$^3$.

12. The orally disintegrating tablet according to any one of claims 1 to 11, wherein the assembly of crystalline cellulose to be blended has a bulk density of not more than 0.18 g/cm$^3$ and the ratio thereof relative to 100 wt% of the disintegrating tablet is 18 to 30 wt%.

13. The orally disintegrating tablet according to any one of claims 1 to 11, wherein the assembly of crystalline cellulose to be blended has a bulk density of not more than 0.15 g/cm$^3$ and the ratio thereof relative to 100 wt% of the disintegrating tablet is 10 to 30 wt%.

14. An orally disintegrating tablet according to any one of claims 1 to 13, which shows an absolute hardness of not less than 1.5N/mm$^2$ after preservation for 3 days under the conditions of 25°C, relative humidity 75%.

15. A process of preparing an orally disintegrating tablet, comprising mixing and forming; active ingredient-containing particles obtainable by mixing and particulating (1a) an active ingredient, (2a) mannitol and (5a) at least one kind of water-soluble polymer selected from the group consisting of methylcellulose and hydroxypropylcellulose; (2b) mannitol; (3b) crystalline cellulose; (4b) at least two kinds of particular ingredients selected from the group consisting of low-substituted hydroxypropylcellulose, cornstarch and carmellose; and, where necessary, (5b) at least one kind of water-soluble polymer selected from the group consisting of methylcellulose, hydroxypropylcellulose, polyvinyl alcohol, hypromellose and polyvinylpyrrolidone, wherein the blending ratio relative to 100 wt% of the disintegrating tablet is (1a) 0.01 to 50 wt%, the total of (2a) and (2b) 20 to 86 wt%, (3b) 10 to 30 wt%, each particular ingredient of (4b) 1 to 20 wt% and the total of the particular ingredients to be blended 3 - 60 wt%, and when (5a) and (5b) are blended, the total thereof 0.5 to 10 wt%, and an assembly of (3b) crystalline cellulose to be blended has a bulk density of not more than 0.18 g/cm$^3$.

**Patentansprüche**

1. Eine oral zerfallende Tablette, umfassend (1) einen Wirkstoff, (2) Mannitol, (3) kristalline Cellulose und (4) mindestens zwei Arten besonderer Bestandteile, ausgewählt aus der Gruppe bestehend aus gering substituierter Hydroxypropylcellulose, Maisstärke und Carmellose, wobei das Mischverhältnis jedes Bestandteils, bezogen auf 100 Gen.-% der zerfallenden Tablette, (1) 0,01 bis 50 Gew.-%, (2) 20 bis 86 Gew.-%, (3) 10 bis 30 Gen.-% und (4) 1 bis 20 Gew.-% für jeden besonderen Bestandteil und 3 bis 60 Gew.-% als die Gesamtmenge der beizumischenden besonderen Bestandteile, beträgt und ein beizumischendes Aggregat der (3) kristallinen Cellulose eine Schüttdichte von nicht mehr als 0,18 g/cm$^3$ aufweist.

2. Die oral zerfallende Tablette gemäß Anspruch 1, wobei (4) die besonderen Bestandteile Carmellose und Maisstärke umfassen.

3. Die oral zerfallende Tablette gemäß Anspruch 1 oder 2, wobei das Mischverhältnis von (2) Mannitol 20 bis 75 Gew.-%, bezogen auf 100 Gew.-% der zerfallenden Tablette, beträgt.

4. Die oral zerfallende Tablette gemäß Anspruch 3, wobei in (4), den besonderen Bestandteilen, das Mischverhältnis von Carmellose 1 bits 10 Gew.-% beträgt und das von Maisstärke 5 bis 20 Gew.-%, bezogen auf 100 Gew.-% der zerfallenden Tablette, beträgt.

5. Die oral zerfallende Tablette gemäß Anspruch 1, wobei (4) die besonderen Bestandteile gering substituierte Hydroxypropylcellulose, Carmellose und Maisstärke umfassen.

6. Die oral zerfallende Tablette gemäß Anspruch 5, wobei (4), die besonderen Bestandteile das Mischverhältnis von gering substituierter Hydroxypropylcellulose 1 bis 10 Gew.-% beträgt, das von Carmellose 1 bis 10 Gew.-% beträgt und das von Maisstärke 5 bis 20 Gew.-% beträgt, bezogen auf 100 Gew.-% der zerfallenden Tablette.

7. Die oral zerfallende Tablette gemäß einem der Ansprüche 1 bis 6, wobei (1) der Wirkstoff 4-Amino-5-chlor-2-ethoxy-N-[[4-(4-fluorbenzyl)-2-morpholinyl]methyl]-benzamid (nachstehend als "Verbindung A" bezeichnet) oder ein pharmazeutisch verträgliches Salz davon ist,

8. Die oral zerfallende Tablette gemäß einen der Ansprüche 1 bis 7, ferner umfassend (5) mindestens eine Art eines wasserlöslichen Polymers, ausgewählt aus der Gruppe bestehend aus Methylcellulose, Hydroxypropylcellulose, Polyvinylalkohol, Hypromellose und Polyvinylpyrrolidon, in einem Gesamt-Mischverhältnis von (5) 0,5 bis 10 Gew.-%, bezogen auf 100 Gew.-% der zerfallenden Tablette.

9. Die oral zerfallende Tablette gemäß Anspruch 8, wobei (5) das wasserlösliche Polymer Methylcellulose oder Hydroxypropylcellulose ist und das Gesamt-Mrschverhältnis davon 0,5 bis 5 Gew.-%, bezogen auf 100 Gew.-% der zerfallenden Tablette, beträgt.

10. Eine oral zerfallende Tablette gemäß einem der Ansprüche 1 bis 9, umfassend wirkstoffhaltige Teilchen, erhältlich durch Mischen und zu Teilchen formen von (1a) einem Wirkstoff, (2a) Mannitol und (5a) mindestens einer Art eines wasserlöslichen Polymers, ausgewählt aus der Gruppe bestehend aus Methylcellulose und Hydroxypropylcellulose; (2b) Manminol; (3b) kristalliner Cellulose; (4b) mindestens zwei Arten besonderer Bestandteile, ausgewählt aus der Gruppe bestehend aus gering substituierter Hydroxypropylcellulose, Maisstärke und Carmellose und, wenn erforderlich; (5b) mindestens einer Art eines wasserlöslichen Polymers, ausgewählt aus der Gruppe bestehend aus Methylcellulose, Hydroxypropylcellulose, Polyvinylalkohol, Hypromellose und Polyvinylpyrrolidon, wobei das Mischverhältnis, bezogen auf 100 Gew.-% der zerfallenden Tablette, (1a) 0,01 bis 50 Gew.-%, die Gesamtmenge von (2a) und (2b) 20 bis 86 Gew.-%, (3b) 10 bis 30 Gew.-%, jeder besondere Bestandteil von (4b) 1 bis 20 Gew.-% und die Gesamtmenge der zu mischenden besonderen Bestandteile 3 bis 60 Gew.-%, und wenn (5a) und (5b) beigemischt werden, die Gesamtmenge davon 0,5 bis 10 Gew.-% beträgt und ein beizumischendes Aggregat der (3b) kristallinen Cellulose eine Schüttdichte, von nicht mehr als 0,18 g/cm$^3$ aufweist.

11. Eine oral zerfallende Tablette gemäß einem der Ansprüche 1 bis 9, erhältlich durch Bilden eines Gemisches aus Granulat, erhältlich durch Granulieren eines Gemisches aus (1) einem Wirkstoff, (2b) Mannitol, (3b) kristalliner Cellulose und (4b) mindestens einer Art eines besonderen Bestandteils, ausgewählt aus der Gruppe bestehend aus gering substituierter Hydroxypropylcellulose, Maisstärke und Carmellose und, wenn erforderlich, (5b) mindestens einer Art eines wasserlöslichen Polymers, ausgewählt aus der Gruppe bestehend aus Methylcellulose, Hydroxypropylcellulose, Polyvinylalkohol, Hyprotnellose und Polyvinylpyrrolidon; (3c) kristalliner Cellulose; (4c) mindestens einer Art eines besonderen Bestandteils, ausgewählt aus der Gruppe bestehend aus gering substituierter Hydroxypropylcellulose, Maisstärke und Carmellose, wobei das Mischverhältnis jedes Bestandteils, bezogen aus 100 Gew.-% der zerfallenden Tablette, (1) 0,01 bis 50 Gew.-%, (2b) 20 bis 86 Gew.-%, die Gesamtmenge von (3b) und (3c) 10 bis 30 Gew.-% beträgt und mindestens zwei Arten besonderer Bestandteile in (4b) und (4c) enthalten sind, das Mischverhältnis jedes besonderen Bestandteils 1 bis 20 Gew.-% beträgt und die Gesamtmenge der zu mischenden besonderen Bestandteile 3 bis 60 Gew.-% beträgt und das Mischverhätnis von (5b), wenn beigemischt, 0,5 bis 10 Gew.-% beträft und ein beizumischendes Aggregat der (3b) kristallinen Cellulose und (3c) kristallinen Cellulose eine Schüttdichte von nicht mehr als 0,18 g/cm$^3$ aufweist.

12. Die oral zerfallende Tablette gemäß einem der Ansprüche 1 bis 11, wobei das beizumischende Aggregat der kristallinen Cellulose eine Schüttdichte von nicht mehr als 0,18 g/m$^3$ aufweist und das Verhältnis davon, bezogen auf 100 Gew.-% der zerfallenden Tablette, 18 bis 30 Gew.-% beträgt.

13. Die oral zerfallende Tablette gemäß einem der Ansprüche 1 bis 11, wobei das beizumischende Aggregat der kristallinen Cellulose eine Schüttdichte von nicht mehr als 0,15 g/cm$^3$ aufweist und das Verhältnis davon, bezogen auf 100 Gew.-% der zerfallenden Tablette, 10 bis 30 Gew.-% beträgt.

14. Eine oral zerfallende Tablette gemäß einem der Ansprüche 1 bis 13, die nach 3 Tagen Aufbewahrung unter den Bedingungen 25 °C, relative Feuchtigkeit 75 %, eine absolute Härte von nicht weniger als 1,5 N/mm$^2$ aufweist.

15. Ein Verfahren zur Herstellung einer oral zerfallenden Tablette, umfassend Mischen und Formen von wirkstoffhaltigen Teilchen, erhältlich durch Mischen und zu Teilchen formen von (1a) einem Wirkstoff, (2a) Mannitol und (5a) mindestens einer Art, eines wasserlöslichen Polymers, ausgewählt aus der Gruppe bestehend aus Methylcellulose und Hydroxypropylcellulose;
(2b) Mannitol; (3b) kristalliner Cellulose, (4b) mindestens zwei Arten besonderer Bestandteile, ausgewählt aus der Gruppe bestehend aus gering substituierter Hydroxypropylcellulose, Maisstärke und Carmellose, und, wenn erforderlich, (5b) mindestens einer Art eines wasserlöslichen Polymers, ausgewählt aus der Gruppe bestehend aus Methylcellulose, Hydroxypropylcellulose, Polyvinylalkohol, Hypromellose und Polyvinylpyrrolidon, wobei das Mischverhältnis, bezogen auf 100 Gew.-% der zerfallenden Tablette, (1a) 0,01 bis 50 Gew.-%, die Gesamtmenge von (2a) und (2b) 20 bis 86 Gew.-%, (3b) 10 bis 30 Gew.-%, jedes besonderen Bestandteils von (4b) 1 - 20 Gew.-% und die Gesamtmenge der zu mischenden besonderen, Bestandteile 3 bis 60 Gew.-% und, wenn. (5a) und (5b) beigemischt werden, die Gesamtmenge davon 0,5 bis 10 Gew.-% beträgt und ein beizumischendes Aggregat der (3b) kristallinen Cellulose eine Schüttdichte von nicht mehr als 0,18 g/cm$^3$ aufweist.

**Revendications**

1. Comprimé orodispersible comprenant (1) un principe actif, (2) du mannitol, (3) de la cellulose cristalline et (4) au moins deux types d'ingrédients particuliers choisis dans le groupe constitué de l'hydroxypropylcellulose faiblement substituée, de l'amidon de maïs et de la carmellose, dans lequel le rapport de mélange de chaque ingrédient par rapport à 100 % en poids du comprimé dispersible est (1) de 0,01 à 50 % en poids, (2) de 20 à 86 % en poids, (3) de 10 à 30 % en poids, et (4) de 1 à 20 % en poids pour chaque ingrédient particulier et de 3 à 60 % en poids pour le total des ingrédients particuliers à mélanger, et un ensemble de (3) cellulose cristalline à mélanger a une densité apparente qui n'est pas supérieure à 0,18 g/cm$^3$.

2. Comprimé orodispersible selon la revendication 1, dans lequel (4) les ingrédients particuliers comprennent de la carmellose et de l'amidon de maïs.

3. Comprimé orodispersible selon la revendication 1 ou 2, dans lequel le rapport de mélange du (2) mannitol est de 20 à 75 % en poids, par rapport à 100 % en poids du comprimé dispersible.

4. Comprimé orodispersible selon la revendication 3, dans lequel, dans (4) les ingrédients particuliers, le rapport de mélange de la carmellose est de 1 à 10 % en poids et celui de l'amidon de maïs est de 5 à 20 % en poids, par rapport à 100 % en poids du comprimé dispersible.

5. Comprimé orodispersible selon la revendication 1, dans lequel (4) les ingrédients particuliers comprennent de l'hydroxypropylcellulose faiblement substituée, de la carmellose et de l'amidon de maïs.

6. Comprimé orodispersible selon la revendication 5, dans lequel, dans (4) l'ingrédient particulier, le rapport de mélange de l'hydroxypropylcellulose faiblement substituée est de 1 à 10 % en poids, celui de la carmellose est de 1 à 10 % en poids et celui de l'amidon de maïs est de 5 à 20 % en poids, par rapport à 100 % en poids du comprimé dispersible.

7. Comprimé orodispersible selon l'une quelconque des revendications 1 à 6, dans lequel (1) le principe actif est le 4-amino-5-chloro-2-éthoxy-N-[[4-(4-fluorobenzyl)-2-morpholinyl]méthyl]benzamide (auquel il sera ci-après fait référence comme "Composé A") ou un sel pharmaceutiquement acceptable de celui-ci.

8. Comprimé orodispersible selon l'une quelconque des revendications 1 à 7, comprenant en outre (5) au moins un type de polymère hydrosoluble choisi dans le groupe constitué de la méthylcellulose, de l'hydroxypropylcellulose, de l'alcool polyvinylique, de l'hypromellose et de la polyvinylpyrrolidone en un rapport de mélange total de (5) 0,5 à 10 % en poids par rapport à 100 % en poids du comprimé dispersible.

9. Comprimé orodispersible selon la revendication 8, dans lequel (5) le polymère hydrosoluble est la méthylcellulose ou l'hydroxypropylcellulose, et son rapport de mélange total est de 0,5 à 5 % en poids, par rapport à 100 % en poids du comprimé dispersible.

10. Comprimé orodispersible selon l'une quelconque des revendications 1 à 9 comprenant ; des particules contenant le principe actif pouvant être obtenues par mélange et mise sous la forme de particules (1a) d'un principe actif, (2a) de mannitol et (5a) d'au moins un type de polymère hydrosoluble choisi dans le groupe constitué de la méthylcellulose et de l'hydroxypropylcellulose ;
(2b) du mannitol ; (3b) de la cellulose cristalline ; (4b) au moins deux types d'ingrédients particuliers choisis dans le groupe constitué de l'hydroxypropylcellulose faiblement substituée, de l'amidon de maïs et de la carmellose et, si nécessaire ; (5b) au moins un type de polymère hydrosoluble choisi dans le groupe constitué de la méthylcellulose, de l'hydroxypropylcellulose, de l'alcool polyvinylique, de l'hypromellose et de la polyvinylpyrrolidone, dans lequel le rapport de mélange par rapport à 100 % en poids du comprimé dispersible est (1a) de 0,01 à 50 % en poids, le total de (2a) et (2b) de 20 à 86 % en poids, (3b) de 10 à 30 % en poids, chaque ingrédient particulier de (4b) de 1 à 20 % en poids et le total des ingrédients particuliers à mélanger de 3 à 60 % en poids, et lorsque (5a) et (5b) sont mélangés, leur total est de 0,5 à 10 % en poids, et un ensemble de (3b) cellulose cristalline à mélanger a une densité apparente qui n'est pas supérieure à 0,18 g/cm$^3$.

**11.** Comprimé orodispersible selon l'une quelconque des revendications 1 à 9, pouvant être obtenu par formation d'un mélange de ; granules pouvant être obtenus par granulation d'un mélange (1) d'un principe actif, (2) de mannitol, (3b) de cellulose cristalline et (4b) d'au moins un type d'ingrédient particulier choisi dans le groupe constitué de l'hydroxypropylcellulose faiblement substituée, de l'amidon de maïs et de la carmellose et, si nécessaire, (5b) d'au moins un type de polymère hydrosoluble choisi dans le groupe constitué de la méthylcellulose, de l'hydroxypropyl-cellulose, de l'alcool polyvinylique, de l'hypromellose et de la polyvinylpyrrolidone ; (3c) de cellulose cristalline ; (4c) d'au moins un type d'ingrédient particulier choisi dans le groupe constitué de l'hydroxypropylcellulose faiblement substituée, de l'amidon de maïs et de la carmellose, dans lequel le rapport de mélange de chaque ingrédient par rapport à 100 % en poids du comprimé dispersible est (1) de 0,01 à 50 % en poids, (2) de 20 à 86 % en poids, le total de (3b) et (3c) de 10 à 30 % en poids, et au moins deux types d'ingrédients particuliers sont contenus dans (4b) et (4c), le rapport de mélange de chaque ingrédient particulier est de 1 à 20 % en poids et le total des ingrédients particuliers à mélanger est de 3 à 60 % en poids et le rapport de mélange de (5b) lorsqu'il est mélangé est de 0,5 à 10 % en poids, et un ensemble de (3b) cellulose cristalline et (3c) cellulose cristalline à mélanger a une densité apparente qui n'est pas supérieure à 0,18 g/cm$^3$.

**12.** Comprimé orodispersible selon l'une quelconque des revendications 1 à 11, dans lequel l'ensemble de cellulose cristalline à mélanger a une densité apparente qui n'est pas supérieure à 0,18 g/cm$^3$ et son rapport par rapport à 100 % en poids du comprimé dispersible est de 18 à 30 % en poids.

**13.** Comprimé orodispersible selon l'une quelconque des revendications 1 à 11, dans lequel l'ensemble de cellulose cristalline à mélanger a une densité apparente qui n'est pas supérieure à 0,15 g/cm$^3$ et son rapport par rapport à 100 % en poids du comprimé dispersible est de 10 à 30 % en poids.

**14.** Comprimé orodispersible selon l'une quelconque des revendications 1 à 13, qui présente une dureté absolue qui n'est pas inférieure à 1,5 N/mm$^2$ après une conservation pendant 3 jours dans des conditions de 25°C, humidité relative de 75 %.

**15.** Procédé de préparation d'un comprimé orodispersible, comprenant le mélange et la formation ; de particules contenant le principe actif pouvant être obtenues par mélange et mise sous la forme de particules (1a) d'un principe actif, (2a) de mannitol et (5a) d'au moins un type de polymère hydrosoluble choisi dans le groupe constitué de la méthylcellulose et de l'hydroxypropylcellulose ;
(2b) de mannitol ; (3b) de cellulose cristalline ; (4b) d'au moins deux types d'ingrédients particuliers choisis dans le groupe constitué de l'hydroxypropylcellulose faiblement substituée, de l'amidon de maïs et de la carmellose ; et, si nécessaire, (5b) d'au moins un type de polymère hydrosoluble choisi dans le groupe constitué de la méthylcellulose, de l'hydroxypropylcellulose, de l'alcool polyvinylique, de l'hypromellose et de la polyvinylpyrrolidone, dans lequel le rapport de mélange par rapport à 100 % en poids du comprimé dispersible est (1a) de 0,01 à 50 % en poids, le total de (2a) et (2b) de 20 à 86 % en poids, (3b) de 10 à 30 % en poids, chaque ingrédient particulier de (4b) de 1 à 20 % en poids et le total des ingrédients particuliers à mélanger de 3 à 60 % en poids, et lorsque (5a) et (5b) sont mélangés, leur total est de 0,5 à 10 % en poids, et un ensemble de (3b) cellulose cristalline à mélanger a une densité apparente qui n'est pas supérieure à 0,18 g/cm$^3$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0078292 A
- WO 2003103713 A
- JP 2000273039 A
- JP 2007197438 A
- JP 2003034655 A
- WO 2005055989 A
- JP 3130214 A
- JP 11092403 A
- US 4870074 A
- JP 2008032490 A
- JP 2008113249 A

**Non-patent literature cited in the description**

- 3rd Lecture Meeting by Association for Innovative Formulation Technology. ASAHI KASEI CHEMICALS CORPORATION, 28 November 2006, 121-136
- Japanese Pharmacopoeia